# EUROPEAN PATENT SPECIFICATION

(11) **EP 2 477 650 B1**
(45) Date of publication and mention of the grant of the patent: **04.09.2019**
(21) Application number: 10755070.9
(22) Date of filing: 16.09.2010
(51) Int. Cl.: A61K 39/145, A61K 39/015, C12N 7/04, C07K 14/11, C07K 14/445, C12N 15/82

(54) **VIRUS LIKE PARTICLES COMPRISING TARGET PROTEINS FUSED TO PLANT VIRAL COAT PROTEINS**
VIRUSÄHNLICHE PARTIKEL, DIE FUSIONSPROTEINE BESTEHEND AUS TARGETPROTEINEN UND COATPROTEINEN VON PFLANZENVIREN ENTHALTEN
PARTICULES DE TYPE VIRUS COMPRENANT DES PROTÉINES CIBLE FUSIONNÉES À DES PROTÉINES D'ENVELOPPE VIRALES VÉGÉTALES

(30) Priority: 25.05.2010 US 348069 P; 18.09.2009 US 243774 P
(43) Date of publication of application: 25.07.2012
(73) Proprietor: Fraunhofer USA Inc., Newark, DE 18711-5449 (US)
(72) Inventor: YUSIBOV, Vidadi, Havertown PA 19083 (US); FARRANCE, Christine, E., Oxford PA 19363 (US); MUSIYCHUK, Konstantin, A., Wilmington DE 19808 (US); METT, Vadim, Newark DE 19711 (US); METT, Valentina, Newark DE 19711 (US)
(74) Representative: RatnerPrestia
(86) International application number: PCT/US2010/049089
(87) International publication number: WO 2011/035004

(56) References cited:
- WO-A1-2008/089569
- WO-A2-2007/011904
- US-A1- 2007 128 213
- YUSIBOV V ET AL: "Peptide-based candidate vaccine against respiratory syncytial virus", VACCINE, vol. 23, no. 17-18, 18 March 2005 (2005-03-18), pages 2261-2265, XP004777535, ISSN: 0264-410X, DOI: DOI:10.1016/J.VACCINE.2005.01.039
- SHOJI Y ET AL: "Plant-derived hemagglutinin protects ferrets against challenge infection with the A/Indonesia/05/05 strain of avian influenza", VACCINE, ELSEVIER LTD, GB, vol. 27, no. 7, 11 February 2009 (2009-02-11), pages 1087-1092, XP025898712, ISSN: 0264-410X, DOI: DOI:10.1016/J.VACCINE.2008.11.108 [retrieved on 2009-01-27] cited in the application
- MUSIYCHUK KONSTANTIN ET AL: "A launch vector for the production of vaccine antigens in plants", INFLUENZA AND OTHER RESPIRATORY VIRUSES, BLACKWELL PUBLISHING LTD, UK, vol. 1, no. 1, 1 January 2007 (2007-01-01) , pages 19-25, XP009058889, ISSN: 1750-2640, DOI: DOI:10.1111/J.1750-2659.2006.00005.X [retrieved on 2007-01-19]
- SAUNDERS K ET AL: "Efficient generation of cowpea mosaicvirus empty virus-like particles by the proteolytic processing of precursors in insect cells and plants", VIROLOGY, vol. 393, no. 2, 5 September 2009 (2009-09-05), pages 329-337, XP026691170, ISSN: 0042-6822, DOI: DOI:10.1016/J.VIROL.2009.08.023 [retrieved on 2009-09-05]
- YUSIBOV V ET AL: "PURIFICATION, CHARACTERIZATION, ASSEMBLY AND CRYSTALLIZATION OF ASSEMBLED ALFALFA MOSAIC VIRUS COAT PROTEIN EXPRESSED IN ESCHERICHIA COLI", JOURNAL OF GENERAL VIROLOGY, SOCIETY FOR GENERAL MICROBIOLOGY, SPENCERS WOOD, GB, vol. 77, no. 4, 1 January 1996 (1996-01-01), pages 567-573, XP008021583, ISSN: 0022-1317
- LEE L A ET AL: "Adaptations of nanoscale viruses and other protein cages for medical applications", NANOMEDICINE: NANOTECHNOLOGY, BIOLOGY AND MEDICINE, vol. 2, no. 3, 1 September 2006 (2006-09-01), pages 137-149, XP025149635, ISSN: 1549-9634, DOI: DOI:10.1016/J.NANO.2006.07.009 [retrieved on 2006-09-01]

## Description

### FIELD OF THE INVENTION

This invention relates to a virus like particle consisting of a fusion protein, an immunogenic composition comprising the virus like particle and the virus like particle for use in inducing a protective immune response against an intracellular pathogenic organism in a subject.

### BACKGROUND OF THE INVENTION

Plant viruses can be effective tools for antigen production and delivery. A variety of important protein antigens have been expressed in transgenic plants, but the level of antigenic protein produced has been relatively low. In an attempt to overcome this problem, plant viral coat proteins have been engineered to act as carrier molecules for fused antigenic peptides and used in transient expression systems. The coat proteins have the potential to self-assemble and form recombinant virus particles that display the desirable antigenic epitopes or foreign polypeptides on their surfaces. Because the recombinant viruses are replicated in cytoplasm, the desirable antigenic epitopes or foreign polypeptides might not be properly presented because of inadequate modification at the post-translational level or improper folding. Antigens produced in plants as a result of infection with recombinant tobacco mosaic viruses (TMVs) or cowpea mosaic viruses have been shown to elicit specific antibodies when injected into mice. However, in these systems, the viruses were unable to assemble when peptides greater than 25 amino acids in length were fused to their coat proteins. In a different system, fusion of polypeptides up to 47 amino acids in length to the coat protein of alfalfa mosaic virus (AIMV) did not inhibit viral assembly and the resultant viral particles containing viral nucleic acids were effective immunogens. (Yusibov, V., et al., PNAS 94: 5784-5788, 1997). More recently, a method has been developed to attach a functional fragment of protein A (133 aa) to the C-terminus of the TMV coat protein using a 15-aa peptide linker. The resultant viral constructs were able to assemble into viral particles that were capable of infecting leaf tissue and moving throughout the tissue. (Werner, S., et al., PNAS 103: 17678-17683, 2006). However, all of these systems yield infective viral particles containing viral RNA. Using such particles to elicit a protective immune response, *i.e.,* as a vaccine, would require inoculating the subject with viral RNA as well as the intended immunogen.

There remains a need for methods and materials to present a desirable antigenic epitope or foreign polypeptide on the surface of virus like particles for immunization where the virus like particles are substantially free of viral nucleic acid.

### SUMMARY OF THE INVENTION

The disclosed subject matter of the present invention provides virus like particles as well as methods for use and preparation of the virus like particles.

According to one aspect of the present invention, a virus like particle is provided. The virus like particle consists of a fusion protein and is substantially free of nucleic acid, wherein the fusion protein comprises a plant viral coat protein and a target protein. The virus like particle may be produced in a host cell selected from the group consisting of bacterial, fungal, plant, insect, amphibian, and mammalian cells. The target protein may be fused to the N-terminus of the plant viral coat protein.

The plant viral coat protein may be derived from a coat protein of a plant virus of alfalfa mosaic virus.

The target protein may be derived from a polypeptide of an intracellular pathogen. The target protein may be derived from a cell surface polypeptide of *Plasmodium falciparum,* or a polypeptide of hemagglutinin of an influenza virus. The influenza virus may be an influenza A virus selected from the group consisting of H1N1, H3N2, H5N1, and H7N7. The influenza virus may also be an influenza B virus. The target protein may comprise an amino acid sequence selected from the group consisting of SEQ ID NOs: 4-11 and 49, and antigenic fragments thereof.

According to another aspect of the present invention, an immunogenic composition is provided. The immunogenic composition comprises the virus like particle. The immunogenic composition may further comprise an adjuvant. The adjuvant may be selected from the group consisting of aluminum salts, oil and water emulsions, and saponin-based adjuvants. In some embodiments, the plant viral coat protein is derived from a coat protein of an alfalfa mosaic virus while the target protein comprises an amino acid sequence selected from the group consisting of SEQ ID NOs: 4-11 and 49, and antigenic fragments thereof.

A method for inducing a protective immune response against an intracellular pathogen in a subject is provided. The method comprises administering to the subject an immunologically effective amount of a first composition comprising a first virus like particle, wherein the first virus like particle comprises a first fusion protein and is substantially free of nucleic acid, and wherein the first fusion protein comprises a plant viral coat protein and a first target protein derived from the first intracellular pathogen. In some embodiments, the plant viral coat protein is derived from a coat protein of an alfalfa mosaic virus while the first target protein comprises an amino acid sequence selected from the group consisting of SEQ ID NOs: 4-11 and 49, and antigenic fragments thereof. In some other embodiments, the composition further comprises an adjuvant.

The method may further comprise administering to the subject an immunologically effective amount of a second composition comprising a second virus like particle prior to the administering the first composition, wherein the second virus like particle comprises a second fusion protein and is substantially free of nucleic acid, and wherein the second fusion protein comprises the plant viral coat protein and a second target protein. The second target protein may be derived from a second intracellular pathogen that is not the first intracellular pathogen. In some embodiments, the first target intracellular pathogen may be an influenza virus, the plant viral coat protein is derived from a coat protein of an alfalfa mosaic virus, the first target protein comprises an amino acid sequence selected from the group consisting of SEQ ID NOs: 6-11, and the second target protein comprises an amino acid sequence selected from the group consisting of SEQ ID NOs: 4, 5 and 49. In particular, the first and second target proteins may comprise amino acid sequences of SEQ ID NOs: 6 and 4, respectively.

A method of producing a virus like particle in a host cell comprising a nucleotide sequence encoding a fusion protein is also provided where the virus like particle comprises the fusion protein and is substantially free of nucleic acid, and the fusion protein comprises a plant viral coat protein and a target protein. The method comprises expressing the fusion protein in the host cell under conditions that permit assembly of the virus like particle in the host cell. The method may further comprise purifying the virus like particle from the host cell.

In some embodiments, the host cell is a cell selected from the group consisting of bacterial, fungal, plant, insect, amphibian, and mammalian cells. Where the host cell is a plant cell, the method may further comprise infecting the plant cell with a recombinant bacterium capable of infecting the plant cell, wherein the recombinant bacterium comprises a nucleotide sequence encoding the fusion protein.

In some other embodiments, the target protein is derived from an intracellular pathogen.

In yet some other embodiments, the plant viral coat protein is derived from a coat protein of an alfalfa mosaic virus and the target protein comprises an amino acid sequence selected from the group consisting of SEQ ID NOs: 4-11 and 49, and antigenic fragments thereof.

### BRIEF DESCRIPTION OF THE DRAWINGS

Figure 1 schematically illustrates fusion of a plant viral coat protein to a target protein and self-assembly of the fusion protein into a virus like particle.
Figure 2 shows (A) an exemplary gene construct comprising a fusion protein (CP-fusion construct) and a signal sequence (PR1a); (B) cloning the CP-fusion construct into expression vector pGR-D4; and (C) cloning the CP-fusion construct into expression vector pBI121. RB and LB represent the right and left borders of the area introduced into a plant cell by a recombinant *Agrobacterium.*
Figure 3 shows transmission electron micrographs of virus like particles made up of a fusion protein of an AIMV coat protein to *Plasmodium falciparum* Pfs25 (top panels), Pfs28 (middle panels), or influenza virus HA3A target protein (bottom panels). The virus like particles were negatively stained (left panels) and labeled with immuno-gold tagged antibodies specific for each respective target protein (right panels). Bar = 100 nm or 200 nm as labeled.

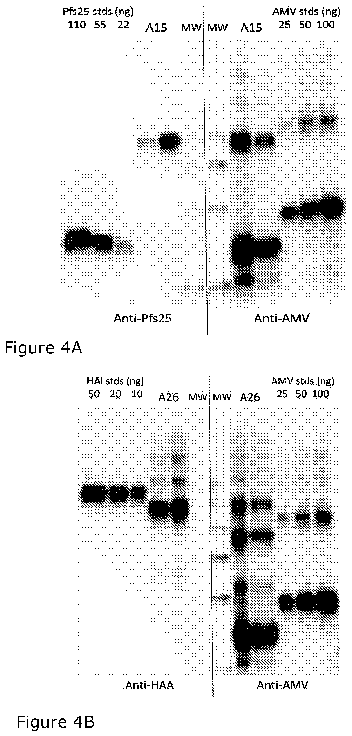

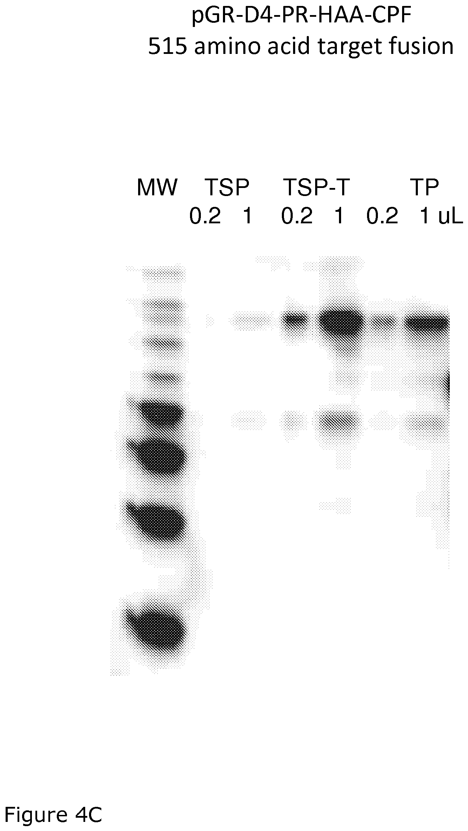

Figures 4A and 4B show immunoblots of purified fusion proteins of AIMV coat protein fused with (A) Pfs25 or (B) HA3A. The right half of each blot was incubated with an antibody against AIMV. The left half of each blot was incubated with a monoclonal antibody to (A) Pfs25 or (B) HA3A. Figure 4C is an immunoblot of crude extract of plant proteins containing the AIMV coat protein fused with the full length hemagglutinin HAA target protein extracted under different conditions to assess solubility. The blot was reacted with an anti-HAA monoclonal antibody. TSP=total soluble protein; TSP-T=triton-extracted soluble protein; TP=total protein.
Figure 5A shows anti-A/Anhui/1/05 IgG responses in sera samples from mice 28 or 56 days after immunization with VLPs containing HA3A-CPF ("CPF" designates "coat protein fusion") with or without an adjuvant, QUIL A™ or ALHYDROGEL™, or HAA1 with QUIL A™. Figure 5B shows serum hemagglutination inhibition (HI) antibody responses in sera samples from the same mice as in Figure 5A.
Figure 6 shows IgG titers in sera collected from mice 56 days after immunization with VLPs containing Pfs25-CPF or Pfs28-CPF, with or without an adjuvant, QUIL A™ or ALHYDROGEL™.
Figure 7 shows anti-A/Indonesia/5/05 IgG isotype titers in sera samples collected from mice 35 days after immunization with HAI3-CPF VLPs or HAI1, with or without ALHYDROGEL™, or CP only.
Figure 8 shows serum hemagglutination inhibition (HI) antibody responses in sera samples from mice after immunization with HAI3-CPF VLPs or HAI1, with or without ALHYDROGEL™, or CP only.
Figures 9A and 9B show serum hemagglutination inhibition (HI) antibody responses against A/Anhui/1/05 in sera samples from mice after a primary vaccination with Pfs25-CPF VLPs or PBS, with or without ALHYDROGEL™, and a secondary vaccination with PBS or HA3A-CPF VLPs, with or without ALHYDROGEL™.

### DETAILED DESCRIPTION OF THE INVENTION

The present invention generally relate to virus like particles (VLPs) comprising a fusion protein that is substantially free of nucleic acid, and to methods of using and preparing the VLPs.

The term "virus like particle" or "VLP" used herein refers to a nonreplicating viral shell derived from a virus, for example, a plant virus as discussed below. VLPs are generally composed of at least one viral protein (e.g., capsid, coat, shell, surface or envelope protein), which can form spontaneously upon expression of the viral protein in a variety of suitable host cells, for example, bacterial, fungal, plant, yeast, insect, amphibian, and mammalian cells. The presence of the VLPs may be detected using conventional techniques known in the art (e.g., electron microscopy, dynamic light scattering, and size-exclusion chromatography). VLPs may also be isolated using conventional techniques in the art (e.g., density gradient centrifugation, size-exclusion chromatography, and affinity chromatography).

The terms "protein" and "polypeptide" are used herein interchangeably, and refer to a polymer of amino acid residues with no limitation with respect to the minimum length of the polymer. The definition includes both full-length proteins and fragments thereof, as well as modifications thereof (e.g., glycosylation, phosphorylation, deletions, additions and substitutions).

The term "derived from" used herein refers to the origin or source, and may include naturally occurring, recombinant, unpurified or purified molecules.

The term "fragment" of a protein as used herein refers to a polypeptide having an amino acid sequence that is the same as a part, but not all, of the amino acid sequence of the protein.

The term "variant" of a protein as used herein refers to a polypeptide having an amino acid sequence that is the same as the amino acid sequence of the protein except having at least one amino acid modified, for example, deleted, inserted, or replaced. The variant may have an amino acid sequence at least about 80%, 90%, 95%, or 99%, preferably at least about 90%, more preferably at least about 95%, identical to the amino acid sequence of the protein.

The term "antigen" as used herein refers to a molecule containing one or more epitopes (linear and/or conformational) that are capable of stimulating the immune system of a subject to make a humoral and/or cellular antigen-specific response. A humoral immune response refers to an immune response mediated by antibodies produced by B lymphocytes, or B cells, while a cellular immune response refers to an immune response mediated by T lymphocytes, or T cells, and/or other white blood cells. In general, a B-cell epitope contains at least about 5 amino acids but can be 3-4 amino acids while a T-cell epitope includes at least about 7-9 amino acids and a helper T-cell epitope includes at least 12-20 amino acids. An antigen may be derived from a protein (e.g., a surface protein) of an intracellular pathogenic organism or pathogen.

The term "immunogenic composition" as used herein refers to a composition that comprises an antigenic molecule and is capable of eliciting a humoral and/or cellular antigen-specific response in a subject upon administration of the composition to the subject.

The term "subject" as used herein refers to a mammal, preferably a human.

The term "about" as used herein when referring to a measurable value such as an amount, a percentage, and the like, is meant to encompass variations of ±20% or ±10%, more preferably ±5%, even more preferably ±1%, and still more preferably ±0.1% from the specified value, as such variations are appropriate to perform the disclosed methods.

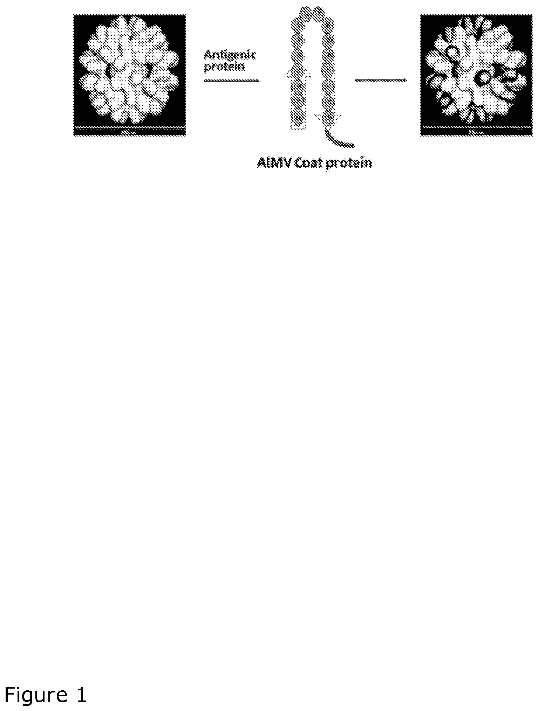

According to one aspect of the present invention, a virus like particle (VLP) is provided. The VLP consists of a fusion protein and is substantially free of nucleic acid, wherein the fusion protein comprises a plant viral coat protein and a target protein. Figure 1 demonstrates schematically that fusion of a target protein with a plant viral coat protein results in assembly of a VLP presenting the target protein on the surface of the VLP.

The plant viral coat protein may be derived from a coat protein of any virus that is capable of self-assembling into particles substantially free of nucleic acid. The plant viral coat protein may be a full length coat protein, or a functional fragment or variant thereof. Examples of suitable plant viral coat proteins include proteins derived from coat proteins of alfalfa mosaic virus (AIMV-CP, CP or CPF), potato virus Y (Stram, et al., Virus Research 28: 29-35, 2002) and Johnsongrass mosaic virus (Jagadish, et al., J. Gen. Virol. 74: 893-896, 1993).

The amino acid sequence of a coat protein may be genetically modified to improve particle assembly or immunogenicity. The amino acids of the coat protein may be deleted, inserted, or replaced, provided that the resulting variant of the coat protein retains the ability to assemble into a particle in the absence of nucleic acid when fused to a target protein. A fragment or variant of a coat protein is functional where the fragment or variant is capable of self-assembling into a particle that is substantially free of nucleic acid. For example, the protein represented by SEQ ID NO:1 lacks the first methionine (from the start codon) of the native AIMV-CP sequence, and SEQ ID NO:2 is a truncated fragment of the AIMV-CP sequence that is missing the first 25 amino acids of the native protein sequence.

Target proteins may be derived from any desirable antigen or polypeptide of any source. They may have at least about 6, 10, 50, 100, 200, 300, or 500 amino acids. Preferred target proteins are derived from surface proteins from intracellular pathogenic organisms, *e.g.*, bacterial, viral, fungal, and parasitic organisms that are suitable for use in vaccines. Examples of viral organisms include *Plasmodium falciparum* and influenza viruses. Influenza viruses include various strains of influenza A viruses (e.g., H1N1, H3N2, H5N1, and H7N7) and influenza B viruses. A target protein derived from a surface protein may be the full length surface protein or a fragment or variant thereof. For example, the target protein may be derived from a cell surface polypeptide of *Plasmodium falciparum,* or a polypeptide of hemagglutinin of an influenza virus.

For use in a vaccine, preferred fragments or variants are those that are antigenic, i.e., capable of inducing a protective immune response in a subject. The fragments or variants have at least about 6, 7, 8, 9, 10, 11, 12, 13, 14 or 15 amino acids in length. Any protein, protein fragment, or protein variant may be used as a target protein, as long as particles substantially free of nucleic acid are self-assembled when it is fused to a plant viral coat protein. Some examples of target proteins are described by SEQ ID NOs: 4-19 and 23-49 in the Sequence Listing.

The target fusion protein may further comprise a signal sequence to target the fusion protein to a particular site in a host cell (*e.g.*, ER, chloroplast) or to direct extracellular secretion of the fusion protein. The absence of a signal peptide results in translation of the proteins in the cytoplasm. Any signal sequence appropriate for the host cell may be utilized, or the signal sequence may be omitted. Signal sequences have been found to be conserved across phyla and kingdoms, and, in general, almost any signal sequence may be used. Bennett and Scheller, PNAS 90: 2559-2563, 1993; Luirink and Sinning, Biochim. Biophys. Acta 1694: 17-35, 2005; Doudna and Batey, Ann. Rev. Biochem. 73: 539-557, 2004; Stern, et al., Trends in Cell and Mol. Biol. 2: 1-17, 2007. In one embodiment for expression in a plant tissue, a preferred signal sequence is the plant pathogenesis-related protein 1 (PR-1) of *Nicotiana tabacum* (SEQ ID NO:3).

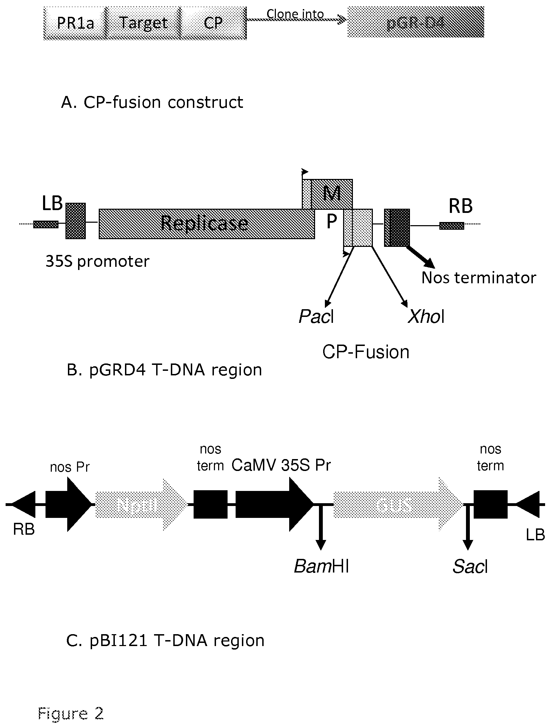

The fusion proteins can be prepared using standard methods of cloning and molecular biology, e.g., as described in Example 1. An exemplary construct containing coding sequences for a plant viral coat protein and a target protein may be prepared and subcloned into an expression vector (Figure 2). The target protein may be fused to the plant viral coat protein directly or indirectly (e.g., via a peptide linker). For example, the target protein may be fused to the N-terminus of the plant viral coat protein by placing the coding sequence for the target protein at the 5' end of the code sequence of the plant viral protein (Figure 2). The expression construct may optionally include a signal sequence to direct the localization or secretion of the fusion protein. The expression vector may be introduced into a suitable expression system for expression of the fusion protein in a host cell using convention techniques known in the art (e.g., transfection, transduction, infection and electroporation). Any expression system appropriate for a selected host cell may be used, including plasmid and viral vectors. Suitable host cells may include bacterial, mammalian, plant, fungal, amphibian and insect cells. The use of plasmid and viral vectors for expression of the fusion proteins in plant cells is described in Examples 1-3. Transgenic organisms may also be created to produce the fusion proteins. Appropriate expression vectors and methods for producing recombinant proteins in these host systems and for isolating and purifying the recombinant proteins are known in the art and are described, for example, in the "Practical Approach" series published by Oxford University Press, which includes Protein Expression, a Practical Approach, S.J. Higgins and B.D. Hames, Eds., 1999; Molecular Plant Biology Vols. 1 and 2, P.M. Gilmartin and C. Bowler, Eds., 2002; Protein Purification Techniques, A Practical Approach, S. Roe, Ed., 2001; and the "Methods in Molecular Biology/Medicine" series published by Humana Press, which includes Transgenic Plants: Methods and Protocols, Leandro Peña, Ed., 2004; Baculovirus and Insect Cell Expression Protocols, D.W. Murhammer, Ed., 2007; and Adenovirus Methods and Protocols, Vols. 1 and 2, W.S.M. Wold and A.E. Tollefson, Eds., 2007.

The invention also encompasses an isolated polynucleotide that encodes a fusion protein comprising a plant viral coat protein and a target protein. The polynucleotides may be obtained by any appropriate means known to the art. For example, the nucleotide sequence of a target protein or a fragment or variant thereof, or the nucleotide sequence of a coat protein or a fragment or variant thereof may already be known or may be determined by screening a library and sequencing a potential target or coat protein. Alternatively, the amino acid sequence of a particular fusion protein may be reverse-translated to obtain appropriate nucleotide sequences and corresponding nucleotides may be prepared synthetically.

The VLPs of the present invention are especially advantageous for antigen presentation. The VLPs are highly immunogenic. Further, the VLPs are substantially free of nucleic acid. For example, the particles may appear empty as being visualized by, for example, TEM and negative staining. The particles may contain less than about 10%, 5%, 1%, or 0.1%, preferably less than about 5%, more preferably less than about 1%, nucleic acid by weight. Accordingly, they provide safe and effective vaccines against viral, bacterial, and eukaryotic pathogens.

An immunogenic composition comprising the VLPs is provided. The immunogenic composition is capable of inducing an immune response (i.e., a humoral and/or cellular response) against the target protein in a subject upon administration of the composition to the subject. In some embodiments, the target protein is derived from an intracellular pathogen, and the immunogenic composition is capable of eliciting a protective immune response to the pathogen in a subject upon administration of the composition to the subject.

The immunogenic composition can be formulated as pharmaceutical compositions comprising an effective amount of the VLPs, a pharmaceutically acceptable carrier, diluent, and/or excipient. An "effective amount" refers to an amount of the VLPs required to exhibit a detectable immunopotentiating effect. The effective amount will depend on route of administration, the nature of the formulation, and the subject's condition. A suitable range of "effective amount" may be from 0.001 to 100 µg virus like particles (or fusion protein particles) per kg body weight of the subject. Suitable carriers, diluents, and excipients are known in the art and include, but are not limited to, saline, buffered saline, mannitol, L-histidine, polysorbate 80, dextrose, water, glycerol, ethanol, and combinations thereof. The composition may optionally contain an adjuvant. Appropriate adjuvants include aluminum salts (e.g., Alhydrogel™), oil and water emulsions, and saponin-based adjuvants (Quil A™).

The immunogenic composition may be formulated for oral, anal, transdermal, nasal, mucosal, or parenteral administration. Parenteral administration includes subcutaneous, intramuscular, intraperitoneal, and intravenous injection.

A method for inducing a protective immune response against a first intracellular pathogen in a subject is also provided. The method comprises administering to the subject a first immunogenic composition comprising a first virus like particle, wherein the first virus like particle comprises a first fusion protein and is substantially free of nucleic acid, and wherein the first fusion protein comprises a plant viral coat protein and a first target protein derived from the first intracellular pathogen. The composition may further comprise an adjuvant.

The method may further comprise, prior to the administering the first immunogenic composition, administering to the subject a second immunogenic composition comprising a second virus like particle, wherein the second virus like particle comprises a second fusion protein and is substantially free of nucleic acid, and wherein the second fusion protein comprises the plant viral coat protein and a second target protein. The second target protein may be derived from a second intracellular pathogen that is not the first intracellular pathogen. In some embodiments, the target intracellular pathogen is a strain of an influenza virus while the first target protein is derived from a different intracellular pathogen (e.g., a malaria parasite, *Plasmodium falciparum)* or a different strain of the influenza virus. For example, the target pathogen is an influenza virus, the plant viral coat protein is derived from a coat protein of an alfalfa mosaic virus, the first target protein comprises an amino acid sequence of SEQ ID NO: 6, 7, 8, 9, 10 or 11, and the second target protein comprises an amino acid sequence of SEQ ID NO: 4, 5 or 49.

A method of producing the VLP in a host cell is further provided. The method comprises expressing a fusion protein, which comprises a plant viral coat protein and a target protein, in a host cell comprising a nucleotide sequence encoding the fusion protein under conditions that permit assembly of the virus like particle (VLP) in the host cell. For example, the VLP may be assembled in a plant tissue about 3-7 days after the plant is infiltrated with an expression vector bearing the corresponding fusion construct. The VLP comprises the fusion protein and is substantially free of nucleic acid. The method may further comprise purifying the VLP from the host cell. Suitable host cells include bacterial, fungal, plant, insect, amphibian, and mammalian cells. The nucleotide sequence encoding the fusion protein may or may not be integrated into the host cell genome. Where the host cell is a plant cell, the method may further comprise infecting the plant cell with a recombinant bacterium capable of infecting the plant cell (e.g., *A. tumefaciens* strain GV3101 and other infectious *A. tumefaciens* strains, and infectious *A. rhizogenes* strains), wherein the recombinant bacterium comprises a nucleotide sequence encoding the fusion protein.

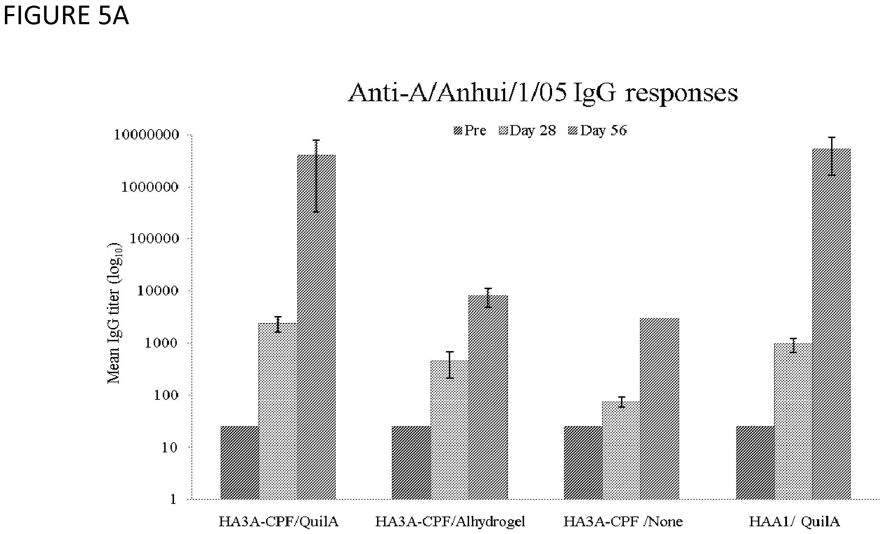

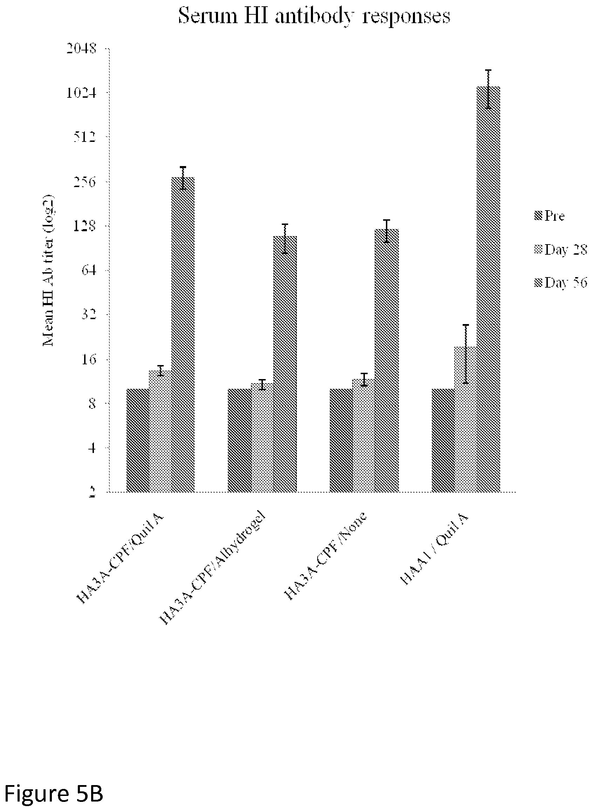

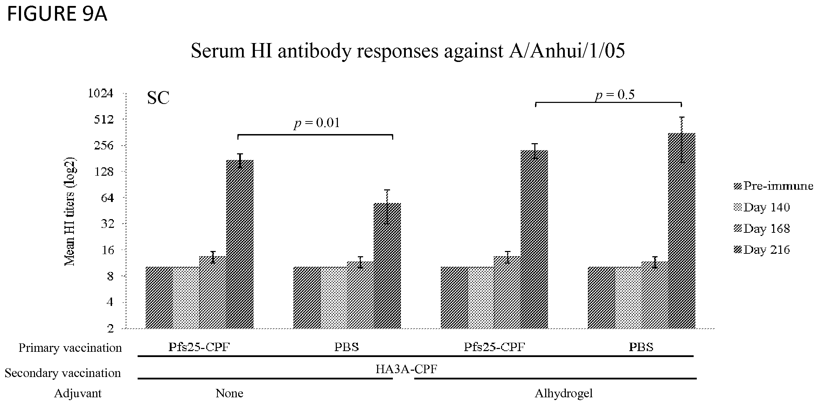

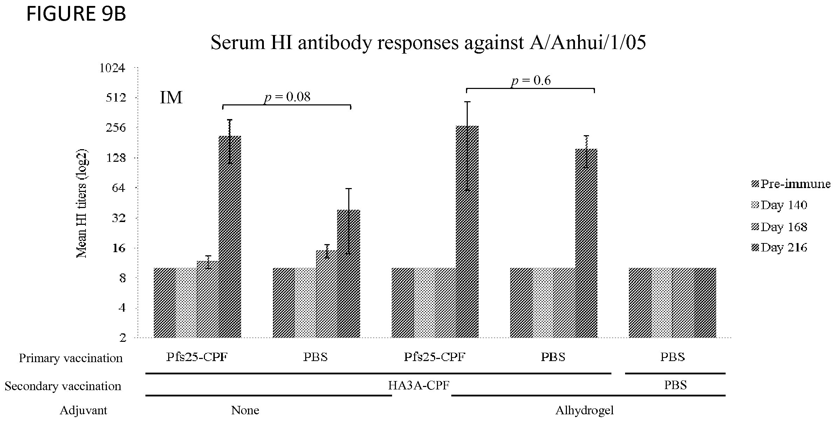

Preparation and testing of exemplary fusion proteins comprising target proteins derived from a malaria parasite, *Plasmodium falciparum,* an influenza virus A/Anhui/1/2005(H5N1), and an influenza virus A/Indonesia/5/2005(H5N1) are described in examples below. When these target proteins were fused with the AIMV coat protein, the resultant fusion proteins assembled into apparently empty VLPs. These particles bound antibodies specific to AIMV and specific to the target proteins. Sera from mice immunized with the VLPs containing the malaria target proteins produced high titers of parasite-binding antibodies and were capable of preventing transmission of the parasite in mosquitoes (Tables 2 and 3). Mice immunized with VLPs containing the influenza proteins produced protective levels of antibody titers as described in Examples 4 and 8, and shown in Figures 5, 7 and 8. Pre-existing immunity against a plant viral coat protein did not interfere with the protective levels of antibody titers in mice immunized with VLPs containing the same plant viral coat protein as described in Example 9 and shown in Figure 9.

### Example 1. Construction of a heterologous vector for the expression of AIMV-CP fusion proteins

Target genes included cell surface proteins specific to different sexual stages of the malaria parasite, *P. falciparum,* (Pfs25 (SEQ ID NO:4), Pfs28 (SEQ ID NO:5), and Pfs230 (SEQ ID NO: 49)), the globular domain of hemagglutinin (HA) from the Anhui strain of influenza virus (HA3A (SEQ ID NO:6)), the globular domain of HA from the California strains of influenza virus (HA3CO4 (SEQ ID NO:7) and HA3C06 (SEQ ID NO:8)), the globular domain of HA from the Indonesia strains of influenza virus (HA3I (SEQ ID NO: 11)), full length HA from the Anhui strain (HAA or HAA1 (SEQ ID NO:9)), and full length HA from the Indonesia strain (HAI or HAI1 (SEQ ID NO: 10)). Each target gene was cloned, using standard methods of molecular biology, as an N-terminal fusion to the AIMV coat protein (AIMV-CP, CP or CPF) or the optimized AIMV coat protein (CPO), which is encoded by a coding sequence optimized for expression in plants, in a shuttle vector with PacI and BsmBI restriction enzymes. Figure 2 shows an exemplary structure of the fusion protein constructs. Sequences were verified by automated sequencing.

Each target-CP sequence was then subcloned into an expression vector, which optionally encoded the signal peptide of the plant pathogenesis-related protein 1 (PR-1) (SEQ ID NO:3). Specifically the sequence was subcloned into the BamHI-SacI sites of pBI121 (Clontech), or into the PacI-XhoI sites of pGRD4 (described in Shoji, et al., Vaccine 27: 108701092, 2009). The cloning strategy is shown in Figure 2.

### Example 2. Infiltration of plants with expression vectors bearing fusion constructs

The expression vectors produced in Example 1 were then introduced into *Agrobacterium tumefaciens* strain GV3101 and the resulting bacteria were grown overnight in minimal medium. The optical density of the cultures was determined and the protein expression strain was mixed with an *Agrobacterium* strain expressing the suppressor of silencing protein, p19, at a 4:1 ratio to a final O.D. of 0.5. The *Agrobacterium* solution was introduced by hand infiltration into the aerial parts of 6 week old, soil-grown, *Nicotiana benthamiana* plants as described previously (Green, et al., Biotechnol. J. 4: 1-8, 2009).

Plant tissue samples were taken from 3-7 days post-infiltration for determination of the levels of expression and solubility of the fusion protein. Samples were weighed and extracted in three volumes of extraction buffer (100mM Na₂HPO₄, pH 7.1; 2.5mM EDTA, pH 8.0) for total soluble protein, extraction buffer plus 0.5% Triton-X 100 for total soluble protein-Triton, or gel loading buffer for total protein. Proteins were resolved on 10% SDS-PAGE gels and transferred to PVDF membranes. Levels of fusion protein were assessed by immunoblot analysis with anti-AIMV rabbit polyclonal antibody compared with an AIMV standard or with a target specific antibody and appropriate standard. A representative immunoblot is shown in Figure 4C. Peak days of expression and solubility profiles were determined. Constructs of interest were vacuum infiltrated on a larger scale into hydroponically grown *N. benthamiana* for expression of the fusion proteins.

### Example 3. Isolation and purification of virus like particles

On the day of maximum expression, leaves were harvested and homogenized in a blender in three volumes of phosphate-based extraction buffer with 0.5% Triton X-100. The homogenate was stirred for 30 minutes at 4°C, then centrifuged for 30 minutes at 5,000 x g. The supernatant was filtered through miracloth and centrifuged at 15,000 x g for 1 to 1.5 hours. The supernatant was precipitated with PEG and then again centrifuged for 30 min at 15,000 x g. The pellet was resuspended in a phosphate based buffer and frozen at -20°C. After thawing, the suspension was centrifuged 30 minutes at 30,000 x g. Aliquots of supernatant were analyzed by SDS-PAGE to estimate protein concentration. Supernatant was centrifuged for 2h at 60,000 x g in a Ti70 rotor. The pellet was resuspended in phosphate-based buffer. The resulting supernatant was analyzed by SDS-PAGE and Coomassie blue staining. Protein concentration was determined colorimetrically by comparison with BSA standards.

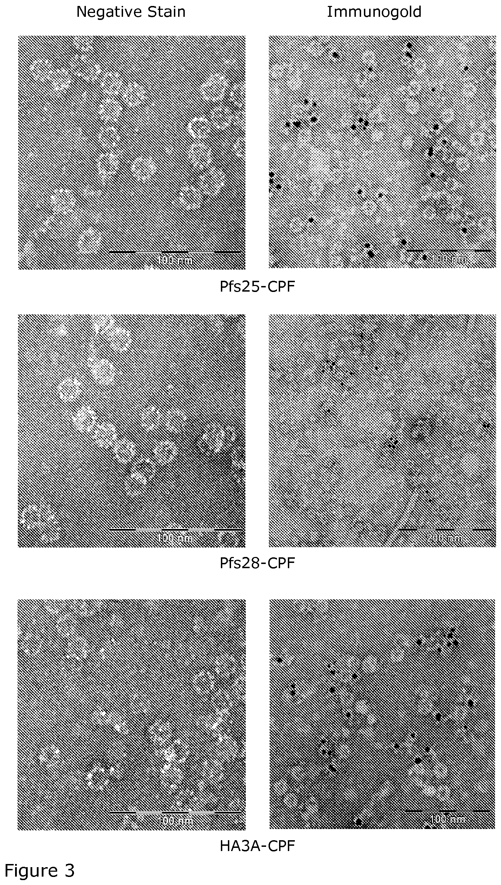

Purified particles were visualized by TEM and negative staining (Figure 3). The particles appear to be empty and substantially free of nucleic acid. Immuno-gold labeling with target-specific antibodies confirmed the presence of the target protein on the surface of the particles. Purified particles were stored in a buffer solution of 10mM Na₂HPO₄, pH 7.1; 1mM EDTA, pH 8.0, at -80°C.

The purified proteins were subjected to immunoblotting as described in Example 3. On immunoblots having the purified particles, the fusion protein was recognized by both the target specific antibody and by the AIMV specific antibody, which binds to the coat protein. (Figure 4A and 4B).

Expression results of fusion proteins produced in leaves are summarized in Table 1. Expression and purification amounts were sufficiently high to allow testing for immunogenicity In mice.

**Table 1. Protein expression from fusion constructs infiltrated into plant leaves**

| **Fusion construct** | **Target SEQ ID No.** | **Target protein** | **Target protein size in amino acids** | **Total Protein Expression (mg/kg)** |
|---|---|---|---|---|
| pGR-D4-PR-Pfs25-CPF | 4 | *P. falciparum* sexual stage protein, Pfs25 | 171 | 130 |
| pBI-PR-Pfs25-CPF | 4 | *P. falciparum* sexual stage protein, Pfs25 | 171 | 174 |
| pGR-D4-Pfs28-CPF | 5 | *P. falciparum* sexual stage protein, Pfs28 | 175 | 101 |
| pGR-D4-PR-Pfs28-CPF | 5 | *P. falciparum* sexual stage protein, Pfs28 | 175 | 79 |
| pBI-PR-Pfs28-CPF | 5 | *P. falciparum* sexual stage protein, Pfs28 | 175 | 100 |
| pBI-PR-HA3A-CPF | 6 | Hemagglutinin globular domain A/Anhui/1/2005(H5N1) | 233 | 75 |
| pGR-D4-PR-HA3A-CPF | 6 | Hemagglutinin globular domain A/Anhui/1/2005(H5N1) | 233 | 60 |
| pGR-D4-PR-HA3C04-CPF | 7 | Hemagglutinin globular domain A/California/04/2009(H1N1) | 234 | 14 |
| pGR-D4-PR-HA3C06-CPF | 8 | Hemagglutinin globular domain, A/California/06/2009(H1N1) | 234 | 17 |
| pGR-D4-PR-HAA-CPF | 9 | Hemagglutinin A/Anhui/1/2005(H5N1) | 515 | 4 |
| pGR*-D4-PR-HA3I-CPF | 11 | Hemagglutinin globular domain A/Indonesia/5/2005(H5N1) | 233 | 4 |
| pGR-D4-PR-HA3I-CPO | 11 | Hemagglutinin, A/Indonesia/5/05(H5N1) | 233 | 24 |
| pGR-D4-PR-230AM-CPO | 49 | *P. falciparum* sexual stage protein, Pfs230 | 444 | 23 |

### Example 4. Immunization of mice with virus like particles

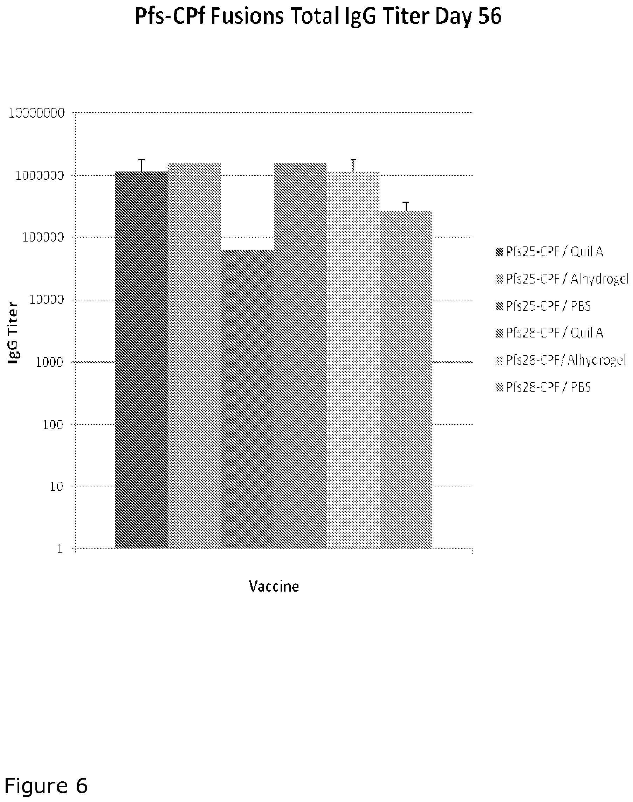

Groups of six, 6-8 weeks old Balb/c mice were immunized subcutaneously on study days 0 and 28 with 10µg of virus like particles containing Pfs25-CPF or Pfs28-CPF or 1µg of virus like particles containing HA3A-CPF. The vaccine was prepared just before using. Aliquots of purified virus like particles (or fusion protein particles) were thawed in buffer and injected with or without an adjuvant. The vaccine was injected in PBS alone or in the presence of either QUIL A™ (saponin based adjuvant, Brenntag Biosector, Frederikssund Denmark) or ALHYDROGEL™ (aluminum hydroxide based adjuvant, Brenntag Biosector, Frederikssund Denmark) as an adjuvant. The full length soluble HA from the Anhui strain of influenza virus (HAA1) was also injected with QUIL A™ as a positive control. Serum samples were collected on study day 56 and analyzed for antigen-specific IgG responses by ELISA. In the assay, plates were coated with 1 µg/ml of the following proteins: recombinant Pfs25 or Pfs28 produced in *Pichia pastoris,* or coated with inactivated A/Anhui/1/2005 reassortant virus obtained from the CDC at a 1:128 dilution. Serum samples were plated in 4-fold dilutions down the plate. End point titers of IgG were determined as the dilution at which the corresponding OD values are greater than four times the blank value. Results demonstrated that high antibody titers (>10,000) were elicited by Pfs25-CPF and Pfs28-CPF on Day 56 with or without an adjuvant (Figure 6). HA3A-CPF also elicited antigen-specific antibody titers in the presence or absence of an adjuvant (Figure 5A).

To test the functionality of the antibodies elicited by the HA3A-CPF vaccine, hemagglutination inhibition (HI) assays were performed as described in Yoko et al. (Vaccine 27: 3467-3470, 2009). Results showed that all mice immunized with HA3A-CPF in PBS or QUIL A™ had an HI titer of >1:40, a level considered protective in humans (Figure 5B). Four out of five animals immunized with HA3A-CPF vaccine adsorbed to ALHYDROGEL™ had an HI titer >1:40. Based on these results, HA3A-CPF appears to be a viable candidate for a pandemic influenza vaccine that can be delivered in the absence of an adjuvant.

Efficacy of the antibodies elicited by the Pfs25-CPF and Pfs28-CPF vaccines were evaluated using a different group of assays. IgG titers as determined by ELISA in sera collected on day 56 are shown in Figure 6. Additionally, these serum samples were sent to Radboud University, Nijmegen, The Netherlands, to assess the presence of gametocyte-binding antibodies. Initial testing of sera was done using an immunofluorescence assay (IFA) on fixed gametocytes where a titer of parasite binding antibodies in each serum sample was determined (Table 2). Positive sera from the IFA assay was then tested in a suspension IFA assay (SIFA) using live gametocytes at a serum dilution of 1:50 and 1:250. SIFA results are shown in Table 2.

**Table 2. Efficacy of Malaria CP-Fusion Vaccines**

| **Vaccine candidate** | **Adjuvant** | **Dose (µg)** | **IFA (Ab Titer)** | **SIFA (Ab Titer)** |
|---|---|---|---|---|
| Pfs25-CPF | Quil A | 10 | Positive (6400) | Very Positive (250) |
| Pfs25-CPF | Alhydrogel | 10 | Positive (6400) | Very Positive (250) |
| Pfs25-CPF | PBS | 10 | Positive (800) | Very Positive (250) |

The sera were then tested for the presence of transmission blocking antibodies in a standard membrane feeding assay (SMFA). The SMFA assay is the gold standard for measurement of transmission-blocking activity in test sera. The bioassay mimics the natural infection situation, and is therefore considered to be a valuable assay to measure transmission between man and mosquito. This assay enables transmission blocking vaccine (TBV) development to proceed with a confidence that is not available to most other malaria vaccines. Briefly, laboratory-reared *Anopheles stephensi* mosquitoes are allowed to take a blood meal from membrane-covered devices that contain serum from the above-immunized mice combined with complement and red blood cell suspensions infected with *P. falciparum* gametocytes. After one week, the number of infected mosquitoes (%InfMosq), as well as the number of developed oocysts per mosquito is determined. Transmission reducing activity is determined by comparing oocyst numbers in mosquitoes that are fed with test versus control (preimmune) serum. These results are shown in Table 3.

**Table 3. Standard Membrane Feeding Assay**

| **Vaccine candidate** | **Adjuvant** | **IFA Ab Titer** | **SIFA** | **SMFA** | | | | |
|---|---|---|---|---|---|---|---|---|
| - | - | - | - | No. mosqs | Total Oocysts | % InfMosq | AMO/ feeder | Pvalue |
| Pfs25-CPF | Quil A | Positive (6400) | Positive (250) | 20 | 0 | 0 | 0 | P<0.001 |
| Pfs25-CPF | Alhydrogel | Positive (6400) | Very Positive (250) | 20 | 0 | 0 | 0 | P<0.001 |
| Pfs25-CPF | PBS | Positive (800) | Positive (250) | 20 | 0 | 0 | 0 | P<0.001 |
| Pfs28-CPF | Quil A | Negative | Negative | 20 | 75 | 55 | 3.8 | P<0.01 |
| Pfs28-CPF | Alhydrogel | Negative | Negative | 20 | 104 | 75 | 5.2 | P>0.05 |
| Pfs28-CPF | PBS | Negative | ND | 20 | 151 | 75 | 7.6 | P>0.05 |
| Preimmune Sera GRP 1 | | | | 20 | 616 | 95 | 30.8 | ND |

| | | | | | | | | |
|---|---|---|---|---|---|---|---|---|
| Dose was 10ug for each vaccine candidate ND=not determined; AMO=arithmetic mean oocysts/feeder | | | | | | | | |

Analysis of the serum samples generated using the Pfs25-CPF demonstrated that high titers (1:6400) of parasite binding antibodies were detected in the adjuvanted groups with moderate titers (1:800) elicited by the vaccine alone. When analyzed on live parasites, all three Pfs25-CPF compositions, QUIL A™, ALHYDROGEL™, and PBS alone showed an antibody titer of 1:250. Similarly, when evaluated in the SMFA assay, all three Pfs25-CPF formulations elicited serum that completely blocked parasite transmission in the mosquitoes. These data confirm the potential of this approach in generating an efficacious vaccine against an infectious agent without the need for an adjuvant.

### Example 5. Preparation of adenovirus fusion protein constructs for transfection of mammalian cells.

For expression of the virus free particles (or fusion protein particles) composed of AIMV-CP-fusion proteins in a mammalian system, PCR techniques were used to fuse the *Gaussia* luciferase signal peptide to HA3A-CPF and HA3C04-CPF (globular domain (3) of HA from Anhui (SEQ ID NO:6), and California 04 (SEQ ID NO:7), respectively). The forward primers (SEQ ID NO:20 and 21) contained the TOPO integration sequence (CACC) and the nucleic acid sequence encoding the luciferase signal peptide and the start of the target protein. The reverse primer was specific to the AIMV-CP 3' terminus (SEQ ID NO: 22). The resulting amplicons were cloned into the pENTR-D-TOPO vector according to manufacturer's instructions (Invitrogen Corporation). Sequences were confirmed by automated sequencing. Following sequence verification, the target genes were recombined into the pAd/CMV/V5-DEST vector using the Gateway technology from Invitrogen Corporation according to the manufacturer's instructions.

### Example 6. Preparation of adenovirus containing a fusion protein gene

Purified pAd-HA3A-CPF or pAd-HA3C04-CPF plasmid was digested with *Pac*1 to expose the viral inverted terminal repeats (ITRs). The digested pAd-HA3A-CPF or pAd-HA3C04-CPF was transfected into the 293 cell line, which contains an integrated copy of E1 from adenovirus, using a cationic lipid-based transfection reagent. When the cytopathic effects of the virus were observed in transfected cells (10-14 days after transfection), adenovirus-containing cells and medium were harvested. The harvested cells and culture medium containing adenovirus were subjected to two rounds of freezing and thawing (at -80°C for 30 min and then at 37°C for 15 min) to lyse cells and allow the release of intracellular viral particles. After the second freeze/thaw cycle, the cell lysate was centrifuged and the supernatant containing viral particles was harvested and stored at -80°C.

### Example 7. Method for transduction of the adenoviral construct with HA3A-CPF or HA3C04-CPF sequences and production of the virus free particles

This is a prophetic example. The harvested adenovirus from Example 6 containing the HA3A-CPF or HA3C04-CPF gene will be amplified by infecting 293 cells, then again harvested and stored as described above to make an adenoviral stock. The adenoviral stock will be titrated using a plaque assay.

Since the pAd-HA3A-CPF and pAd-HA3C04-CPF adenoviral constructs are replication-incompetent and do not integrate into the host genome, no progeny virus will be produced after their transduction into mammalian cells. Host cells will be Madin-Darby canine kidney (MDCK) cells. MDCK cells will be adapted to grow in serum-free medium to avoid the possibility of animal by-product contamination in the final preparation. MDCK cells will be transduced with adenovirus-containing HA3A-CPF or HA3C04-CPF sequences to determine a suitable multiplicity of infection. Two or three days after transduction, the expression of HA3A-CPF or HA3C04-CPF will be analyzed by fluorescence microscopy using anti-HA and anti-AIMV antibodies followed by FITC-conjugated anti-mouse IgG. Alternatively, cell culture supernatant will be collected and concentrated for immunoblotting and detection of fusion proteins.

To obtain a scalable mammalian cell production platform for HA3A-CPF or HA3C04-CPF, MDCK cells will be cultured in suspension using a microcarrier system based on cross-linked dextran, e.g., CYTODEX™ 1 from GE Healthcare. After transduction of the adenovirus construct, MDCK cells will be incubated with the microcarrier and cultured in spinner flasks. After two to three days, samples of cell supernatants will be collected on a daily basis and expression levels will be analyzed by immunoblotting using a target specific anti-HA monoclonal antibody.

When the presence of fusion proteins is confirmed, the MDCK culture medium will be collected and centrifuged. Fusion proteins will be purified from the supernatant using the procedures described in Example 3, beginning with PEG precipitation.

### Example 8. Immunization of mice with virus like particles containing globular domain of H5 HA (A/Indonesia/5/2005) AMV CP fusion

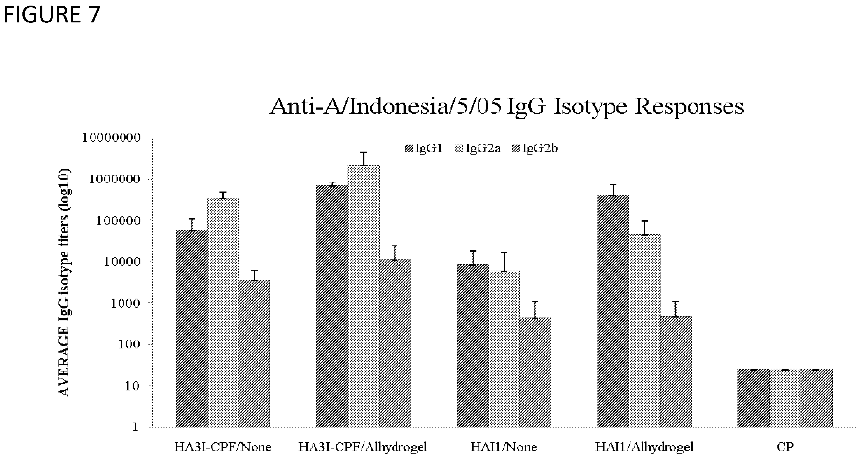

Groups of six, 6-8 weeks old Balb/c mice were immunized subcutaneously on study days 0 and 21 with 5 µg of virus like particles (VLPs) containing globular domain of H5 HA (A/Indonesia/5/2005) AMV CP fusion (HA3I-CPF). The full length soluble HA from the Indonesia strain of influenza virus (HAI1) was also injected with or without ALHYDROGEL™ as comparators. CP only was included as a negative control. The vaccine was prepared just before using. Aliquots of purified virus like particles (or fusion protein particles) were thawed in buffer and injected with or without an adjuvant, ALHYDROGEL™. Serum samples were collected on study day 35 and analyzed for antigen-specific IgG isotope responses by ELISA. In the assay, plates were coated with inactivated A/Indonesia/5/2005 reassortant virus obtained from the CDC at a 1:128 dilution. Serum samples were plated in 4-fold serial dilutions down the plate. End point titers of IgG1, IgG2a and IgG2b were determined as the dilution at which the corresponding OD values are greater than three times the negative control value. Results demonstrated that virus like particles containing HA3I-CPF induced stronger IgG2a response in the presence or absence of ALHYDROGEL™ than the soluble subunit alone (HAI1) in the presence or absence of ALHYDROGEL™ (Figure 7) suggesting Th1 bias of immune response to VLPs. As mice infected with influenza have been shown to generate Th1 immune responses, plant-produced influenza HA globular domain AMV-based VLPs are better vaccine candidates than the soluble subunit.

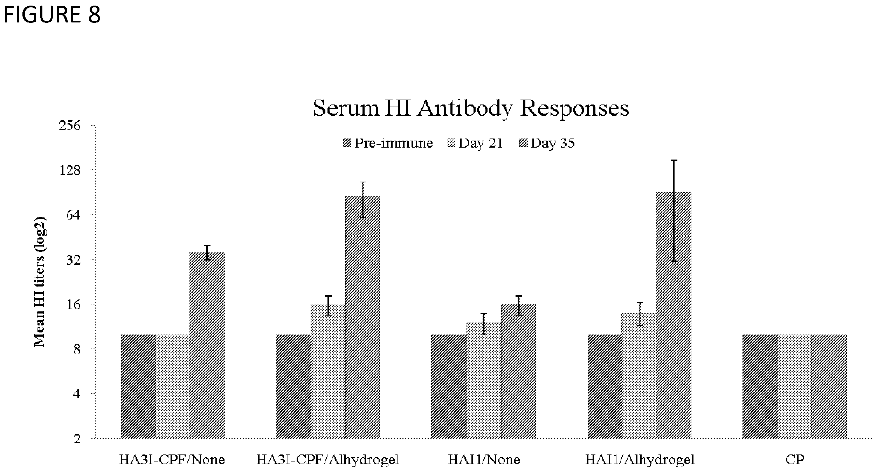

To test the functionality of the antibodies elicited by the HA3I-CPF vaccine, hemagglutination inhibition (HI) assays were performed as described in Yoko et al. (Vaccine 27: 3467-3470, 2009). Results showed that 80% of the mice 35 days after being immunized with HA3I-CPF in the absence of ALHYDROGEL™ had an HI titer of >1:40, which is significantly higher than the mice immunized with HAI1 (p = 0.003) (Figure 8), indicating that HA3I-CPF is a better influenza vaccine candidate than the soluble subunit for delivery without an adjuvant.

### Example 9. Effects of pre-existing immunity against CP on serum antibody responses in mice

Groups of six, 6-8 weeks old Balb/c mice were immunized subcutaneously (SC) or intramuscularly (IM) with PBS or 10 µg of virus like particles (VLPs) containing Pfs25-CPF on study days 0 and 28 with or without an adjuvant, ALHYDROGEL™ (Alum), and subsequently with PBS or 1 µg of VLPs containing globular domain of H5 HA (A/Anhui/1/2005) AMV CP fusion (HA3I-CPF) via the same route of administration on study days 140 and 168 with or without ALHYDROGEL™ (Alum).

The vaccine was prepared just before using. Aliquots of purified virus like particles (or fusion protein particles) were thawed in buffer and injected with or without an adjuvant, ALHYDROGEL™. Serum samples were collected before the study (Pre-bleed) and on study days 28, 56, 84, 112, 140, 168 and 216, and analyzed for antigen-specific IgG responses by ELISA. In the assay, plates were coated with 2 µg/ml AIMV, 1 µg/ml of recombinant Pfs25 produced in *Pichia pastoris,* or inactivated A/Anhui/1/2005 reassortant virus obtained from the CDC at a 1:128 dilution. Serum samples were plated in 4-fold dilutions down the plate. End point titers of IgG were determined as the dilution at which the corresponding OD values are greater than four times the blank value.

Two immunizations with Pfs25-CPF, either with or without an adjuvant, elicited high IgG antibody titers (>10,000) against AIMV (CP) and those titers were maintained throughout the course of the study. After immunizations with HA3A-CPF, either with or without an adjuvant, the IgG titers against AIMV (CP) in Pfs25-CPF pre-immunized group slightly increased while the IgG titers in PBS pre-immunized groups reached the equivalent levels to those in the Pfs25-CPF pre-immunized groups.

Two immunizations with Pfs25-CPF, either with or without an adjuvant, elicited high anti-Pfs25 IgG antibody titers (>10,000), which were maintained and did not significantly change after immunizations with Pfs25-CPF.

Two immunizations with Pfs25-CPF, by either the SC or IM route, elicited significant anti-A/Anhui/1/2005 IgG responses in sera from all groups except the groups pre-immunized with PBS in which the titers were one or two log lower than those in other groups (>10,000).

To test the functionality of the antibodies elicited by the HA3A-CPF vaccine, hemagglutination inhibition (HI) assays were performed as described in Yoko et al. (Vaccine 27: 3467-3470, 2009). Results showed that two immunizations with HA3A-CPF induced serum HI antibody responses in all groups except for the negative control (PBS/PBS) group (Figures 9A and 9B). Without an adjuvant, significant differences in HI titers between groups pre-immunized with Pfs25-CPF and PBS, either via SC (*p* = 0.01) or IM (*p* = 0.08) routes, were observed. With an adjuvant, there were no significant differences observed between groups pre-immunized with Pfs25-CPF and PBS via SC (*p* = 0.5) or IM (*p* = 0.6). Based on these results, without ALHYDROGEL™ as an adjuvant, pre-immunization with VLPs containing a fusion protein of a plant viral coat protein and a first target protein did not interfere with serum HI antibody responses to a second target protein in mice immunized with VLPs containing a fusion protein of the same plant viral coat protein and the second target protein, via SC or IM routes.

Although the invention is illustrated and described herein with reference to specific embodiments, the invention is not intended to be limited to the details shown. Rather, various modifications may be made in the details within the scope and range of equivalents of the claims and without departing from the invention.
## Claims

1. A virus like particle consisting of a fusion protein, wherein the fusion protein comprises (a) a protein having an amino acid sequence at least 80% identical to that of a coat protein of alfalfa mosaic virus, wherein the coat protein has an amino acid sequence of SEQ ID NO: 1, and (b) a target protein, wherein the target protein is derived from a surface protein from an intracellular pathogenic organism that is suitable for use in vaccines, and wherein the virus like particle contains less than 10% of nucleic acid by weight.

2. The virus like particle of claim 1, wherein the intracellular pathogenic organism is selected from the group consisting of bacteria, viruses, fungi, and parasitic organisms.

3. The virus like particle of claim 1 or 2, wherein the target protein is a cell surface polypeptide of *Plasmodium falciparum* or an antigenic fragment thereof.

4. The virus like particle of claim 1 or 2, wherein the target protein is a polypeptide of hemagglutinin of an influenza virus or an antigenic fragment thereof.

5. The virus like particle of claim 4, wherein the influenza virus is an influenza A virus selected from the group consisting of H1N1, H3N2, H5N1, and H7N7.

6. The virus like particle of claim 4, wherein the influenza virus is an influenza B virus.

7. The virus like particle of any one of claims 1-6, wherein the target protein comprises an amino acid sequence selected from the group consisting of SEQ ID NOs: 4-11 and 49, and antigenic fragments thereof.

8. The virus like particle of any one of claims 1-7, wherein the target protein is fused to the N-terminus of the coat protein.

9. The virus like particle of any one of claims 1-8, wherein the virus like particle is produced in a host cell selected from the group consisting of bacterial, fungal, plant, insect, amphibian, and mammalian cells.

10. An immunogenic composition comprising the virus like particle of any one of claims 1-9.

11. The immunogenic composition of claim 10, further comprising an adjuvant.

12. The immunogenic composition of claim 11, wherein the adjuvant is selected from the group consisting of aluminum salts, oil and water emulsions, and saponin-based adjuvants.

13. A virus like particle according to any of claims 1 to 9 for use in inducing a protective immune response against an intracellular pathogenic organism in a subject, comprising an immunologically effective amount of a composition comprising the virus like particle of any one of claims 1-9.

## Patentansprüche

1. Virusähnliches Partikel, bestehend aus einem Fusionsprotein, wobei das Fusionsprotein Folgendes umfasst: (a) ein Protein mit einer Aminosäuresequenz, die wenigstens 80 % identisch mit der eines Hüllproteins des Alfalfa-Mosaikvirus ist, wobei das Hüllprotein eine Aminosäuresequenz von SEQ ID NO: 1 hat, und (b) ein Zielprotein, wobei das Zielprotein von einem Oberflächenprotein eines intrazellulären pathogenen Organismus abgeleitet ist, der zur Verwendung in Impfstoffen geeignet ist, und wobei das virusähnliche Partikel weniger als 10 Gew.-% Nucleinsäure enthält.

2. Virusähnliches Partikel nach Anspruch 1, wobei der intrazelluläre pathogene Organismus ausgewählt ist aus der Gruppe bestehend aus Bakterien, Viren, Pilzen und parasitären Organismen.

3. Virusähnliches Partikel nach Anspruch 1 oder 2, wobei das Zielprotein ein Zelloberflächenpolypeptid von *Plasmodium falciparum* oder ein antigenes Fragment davon ist.

4. Virusähnliches Partikel nach Anspruch 1 oder 2, wobei das Zielprotein ein Polypeptid von Hämagglutinin eines Influenzavirus oder ein antigenes Fragment davon ist.

5. Virusähnliches Partikel nach Anspruch 4, wobei das Influenzavirus ein Influenza-A-Virus ist, ausgewählt aus der Gruppe bestehend aus H1N1, H3N2, H5N1 und H7N7.

6. Virusähnliches Partikel nach Anspruch 4, wobei das Influenzavirus ein Influenza-B-Virus ist.

7. Virusähnliches Partikel nach einem der Ansprüche 1-6, wobei das Zielprotein eine Aminosäuresequenz, ausgewählt aus der Gruppe bestehend aus SEQ ID NO: 4-11 und 49, und antigene Fragmente davon umfasst.

8. Virusähnliches Partikel nach einem der Ansprüche 1-7, wobei das Zielprotein mit dem N-Terminus des Hüllproteins fusioniert ist.

9. Virusähnliches Partikel nach einem der Ansprüche 1-8, wobei das virusähnliche Partikel in einer Wirtszelle produziert wird, die ausgewählt ist aus der Gruppe bestehend aus Bakterien-, Pilz-, Pflanzen-, Insekten-, Amphibien- und Säugetierzellen.

10. Immunogene Zusammensetzung, die das virusähnliche Partikel nach einem der Ansprüche 1-9 umfasst.

11. Immunogene Zusammensetzung nach Anspruch 10, die ferner ein Adjuvans umfasst.

12. Immunogene Zusammensetzung nach Anspruch 11, wobei das Adjuvans ausgewählt ist aus der Gruppe bestehend aus Aluminiumsalzen, Öl- und Wasseremulsionen und Saponin-basierten Adjuvanzien.

13. Virusähnliches Partikel nach einem der Ansprüche 1 bis 9 zur Verwendung beim Induzieren einer schützenden Immunreaktion auf einen intrazellulären pathogenen Organismus in einem Subjekt, umfassend eine immunologisch wirksame Menge einer Zusammensetzung, die das virusähnliche Partikel nach einem der Ansprüche 1-9 umfasst.

## Revendications

1. Pseudo-particule virale consistant en une protéine de fusion, où la protéine de fusion comprend (a) une protéine ayant une séquence d'acides aminés au moins 80 % identique à celle d'une protéine de la capside du virus de la mosaïque de la luzerne, où la protéine de la capside a une séquence d'acides aminés SEQ ID N° : 1, et (b) une protéine cible, où la protéine cible est dérivée d'une protéine de surface d'un organisme pathogène intracellulaire qui se prête à une utilisation dans des vaccins, et où la pseudo-particule virale contient moins de 10 % en poids d'acide nucléique.

2. Pseudo-particule virale de la revendication 1, où l'organisme pathogène intracellulaire est sélectionné dans le groupe consistant en des organismes bactériens, viraux, fongiques et parasites.

3. Pseudo-particule virale de la revendication 1 ou 2, où la protéine cible est un polypeptide de surface cellulaire de *Plasmodium falciparum* ou un fragment antigénique de celui-ci.

4. Pseudo-particule virale de la revendication 1 ou 2, où la protéine cible est un polypeptide de l'hémagglutinine d'un virus de la grippe ou un fragment antigénique de celui-ci.

5. Pseudo-particule virale de la revendication 4, où le virus de grippe est un virus de la grippe A sélectionné dans le groupe consistant en H1N1, H3N2, H5N1 et H7N7.

6. Pseudo-particule virale de la revendication 4, où le virus de grippe est un virus de la grippe B.

7. Pseudo-particule virale de l'une quelconque des revendications 1-6, où la protéine cible comprend une séquence d'acides aminés sélectionnée dans le groupe consistant en les SEQ ID N° : 4-11 et 49 et des fragments antigéniques de celles-ci.

8. Pseudo-particule virale de l'une quelconque des revendications 1-7, où la protéine cible est fusionnée au N-terminal de la protéine de la capside.

9. Pseudo-particule virale de l'une quelconque des revendications 1-8, où la pseudo-particule virale est produite dans une cellule hôte sélectionnée dans le groupe consistant en des cellules bactériennes, fongiques, végétales, d'insectes, d'amphibiens et de mammifères.

10. Composition immunogène comprenant la pseudo-particule virale de l'une quelconque des revendications 1-9.

11. Composition immunogène de la revendication 10, qui comprend en outre un adjuvant.

12. Composition immunogène de la revendication 11, où l'adjuvant est sélectionné dans le groupe consistant en des sels d'aluminium, des émulsions huile et eau et des adjuvants à base de saponine.

13. Pseudo-particule virale selon l'une quelconque des revendications 1 à 9 pour une utilisation dans l'induction d'une réponse immune protectrice contre un organisme pathogène intracellulaire chez un sujet, comprenant une quantité immunologiquement efficace d'une composition qui comprend la pseudo-particule virale de l'une quelconque des revendications 1-9.


<110> Fraunhofer USA Inc.
<120> VIRUS LIKE PARTICLES COMPRISING TARGET PROTEINS FUSED TO PLANT VIRAL COAT PROTEINS
<130> FCMB-100WO
<160> 49
<210> 1
   <211> 220
   <212> PRT
   <213> Alfalfa mosaic virus
<220>
   <223> A1MV coat protein
<300>
   <308> NP_041195
<400> 1
   Ser Ser Ser Gln Lys Lys Ala Gly Gly Lys Ala Gly Lys Pro Thr Lys
   1               5                   10                  15
   Arg Ser Gln Asn Tyr Ala Ala Leu Arg Lys Ala Gln Leu Pro Lys Pro
               20                  25                  30
   Pro Ala Leu Lys Val Pro Val Val Lys Pro Thr Asn Thr Ile Leu Pro
           35                  40                  45
   Gln Thr Gly Cys Val Trp Gln Ser Leu Gly Thr Pro Leu Ser Leu Ser
       50                  55                  60
   Ser Phe Asn Gly Leu Gly Val Arg Phe Leu Tyr Ser Phe Leu Lys Asp
   65                  70                  75                  80
   Phe Ala Gly Pro Arg Ile Leu Glu Glu Asp Leu Ile Tyr Arg Met Val
                   85                  90                  95
   Phe Ser Ile Thr Pro Ser Tyr Ala Gly Thr Phe Cys Leu Thr Asp Asp
               100                 105                 110
   Val Thr Thr Glu Asp Gly Arg Ala Val Ala His Gly Asn Pro Met Gln
           115                 120                 125
   Glu Phe Pro His Gly Ala Phe His Ala Asn Glu Lys Phe Gly Phe Glu
       130                 135                 140
   Leu Val Phe Thr Ala Pro Thr His Ala Gly Met Gln Asn Gln Asn Phe
   145                 150                 155                 160
   Lys His Ser Tyr Ala Val Ala Leu Cys Leu Asp Phe Asp Ala Gln Pro
   165 170 175
   Glu Gly Ser Lys Asn Pro Ser Tyr Arg Phe Asn Glu Val Trp Val Glu
   180 185 190
   Arg Lys Ala Phe Pro Arg Ala Gly Pro Leu Arg Ser Leu Ile Thr Val
   195 200 205
   Gly Leu Phe Asp Glu Ala Asp Asp Leu Asp Arg His
   210 215 220
<210> 2
   <211> 196
   <212> PRT
   <213> Alfalfa mosaic virus
<220>
   <223> A1MV coat protein truncated
<400> 2
   Arg Lys Ala Gln Leu Pro Lys Pro Pro Ala Leu Lys Val Pro Val Val
   1               5                   10                  15
   Lys Pro Thr Asn Thr Ile Leu Pro Gln Thr Gly Cys Val Trp Gln Ser
               20                  25                  30
   Leu Gly Thr Pro Leu Ser Leu Ser Ser Phe Asn Gly Leu Gly Val Arg
           35                  40                  45
   Phe Leu Tyr Ser Phe Leu Lys Asp Phe Ala Gly Pro Arg Ile Leu Glu
       50                  55                  60
   Glu Asp Leu Ile Tyr Arg Met Val Phe Ser Ile Thr Pro Ser Tyr Ala
   65                  70                  75                  80
   Gly Thr Phe Cys Leu Thr Asp Asp Val Thr Thr Glu Asp Gly Arg Ala
                   85                  90                  95
   Val Ala His Gly Asn Pro Met Gln Glu Phe Pro His Gly Ala Phe His
               100                 105                 110
   Ala Asn Glu Lys Phe Gly Phe Glu Leu Val Phe Thr Ala Pro Thr His
           115                 120                 125
   Ala Gly Met Gln Asn Gln Asn Phe Lys His Ser Tyr Ala Val Ala Leu
       130                 135                 140
   Cys Leu Asp Phe Asp Ala Gln Pro Glu Gly Ser Lys Asn Pro Ser Tyr
   145                 150                 155                 160
   Arg Phe Asn Glu Val Trp Val Glu Arg Lys Ala Phe Pro Arg Ala Gly
                   165                 170                 175
   Pro Leu Arg Ser Leu Ile Thr Val Gly Leu Phe Asp Glu Ala Asp Asp
               180                 185                 190
   Leu Asp Arg His
           195
<210> 3
   <211> 30
   <212> PRT
   <213> Nicotiana tabacum
<220>
   <223> Signal peptide, plant pathogenesis-related protein 1
<400> 3
   Met Gly Phe Val Leu Phe Ser Gln Leu Pro Ser Phe Leu Leu Val Ser
   1               5                   10                  15
   Thr Leu Leu Leu Phe Leu Val Ile Ser His Ser Cys Arg Ala
               20                  25                  30
<210> 4
   <211> 171
   <212> PRT
   <213> Plasmodium falciparum
<220>
   <223> Sexual stage cell surface protein, Pfs-25
<300>
   <308> AAF63684.1
<400> 4
   Lys Val Thr Val Asp Thr Val Cys Lys Arg Gly Phe Leu Ile Gln Met
   1               5                   10                  15
   Ser Gly His Leu Glu Cys Lys Cys Glu Asn Asp Leu Val Leu Val Asn
               20                  25                  30
   Glu Glu Thr Cys Glu Glu Lys Val Leu Lys Cys Asp Glu Lys Thr Val
           35                  40                  45
   Asn Lys Pro Cys Gly Asp Phe Ser Lys Cys Ile Lys Ile Asp Gly Asn
       50                  55                  60
   Pro Val Ser Tyr Ala Cys Lys Cys Asn Leu Gly Tyr Asp Met Val Asn
   65                  70                  75                  80
   Asn Val Cys Ile Pro Asn Glu Cys Lys Asn Val Thr Cys Gly Asn Gly
                   85                  90                  95
   Lys Cys Ile Leu Asp Thr Ser Asn Pro Val Lys Thr Ala Val Cys Ser
   100 105 110
   Cys Asn Ile Gly Lys Val Pro Asn Val Gln Asp Gln Asn Lys Cys Ser
   115 120 125
   Lys Asp Gly Glu Thr Lys Cys Ser Leu Lys Cys Leu Lys Glu Asn Glu
   130 135 140
   Thr Cys Lys Ala Val Asp Gly Ile Tyr Lys Cys Asp Cys Lys Asp Gly
   145 150 155 160
   Phe Ile Ile Asp Asn Glu Ser Ser Ile Cys Thr
   165 170
<210> 5
   <211> 175
   <212> PRT
   <213> Plasmodium falciparum
<220>
   <223> Sexual stage cell surface protein, Pfs28
<300>
   <308> AAT00624.1
<400> 5
   Arg Val Thr Glu Asn Thr Ile Cys Lys Tyr Gly Tyr Leu Ile Gln Met
   1               5                   10                  15
   Ser Asn His Tyr Glu Cys Lys Cys Ile Glu Gly Tyr Val Leu Ile Asn
               20                  25                  30
   Glu Asp Thr Cys Gly Lys Lys Val Val Cys Asp Lys Val Glu Asn Ser
           35                  40                  45
   Phe Lys Ala Cys Asp Glu Tyr Ala Tyr Cys Phe Asp Leu Gly Asn Lys
       50                  55                  60
   Asn Asn Glu Lys Gln Ile Lys Cys Met Cys Arg Thr Glu Tyr Thr Leu
   65                  70                  75                  80
   Thr Ala Gly Val Cys Val Pro Asn Val Cys Arg Asp Lys Val Cys Gly
                   85                  90                  95
   Lys Gly Lys Cys Ile Val Asp Pro Ala Asn Ser Leu Thr His Thr Cys
               100                 105                 110
   Ser Cys Asn Ile Gly Thr Ile Leu Asn Gln Asn Lys Leu Cys Asp Ile
           115                 120                 125
   Gln Gly Asp Thr Pro Cys Ser Leu Lys Cys Ala Glu Asn Glu Val Cys
       130                 135                 140
   Thr Leu Glu Gly Asn Tyr Tyr Thr Cys Lys Glu Asp Pro Ser Ser Asn
   145                 150                 155                 160
   Gly Gly Gly Asn Thr Val Asp Gln Ala Asp Thr Ser Tyr Ser Val
                   165                 170                 175
<210> 6
   <211> 233
   <212> PRT
   <213> Influenza A
<220>
   <223> Hemagglutinin, globular domain, Anhui/1/2005/H5N1, HA3A
<400> 6
   Cys Asp Leu Asp Gly Val Lys Pro Leu Ile Leu Arg Asp Cys Ser Val
   1               5                   10                  15
   Ala Gly Trp Leu Leu Gly Asn Pro Met Cys Asp Glu Phe Ile Asn Val
               20                  25                  30
   Pro Glu Trp Ser Tyr Ile Val Glu Lys Ala Asn Pro Ala Asn Asp Leu
           35                  40                  45
   Cys Tyr Pro Gly Asn Phe Asn Asp Tyr Glu Glu Leu Lys His Leu Leu
       50                  55                  60
   Ser Arg Ile Asn His Phe Glu Lys Ile Gln Ile Ile Pro Lys Ser Ser
   65                  70                  75                  80
   Trp Ser Asp His Glu Ala Ser Ser Gly Val Ser Ser Ala Cys Pro Tyr
                   85                  90                  95
   Gln Gly Thr Pro Ser Phe Phe Arg Asn Val Val Trp Leu Ile Lys Lys
               100                 105                 110
   Asn Asn Thr Tyr Pro Thr Ile Lys Arg Ser Tyr Asn Asn Thr Asn Gln
           115                 120                 125
   Glu Asp Leu Leu Ile Leu Trp Gly Ile His His Ser Asn Asp Ala Ala
       130                 135                 140
   Glu Gln Thr Lys Leu Tyr Gln Asn Pro Thr Thr Tyr Ile Ser Val Gly
   145                 150                 155                 160
   Thr Ser Thr Leu Asn Gln Arg Leu Val Pro Lys Ile Ala Thr Arg Ser
                   165                 170                 175
   Lys Val Asn Gly Gln Ser Gly Arg Met Asp Phe Phe Trp Thr Ile Leu
               180                 185                 190
   Lys Pro Asn Asp Ala Ile Asn Phe Glu Ser Asn Gly Asn Phe Ile Ala
           195                 200                 205
   Pro Glu Tyr Ala Tyr Lys Ile Val Lys Lys Gly Asp Ser Ala Ile Val
       210                 215                 220
   Lys Ser Glu Val Glu Tyr Gly Asn Cys
   225                 230
<210> 7
   <211> 234
   <212> PRT
   <213> Influenza A
<220>
   <223> Hemagglutinin, globular domain, California/04/2009/H1N1, HA3C04
<400> 7
   Cys Lys Leu Arg Gly Val Ala Pro Leu His Leu Gly Lys Cys Asn Ile
   1               5                   10                  15
   Ala Gly Trp Ile Leu Gly Asn Pro Glu Cys Glu Ser Leu Ser Thr Ala
               20                  25                  30
   Ser Ser Trp Ser Tyr Ile Val Glu Thr Pro Ser Ser Asp Asn Gly Thr
           35                  40                  45
   Cys Tyr Pro Gly Asp Phe Ile Asp Tyr Glu Glu Leu Arg Glu Gln Leu
       50                  55                  60
   Ser Ser Val Ser Ser Phe Glu Arg Phe Glu Ile Phe Pro Lys Thr Ser
   65                  70                  75                  80
   Ser Trp Pro Asn His Asp Ser Asn Lys Gly Val Thr Ala Ala Cys Pro
                   85                  90                  95
   His Ala Gly Ala Lys Ser Phe Tyr Lys Asn Leu Ile Trp Leu Val Lys
               100                 105                 110
   Lys Gly Asn Ser Tyr Pro Lys Leu Ser Lys Ser Tyr Ile Asn Asp Lys
           115                 120                 125
   Gly Lys Glu Val Leu Val Leu Trp Gly Ile His His Pro Ser Thr Ser
       130                 135                 140
   Ala Asp Gln Gln Ser Leu Tyr Gln Asn Ala Asp Thr Tyr Val Phe Val
   145                 150                 155                 160
   Gly Ser Ser Arg Tyr Ser Lys Lys Phe Lys Pro Glu Ile Ala Ile Arg
                   165                 170                 175
   Pro Lys Val Arg Asp Gln Glu Gly Arg Met Asn Tyr Tyr Trp Thr Leu
               180                 185                 190
   Val Glu Pro Gly Asp Lys Ile Thr Phe Glu Ala Thr Gly Asn Leu Val
           195                 200                 205
   Val Pro Arg Tyr Ala Phe Ala Met Glu Arg Asn Ala Gly Ser Gly Ile
       210                 215                 220
   Ile Ile Ser Asp Thr Pro Val His Asp Cys
   225                 230
<210> 8
   <211> 234
   <212> PRT
   <213> Influenza A
<220>
   <223> Hemagglutinin, globular domain, California/06/2009/H1N1, HA3C06
<400> 8
   Cys Lys Leu Arg Gly Val Ala Pro Leu His Leu Gly Lys Cys Asn Ile
   1               5                   10                  15
   Ala Gly Trp Ile Leu Gly Asn Pro Glu Cys Glu Ser Leu Ser Thr Ala
               20                  25                  30
   Ser Ser Trp Ser Tyr Ile Val Glu Thr Ser Ser Ser Asp Asn Gly Thr
           35                  40                  45
   Cys Tyr Pro Gly Asp Phe Ile Asp Tyr Glu Glu Leu Arg Glu Gln Leu
       50                  55                  60
   Ser Ser Val Ser Ser Phe Glu Arg Phe Glu Ile Phe Pro Lys Thr Ser
   65                  70                  75                  80
   Ser Trp Pro Asn His Asp Ser Asn Lys Gly Val Thr Ala Ala Cys Pro
                   85                  90                  95
   His Ala Gly Ala Lys Ser Phe Tyr Lys Asn Leu Ile Trp Leu Val Lys
               100                 105                 110
   Lys Gly Asn Ser Tyr Pro Lys Leu Ser Lys Ser Tyr Ile Asn Asp Lys
           115                 120                 125
   Gly Lys Glu Val Leu Val Leu Trp Gly Ile His His Pro Ser Thr Ser
       130                 135                 140
   Ala Asp Gln Gln Ser Leu Tyr Gln Asn Ala Asp Ala Tyr Val Phe Val
   145                 150                 155                 160
   Gly Ser Ser Arg Tyr Ser Lys Lys Phe Lys Pro Glu Ile Ala Ile Arg
                   165                 170                 175
   Pro Lys Val Arg Asp Gln Glu Gly Arg Met Asn Tyr Tyr Trp Thr Leu
               180                 185                 190
   Val Glu Pro Gly Asp Lys Ile Thr Phe Glu Ala Thr Gly Asn Leu Val
           195                 200                 205
   Val Pro Arg Tyr Ala Phe Ala Met Glu Arg Asn Ala Gly Ser Gly Ile
       210                 215                 220
   Ile Ile Ser Asp Thr Pro Val His Asp Cys
   225                 230
<210> 9
   <211> 515
   <212> PRT
   <213> Influenza A
<220>
   <223> Hemagglutinin, Anhui/1/2005/H5N1, HAA
<300>
   <308> ABD28180
<400> 9
   Asp Gln Ile Cys Ile Gly Tyr His Ala Asn Asn Ser Thr Glu Gln Val
   1               5                   10                  15
   Asp Thr Ile Met Glu Lys Asn Val Thr Val Thr His Ala Gln Asp Ile
               20                  25                  30
   Leu Glu Lys Thr His Asn Gly Lys Leu Cys Asp Leu Asp Gly Val Lys
           35                  40                  45
   Pro Leu Ile Leu Arg Asp Cys Ser Val Ala Gly Trp Leu Leu Gly Asn
       50                  55                  60
   Pro Met Cys Asp Glu Phe Ile Asn Val Pro Glu Trp Ser Tyr Ile Val
   65                  70                  75                  80
   Glu Lys Ala Asn Pro Ala Asn Asp Leu Cys Tyr Pro Gly Asn Phe Asn
                   85                  90                  95
   Asp Tyr Glu Glu Leu Lys His Leu Leu Ser Arg Ile Asn His Phe Glu
               100                 105                 110
   Lys Ile Gln Ile Ile Pro Lys Ser Ser Trp Ser Asp His Glu Ala Ser
           115                 120                 125
   Ser Gly Val Ser Ser Ala Cys Pro Tyr Gln Gly Thr Pro Ser Phe Phe
       130                 135                 140
   Arg Asn Val Val Trp Leu Ile Lys Lys Asn Asn Thr Tyr Pro Thr Ile
   145                 150                 155                 160
   Lys Arg Ser Tyr Asn Asn Thr Asn Gln Glu Asp Leu Leu Ile Leu Trp
                   165                 170                 175
   Gly Ile His His Ser Asn Asp Ala Ala Glu Gln Thr Lys Leu Tyr Gln
               180                 185                 190
   Asn Pro Thr Thr Tyr Ile Ser Val Gly Thr Ser Thr Leu Asn Gln Arg
           195                 200                 205
   Leu Val Pro Lys Ile Ala Thr Arg Ser Lys Val Asn Gly Gln Ser Gly
       210                 215                 220
   Arg Met Asp Phe Phe Trp Thr Ile Leu Lys Pro Asn Asp Ala Ile Asn
   225                 230                 235                 240
   Phe Glu Ser Asn Gly Asn Phe Ile Ala Pro Glu Tyr Ala Tyr Lys Ile
                   245                 250                 255
   Val Lys Lys Gly Asp Ser Ala Ile Val Lys Ser Glu Val Glu Tyr Gly
               260                 265                 270
   Asn Cys Asn Thr Lys Cys Gln Thr Pro Ile Gly Ala Ile Asn Ser Ser
           275                 280                 285
   Met Pro Phe His Asn Ile His Pro Leu Thr Ile Gly Glu Cys Pro Lys
       290                 295                 300
   Tyr Val Lys Ser Asn Lys Leu Val Leu Ala Thr Gly Leu Arg Asn Ser
   305                 310                 315                 320
   Pro Leu Arg Glu Arg Arg Arg Lys Arg Gly Leu Phe Gly Ala Ile Ala
                   325                 330                 335
   Gly Phe Ile Glu Gly Gly Trp Gln Gly Met Val Asp Gly Trp Tyr Gly
               340                 345                 350
   Tyr His His Ser Asn Glu Gln Gly Ser Gly Tyr Ala Ala Asp Lys Glu
           355                 360                 365
   Ser Thr Gln Lys Ala Ile Asp Gly Val Thr Asn Lys Val Asn Ser Ile
       370                 375                 380
   Ile Asp Lys Met Asn Thr Gln Phe Glu Ala Val Gly Arg Glu Phe Asn
   385                 390                 395                 400
   Asn Leu Glu Arg Arg Ile Glu Asn Leu Asn Lys Lys Met Glu Asp Gly
                   405                 410                 415
   Phe Leu Asp Val Trp Thr Tyr Asn Ala Glu Leu Leu Val Leu Met Glu
               420                 425                 430
   Asn Glu Arg Thr Leu Asp Phe His Asp Ser Asn Val Lys Asn Leu Tyr
           435                 440                 445
   Asp Lys Val Arg Leu Gln Leu Arg Asp Asn Ala Lys Glu Leu Gly Asn
       450                 455                 460
   Gly Cys Phe Glu Phe Tyr His Lys Cys Asp Asn Glu Cys Met Glu Ser
   465                 470                 475                 480
   Val Arg Asn Gly Thr Tyr Asp Tyr Pro Gln Tyr Ser Glu Glu Ala Arg
                   485                 490                 495
   Leu Lys Arg Glu Glu Ile Ser Gly Val Lys Leu Glu Ser Ile Gly Thr
               500                 505                 510
   Tyr Gln Ile
           515
<210> 10
   <211> 516
   <212> PRT
   <213> Influenza A
<220>
   <223> Hemagglutinin, Indonesia/5/2005/H5N1, HAI
<300>
   <308> ABP51969
<400> 10
   Asp Gln Ile Cys Ile Gly Tyr His Ala Asn Asn Ser Thr Glu Gln Val
   1               5                   10                  15
   Asp Thr Ile Met Glu Lys Asn Val Thr Val Thr His Ala Gln Asp Ile
               20                  25                  30
   Leu Glu Lys Thr His Asn Gly Lys Leu Cys Asp Leu Asp Gly Val Lys
           35                  40                  45
   Pro Leu Ile Leu Arg Asp Cys Ser Val Ala Gly Trp Leu Leu Gly Asn
       50                  55                  60
   Pro Met Cys Asp Glu Phe Ile Asn Val Pro Glu Trp Ser Tyr Ile Val
   65                  70                  75                  80
   Glu Lys Ala Asn Pro Thr Asn Asp Leu Cys Tyr Pro Gly Ser Phe Asn
                   85                  90                  95
   Asp Tyr Glu Glu Leu Lys His Leu Leu Ser Arg Ile Asn His Phe Glu
               100                 105                 110
   Lys Ile Gln Ile Ile Pro Lys Ser Ser Trp Ser Asp His Glu Ala Ser
           115                 120                 125
   Ser Gly Val Ser Ser Ala Cys Pro Tyr Leu Gly Ser Pro Ser Phe Phe
       130                 135                 140
   Arg Asn Val Val Trp Leu Ile Lys Lys Asn Ser Thr Tyr Pro Thr Ile
   145                 150                 155                 160
   Lys Lys Ser Tyr Asn Asn Thr Asn Gln Glu Asp Leu Leu Val Leu Trp
                   165                 170                 175
   Gly Ile His His Pro Asn Asp Ala Ala Glu Gln Thr Arg Leu Tyr Gln
               180                 185                 190
   Asn Pro Thr Thr Tyr Ile Ser Ile Gly Thr Ser Thr Leu Asn Gln Arg
           195                 200                 205
   Leu Val Pro Lys Ile Ala Thr Arg Ser Lys Val Asn Gly Gln Ser Gly
       210                 215                 220
   Arg Met Glu Phe Phe Trp Thr Ile Leu Lys Pro Asn Asp Ala Ile Asn
   225                 230                 235                 240
   Phe Glu Ser Asn Gly Asn Phe Ile Ala Pro Glu Tyr Ala Tyr Lys Ile
                   245                 250                 255
   Val Lys Lys Gly Asp Ser Ala Ile Met Lys Ser Glu Leu Glu Tyr Gly
               260                 265                 270
   Asn Cys Asn Thr Lys Cys Gln Thr Pro Met Gly Ala Ile Asn Ser Ser
           275                 280                 285
   Met Pro Phe His Asn Ile His Pro Leu Thr Ile Gly Glu Cys Pro Lys
   290 295 300
   Tyr Val Lys Ser Asn Arg Leu Val Leu Ala Thr Gly Leu Arg Asn Ser
   305 310 315 320
   Pro Gln Arg Glu Ser Arg Arg Lys Lys Arg Gly Leu Phe Gly Ala Ile
   325 330 335
   Ala Gly Phe Ile Glu Gly Gly Trp Gln Gly Met Val Asp Gly Trp Tyr
   340 345 350
   Gly Tyr His His Ser Asn Glu Gln Gly Ser Gly Tyr Ala Ala Asp Lys
   355 360 365
   Glu Ser Thr Gln Lys Ala Ile Asp Gly Val Thr Asn Lys Val Asn Ser
   370 375 380
   Ile Ile Asp Lys Met Asn Thr Gln Phe Glu Ala Val Gly Arg Glu Phe
   385 390 395 400
   Asn Asn Leu Glu Arg Arg Ile Glu Asn Leu Asn Lys Lys Met Glu Asp
   405 410 415
   Gly Phe Leu Asp Val Trp Thr Tyr Asn Ala Glu Leu Leu Val Leu Met
   420 425 430
   Glu Asn Glu Arg Thr Leu Asp Phe His Asp Ser Asn Val Lys Asn Leu
   435 440 445
   Tyr Asp Lys Val Arg Leu Gln Leu Arg Asp Asn Ala Lys Glu Leu Gly
   450 455 460
   Asn Gly Cys Phe Glu Phe Tyr His Lys Cys Asp Asn Glu Cys Met Glu
   465 470 475 480
   Ser Ile Arg Asn Gly Thr Tyr Asn Tyr Pro Gln Tyr Ser Glu Glu Ala
   485 490 495
   Arg Leu Lys Arg Glu Glu Ile Ser Gly Val Lys Leu Glu Ser Ile Gly
   500 505 510
   Thr Tyr Gln Ile
   515
<210> 11
   <211> 233
   <212> PRT
   <213> Influenza A
<220>
   <223> Hemagglutinin, globular domain, Indonesia/5/2005/H5N1, HA3I
<400> 11
   Cys Asp Leu Asp Gly Val Lys Pro Leu Ile Leu Arg Asp Cys Ser Val
   1               5                   10                  15
   Ala Gly Trp Leu Leu Gly Asn Pro Met Cys Asp Glu Phe Ile Asn Val
               20                  25                  30
   Pro Glu Trp Ser Tyr Ile Val Glu Lys Ala Asn Pro Thr Asn Asp Leu
           35                  40                  45
   Cys Tyr Pro Gly Ser Phe Asn Asp Tyr Glu Glu Leu Lys His Leu Leu
       50                  55                  60
   Ser Arg Ile Asn His Phe Glu Lys Ile Gln Ile Ile Pro Lys Ser Ser
   65                  70                  75                  80
   Trp Ser Asp His Glu Ala Ser Ser Gly Val Ser Ser Ala Cys Pro Tyr
                   85                  90                  95
   Leu Gly Ser Pro Ser Phe Phe Arg Asn Val Val Trp Leu Ile Lys Lys
               100                 105                 110
   Asn Ser Thr Tyr Pro Thr Ile Lys Lys Ser Tyr Asn Asn Thr Asn Gln
           115                 120                 125
   Glu Asp Leu Leu Val Leu Trp Gly Ile His His Pro Asn Asp Ala Ala
       130                 135                 140
   Glu Gln Thr Arg Leu Tyr Gln Asn Pro Thr Thr Tyr Ile Ser Ile Gly
   145                 150                 155                 160
   Thr Ser Thr Leu Asn Gln Arg Leu Val Pro Lys Ile Ala Thr Arg Ser
                   165                 170                 175
   Lys Val Asn Gly Gln Ser Gly Arg Met Glu Phe Phe Trp Thr Ile Leu
               180                 185                 190
   Lys Pro Asn Asp Ala Ile Asn Phe Glu Ser Asn Gly Asn Phe Ile Ala
           195                 200                 205
   Pro Glu Tyr Ala Tyr Lys Ile Val Lys Lys Gly Asp Ser Ala Ile Met
       210                 215                 220
   Lys Ser Glu Leu Glu Tyr Gly Asn Cys
   225                 230
<210> **12**
   <211> 227
   <212> PRT
   <213> Influenza A
<220>
   <223> Hemagglutinin, globular domain, Netherlands/219/2003/H7N7, HA3NL
<300>
   <308> AAR02640
<400> 12
   Cys Ser Lys Gly Lys Arg Thr Val Asp Leu Gly Gln Cys Gly Leu Leu
   1               5                   10                  15
   Gly Thr Ile Thr Gly Pro Pro Gln Cys Asp Gln Phe Leu Glu Phe Ser
               20                  25                  30
   Ala Asp Leu Ile Ile Glu Arg Arg Glu Gly Ser Asp Val Cys Tyr Pro
           35                  40                  45
   Gly Lys Phe Val Asn Glu Glu Ala Leu Arg Gln Ile Leu Arg Glu Ser
       50                  55                  60
   Gly Gly Ile Asp Lys Glu Thr Met Gly Phe Thr Tyr Ser Gly Ile Arg
   65                  70                  75                  80
   Thr Asn Gly Thr Thr Ser Ala Cys Arg Arg Ser Gly Ser Ser Phe Tyr
                   85                  90                  95
   Ala Glu Met Lys Trp Leu Leu Ser Asn Thr Asp Asn Ala Ala Phe Pro
               100                 105                 110
   Gln Met Thr Lys Ser Tyr Lys Asn Thr Arg Lys Asp Pro Ala Leu Ile
           115                 120                 125
   Ile Trp Gly Ile His His Ser Gly Ser Thr Thr Glu Gln Thr Lys Leu
       130                 135                 140
   Tyr Gly Ser Gly Asn Lys Leu Ile Thr Val Gly Ser Ser Asn Tyr Gln
   145                 150                 155                 160
   Gln Ser Phe Val Pro Ser Pro Gly Ala Arg Pro Gln Val Asn Gly Gln
                   165                 170                 175
   Ser Gly Arg Ile Asp Phe His Trp Leu Ile Leu Asn Pro Asn Asp Thr
               180                 185                 190
   Val Thr Phe Ser Phe Asn Gly Ala Phe Ile Ala Pro Asp Arg Ala Ser
           195                 200                 205
   Phe Leu Arg Gly Lys Ser Met Gly Ile Gln Ser Glu Val Gln Val Asp
       210                 215                 220
   Ala Asn Cys
   225
<210> 13
   <211> 233
   <212> PRT
   <213> Influenza A
<220>
   <223> Hemagglutinin, globular domain, Vietnam/1194/2004/H5N1, HA3V
<300>
   <308> AAT73273
<400> 13
   Cys Asp Leu Asp Gly Val Lys Pro Leu Ile Leu Arg Asp Cys Ser Val
   1               5                   10                  15
   Ala Gly Trp Leu Leu Gly Asn Pro Met Cys Asp Glu Phe Ile Asn Val
               20                  25                  30
   Pro Glu Trp Ser Tyr Ile Val Glu Lys Ala Asn Pro Val Asn Asp Leu
           35                  40                  45
   Cys Tyr Pro Gly Asp Phe Asn Asp Tyr Glu Glu Leu Lys His Leu Leu
       50                  55                  60
   Ser Arg Ile Asn His Phe Glu Lys Ile Gln Ile Ile Pro Lys Ser Ser
   65                  70                  75                  80
   Trp Ser Ser His Glu Ala Ser Leu Gly Val Ser Ser Ala Cys Pro Tyr
                   85                  90                  95
   Gln Gly Lys Ser Ser Phe Phe Arg Asn Val Val Trp Leu Ile Lys Lys
               100                 105                 110
   Asn Ser Thr Tyr Pro Thr Ile Lys Arg Ser Tyr Asn Asn Thr Asn Gln
           115                 120                 125
   Glu Asp Leu Leu Val Leu Trp Gly Ile His His Pro Asn Asp Ala Ala
       130                 135                 140
   Glu Gln Thr Lys Leu Tyr Gln Asn Pro Thr Thr Tyr Ile Ser Val Gly
   145                 150                 155                 160
   Thr Ser Thr Leu Asn Gln Arg Leu Val Pro Arg Ile Ala Thr Arg Ser
                   165                 170                 175
   Lys Val Asn Gly Gln Ser Gly Arg Met Glu Phe Phe Trp Thr Ile Leu
               180                 185                 190
   Lys Pro Asn Asp Ala Ile Asn Phe Glu Ser Asn Gly Asn Phe Ile Ala
   195 200 205
   Pro Glu Tyr Ala Tyr Lys Ile Val Lys Lys Gly Asp Ser Thr Ile Met
   210 215 220
   Lys Ser Glu Leu Glu Tyr Gly Asn Cys
   225 230
<210> 14
   <211> 226
   <212> PRT
   <213> Influenza A
<220>
   <223> Hemagglutinin, globular domain, Brisbane/10/2007/H3N2, HA3B(H3)
<300>
   <308> ABW23353
<400> 14
   Cys Asp Ser Pro His Gln Ile Leu Asp Gly Glu Asn Cys Thr Leu Ile
   1               5                   10                  15
   Asp Ala Leu Leu Gly Asp Pro Gln Cys Asp Gly Phe Gln Asn Lys Lys
               20                  25                  30
   Trp Asp Leu Phe Val Glu Arg Ser Lys Ala Tyr Ser Asn Cys Tyr Pro
           35                  40                  45
   Tyr Asp Val Pro Asp Tyr Ala Ser Leu Arg Ser Leu Val Ala Ser Ser
       50                  55                  60
   Gly Thr Leu Glu Phe Asn Asn Glu Ser Phe Asn Trp Thr Gly Val Thr
   65                  70                  75                  80
   Gln Asn Gly Thr Ser Ser Ala Cys Ile Arg Arg Ser Asn Asn Ser Phe
                   85                  90                  95
   Phe Ser Arg Leu Asn Trp Leu Thr His Leu Lys Phe Lys Tyr Pro Ala
               100                 105                 110
   Leu Asn Val Thr Met Pro Asn Asn Glu Lys Phe Asp Lys Leu Tyr Ile
           115                 120                 125
   Trp Gly Val His His Pro Gly Thr Asp Asn Asp Gln Ile Phe Pro Tyr
       130                 135                 140
   Ala Gln Ala Ser Gly Arg Ile Thr Val Ser Thr Lys Arg Ser Gln Gln
   145                 150                 155                 160
   Thr Val Ile Pro Asn Ile Gly Ser Arg Pro Arg Val Arg Asn Ile Pro
                   165                 170                 175
   Ser Arg Ile Ser Ile Tyr Trp Thr Ile Val Lys Pro Gly Asp Ile Leu
               180                 185                 190
   Leu Ile Asn Ser Thr Gly Asn Leu Ile Ala Pro Arg Gly Tyr Phe Lys
           195                 200                 205
   Ile Arg Ser Gly Lys Ser Ser Ile Met Arg Ser Asp Ala Pro Ile Gly
       210                 215                 220
   Lys Cys
   225
<210> 15
   <211> 239
   <212> PRT
   <213> Influenza B
<220>
   <223> Hemagglutinin, globular domain, Brisbane-like, HB3B(B)
<400> 15
   Cys Pro Asp Cys Leu Asn Cys Thr Asp Leu Asp Val Ala Leu Gly Arg
   1               5                   10                  15
   Pro Met Cys Val Gly Thr Thr Pro Ser Ala Lys Ala Ser Ile Leu His
               20                  25                  30
   Glu Val Arg Pro Val Thr Ser Gly Cys Phe Pro Ile Met His Asp Arg
           35                  40                  45
   Thr Lys Ile Arg Gln Leu Ala Asn Leu Leu Arg Gly Tyr Glu Asn Ile
       50                  55                  60
   Arg Leu Ser Thr Gln Asn Val Ile Asp Ala Glu Lys Ala Pro Gly Gly
   65                  70                  75                  80
   Pro Tyr Arg Leu Gly Thr Ser Gly Ser Cys Pro Asn Ala Thr Ser Lys
                   85                  90                  95
   Ser Gly Phe Phe Ala Thr Met Ala Trp Ala Val Pro Lys Asp Asn Asn
               100                 105                 110
   Lys Asn Ala Thr Asn Pro Leu Thr Val Glu Val Pro Tyr Ile Cys Thr
           115                 120                 125
   Glu Gly Glu Asp Gln Ile Thr Val Trp Gly Phe His Ser Asp Asp Lys
       130                 135                 140
   Thr Gln Met Lys Asn Leu Tyr Gly Asp Ser Asn Pro Gln Lys Phe Thr
   145                 150                 155                 160
   Ser Ser Ala Asn Gly Val Thr Thr His Tyr Val Ser Gln Ile Gly Gly
                   165                 170                 175
   Phe Pro Asp Gln Thr Glu Asp Gly Gly Leu Pro Gln Ser Gly Arg Ile
               180                 185                 190
   Val Val Asp Tyr Met Met Gln Lys Pro Gly Lys Thr Gly Thr Ile Val
           195                 200                 205
   Tyr Gln Arg Gly Val Leu Leu Pro Gln Lys Val Trp Cys Ala Ser Gly
       210                 215                 220
   Arg Ser Lys Val Ile Lys Gly Ser Leu Pro Leu Ile Gly Glu Ala
   225                 230                 235
<210> 16
   <211> 146
   <212> PRT
   <213> Plasmodium falciparum
<220>
   <223> Sexual stage cell surface protein, Pfs48(6N)/45
<300>
   <308> PF13_0247
<400> 16
   Asn Asn Asp Phe Cys Lys Pro Ser Ser Leu Asn Ser Glu Ile Ser Gly
   1               5                   10                  15
   Phe Ile Gly Tyr Lys Cys Asn Phe Ser Asn Glu Gly Val His Asn Leu
               20                  25                  30
   Lys Pro Asp Met Arg Glu Arg Arg Ser Ile Phe Cys Thr Ile His Ser
           35                  40                  45
   Tyr Phe Ile Tyr Asp Lys Ile Arg Leu Ile Ile Pro Lys Lys Ser Ser
       50                  55                  60
   Ser Pro Glu Phe Lys Ile Leu Pro Glu Lys Cys Phe Gln Lys Val Tyr
   65                  70                  75                  80
   Thr Asp Tyr Glu Asn Arg Val Glu Thr Asp Ile Ser Glu Leu Gly Leu
                   85                  90                  95
   Ile Glu Tyr Glu Ile Glu Glu Asn Asp Thr Asn Pro Asn Tyr Asn Glu
               100                 105                 110
   Arg Thr Ile Thr Ile Ser Pro Phe Ser Pro Lys Asp Ile Glu Phe Phe
           115                 120                 125
   Cys Phe Cys Asp Asn Thr Glu Lys Val Ile Ser Ser Ile Glu Gly Arg
       130                 135                 140
   Ser Ala
   145
<210> 17
   <211> 255
   <212> PRT
   <213> Plasmodium falciparum
<220>
   <223> Sexual stage cell surface protein, Pfs48-10C
<400> 17
   Met Val His Val Arg Val Leu Lys Tyr Pro His Asn Ile Leu Phe Thr
   1               5                   10                  15
   Asn Leu Thr Asn Asp Leu Phe Thr Tyr Leu Pro Lys Thr Tyr Asn Glu
               20                  25                  30
   Ser Asn Phe Val Ser Asn Val Leu Glu Val Glu Leu Asn Asp Gly Glu
           35                  40                  45
   Leu Phe Val Leu Ala Cys Glu Leu Ile Asn Lys Lys Cys Phe Gln Glu
       50                  55                  60
   Gly Lys Glu Lys Ala Leu Tyr Lys Ser Asn Lys Ile Ile Tyr His Lys
   65                  70                  75                  80
   Asn Leu Thr Ile Phe Lys Ala Pro Phe Tyr Val Thr Ser Lys Asp Val
                   85                  90                  95
   Asn Thr Glu Cys Thr Cys Lys Phe Lys Asn Asn Asn Tyr Lys Ile Val
               100                 105                 110
   Leu Lys Pro Lys Tyr Glu Lys Lys Val Ile His Gly Cys Asn Phe Ser
           115                 120                 125
   Ser Asn Val Ser Ser Lys His Thr Phe Thr Asp Ser Leu Asp Ile Ser
       130                 135                 140
   Leu Val Asp Asp Ser Ala His Ile Ser Cys Asn Val His Leu Ser Glu
   145                 150                 155                 160
   Pro Lys Tyr Asn His Leu Val Gly Leu Asn Cys Pro Gly Asp Ile Ile
                   165                 170                 175
   Pro Asp Cys Phe Phe Gln Val Tyr Gln Pro Glu Ser Glu Glu Leu Glu
               180                 185                 190
   Pro Ser Asn Ile Val Tyr Leu Asp Ser Gln Ile Asn Ile Gly Asp Ile
           195                 200                 205
   Glu Tyr Tyr Glu Asp Ala Glu Gly Asp Asp Lys Ile Lys Leu Phe Gly
       210                 215                 220
   Ile Val Gly Ser Ile Pro Lys Thr Thr Ser Phe Thr Cys Ile Cys Lys
   225                 230                 235                 240
   Lys Asp Lys Lys Ser Ala Tyr Met Thr Val Thr Ile Asp Ser Ala
                   245                 250                 255
<210> 18
   <211> 134
   <212> PRT
   <213> Plasmodium falciparum
<220>
   <223> Sexual stage cell surface protein, Pfs48-6C
<400> 18
   Ile His Gly Cys Asn Phe Ser Ser Asn Val Ser Ser Lys His Thr Phe
   1               5                   10                  15
   Thr Asp Ser Leu Asp Ile Ser Leu Val Asp Asp Ser Ala His Ile Ser
               20                  25                  30
   Cys Asn Val His Leu Ser Glu Pro Lys Tyr Asn His Leu Val Gly Leu
           35                  40                  45
   Asn Cys Pro Gly Asp Ile Ile Pro Asp Cys Phe Phe Gln Val Tyr Gln
       50                  55                  60
   Pro Glu Ser Glu Glu Leu Glu Pro Ser Asn Ile Val Tyr Leu Asp Ser
   65                  70                  75                  80
   Gln Ile Asn Ile Gly Asp Ile Glu Tyr Tyr Glu Asp Ala Glu Gly Asp
                   85                  90                  95
   Asp Lys Ile Lys Leu Phe Gly Ile Val Gly Ser Ile Pro Lys Thr Thr
               100                 105                 110
   Ser Phe Thr Cys Ile Cys Lys Lys Asp Lys Lys Ser Ala Tyr Met Thr
           115                 120                 125
   Val Thr Ile Asp Ser Ala
       130
<210> 19
   <211> 3135
   <212> PRT
   <213> Plasmodium falciparum
<220>
   <223> Sexual stage cell surface protein, Pfs230
<300>
   <308> AAA29724
<400> 19
   Met Lys Lys Ile Ile Thr Leu Lys Asn Leu Phe Leu Ile Ile Leu Val
   1               5                   10                  15
   Tyr Ile Phe Ser Glu Lys Lys Asp Leu Arg Cys Asn Val Ile Lys Gly
               20                  25                  30
   Asn Asn Ile Lys Asp Asp Glu Asp Lys Arg Phe His Leu Phe Tyr Tyr
           35                  40                  45
   Ser His Asn Leu Phe Lys Thr Pro Glu Thr Lys Glu Lys Lys Asn Lys
       50                  55                  60
   Lys Glu Cys Phe Tyr Lys Asn Gly Gly Ile Tyr Asn Leu Ser Lys Glu
   65                  70                  75                  80
   Ile Arg Met Arg Lys Asp Thr Ser Val Lys Ile Lys Gln Arg Thr Cys
                   85                  90                  95
   Pro Phe His Lys Glu Gly Ser Ser Phe Glu Met Gly Ser Lys Asn Ile
               100                 105                 110
   Thr Cys Phe Tyr Pro Ile Val Gly Lys Lys Glu Arg Lys Thr Leu Asp
           115                 120                 125
   Thr Ile Ile Ile Lys Lys Asn Val Thr Asn Asp His Val Val Ser Ser
       130                 135                 140
   Asp Met His Ser Asn Val Gln Glu Lys Asn Met Ile Leu Ile Arg Asn
   145                 150                 155                 160
   Ile Asp Lys Glu Asn Lys Asn Asp Ile Gln Asn Val Glu Glu Lys Ile
                   165                 170                 175
   Gln Arg Asp Thr Tyr Glu Asn Lys Asp Tyr Glu Ser Asp Asp Thr Leu
               180                 185                 190
   Ile Glu Trp Phe Asp Asp Asn Thr Asn Glu Glu Asn Phe Leu Leu Thr
   195 200 205
   Phe Leu Lys Arg Cys Leu Met Lys Ile Phe Ser Ser Pro Lys Arg Lys
   210 215 220
   Lys Thr Val Val Gln Lys Lys His Lys Ser Asn Phe Phe Ile Asn Ser
   225 230 235 240
   Ser Leu Lys Tyr Ile Tyr Met Tyr Leu Thr Pro Ser Asp Ser Phe Asn
   245 250 255
   Leu Val Arg Arg Asn Arg Asn Leu Asp Glu Glu Asp Met Ser Pro Arg
   260 265 270
   Asp Asn Phe Val Ile Asp Asp Glu Glu Glu Glu Glu Glu Glu Glu Glu
   275 280 285
   Glu Glu Glu Glu Glu Glu Glu Glu Glu Glu Glu Glu Glu Glu Glu Glu
   290 295 300
   Tyr Asp Asp Tyr Val Tyr Glu Glu Ser Gly Asp Glu Thr Glu Glu Gln
   305 310 315 320
   Leu Gln Glu Glu His Gln Glu Glu Val Gly Ala Glu Ser Ser Glu Glu
   325 330 335
   Ser Phe Asn Asp Glu Asp Glu Asp Ser Val Glu Ala Arg Asp Gly Asp
   340 345 350
   Met Ile Arg Val Asp Glu Tyr Tyr Glu Asp Gln Asp Gly Asp Thr Tyr
   355 360 365
   Asp Ser Thr Ile Lys Asn Glu Asp Val Asp Glu Glu Val Gly Glu Glu
   370 375 380
   Val Gly Glu Glu Val Gly Glu Glu Val Gly Glu Glu Val Gly Glu Glu
   385 390 395 400
   Val Gly Glu Glu Val Gly Glu Glu Val Gly Glu Glu Val Gly Glu Glu
   405 410 415
   Glu Gly Glu Glu Val Gly Glu Gly Val Gly Glu Glu Val Gly Glu Glu
   420 425 430
   Glu Gly Glu Glu Val Gly Glu Glu Glu Gly Glu Tyr Val Asp Glu Lys
   435 440 445
   Glu Arg Gln Gly Glu Ile Tyr Pro Phe Gly Asp Glu Glu Glu Lys Asp
       450                 455                 460
   Glu Gly Gly Glu Ser Phe Thr Tyr Glu Lys Ser Glu Val Asp Lys Thr
   465                 470                 475                 480
   Asp Leu Phe Lys Phe Ile Glu Gly Gly Glu Gly Asp Asp Val Tyr Lys
                   485                 490                 495
   Val Asp Gly Ser Lys Val Leu Leu Asp Asp Asp Thr Ile Ser Arg Val
               500                 505                 510
   Ser Lys Lys His Thr Ala Arg Asp Gly Glu Tyr Gly Glu Tyr Gly Glu
           515                 520                 525
   Ala Val Glu Asp Gly Glu Asn Val Ile Lys Ile Ile Arg Ser Val Leu
       530                 535                 540
   Gln Ser Gly Ala Leu Pro Ser Val Gly Val Asp Glu Leu Asp Lys Ile
   545                 550                 555                 560
   Asp Leu Ser Tyr Glu Thr Thr Glu Ser Gly Asp Thr Ala Val Ser Glu
                   565                 570                 575
   Asp Ser Tyr Asp Lys Tyr Ala Ser Asn Asn Thr Asn Lys Glu Tyr Val
               580                 585                 590
   Cys Asp Phe Thr Asp Gln Leu Lys Pro Thr Glu Ser Gly Pro Lys Val
           595                 600                 605
   Lys Lys Cys Glu Val Lys Val Asn Glu Pro Leu Ile Lys Val Lys Ile
       610                 615                 620
   Ile Cys Pro Leu Lys Gly Ser Val Glu Lys Leu Tyr Asp Asn Ile Glu
   625                 630                 635                 640
   Tyr Val Pro Lys Lys Ser Pro Tyr Val Val Leu Thr Lys Glu Glu Thr
                   645                 650                 655
   Lys Leu Lys Glu Lys Leu Leu Ser Lys Leu Ile Tyr Gly Leu Leu Ile
               660                 665                 670
   Ser Pro Thr Val Asn Glu Lys Glu Asn Asn Phe Lys Glu Gly Val Ile
           675                 680                 685
   Glu Phe Thr Leu Pro Pro Val Val His Lys Ala Thr Val Phe Tyr Phe
       690                 695                 700
   Ile Cys Asp Asn Ser Lys Thr Glu Asp Asp Asn Lys Lys Gly Asn Arg
   705 710 715 720
   Gly Ile Val Glu Val Tyr Val Glu Pro Tyr Gly Asn Lys Ile Asn Gly
   725 730 735
   Cys Ala Phe Leu Asp Glu Asp Glu Glu Glu Glu Lys Tyr Gly Asn Gln
   740 745 750
   Ile Glu Glu Asp Glu His Asn Glu Lys Ile Lys Met Lys Thr Phe Phe
   755 760 765
   Thr Gln Asn Ile Tyr Lys Lys Asn Asn Ile Tyr Pro Cys Tyr Met Lys
   770 775 780
   Leu Tyr Ser Gly Asp Ile Gly Gly Ile Leu Phe Pro Lys Asn Ile Lys
   785 790 795 800
   Ser Thr Thr Cys Phe Glu Glu Met Ile Pro Tyr Asn Lys Glu Ile Lys
   805 810 815
   Trp Asn Lys Glu Asn Lys Ser Leu Gly Asn Leu Val Asn Asn Ser Val
   820 825 830
   Val Tyr Asn Lys Glu Met Asn Ala Lys Tyr Phe Asn Val Gln Tyr Val
   835 840 845
   His Ile Pro Thr Ser Tyr Lys Asp Thr Leu Asn Leu Phe Cys Ser Ile
   850 855 860
   Ile Leu Lys Glu Glu Glu Ser Asn Leu Ile Ser Thr Ser Tyr Leu Val
   865 870 875 880
   Tyr Val Ser Ile Asn Glu Glu Leu Asn Phe Ser Leu Phe Asp Phe Tyr
   885 890 895
   Glu Ser Phe Val Pro Ile Lys Lys Thr Ile Gln Val Ala Gln Lys Asn
   900 905 910
   Val Asn Asn Lys Glu His Asp Tyr Thr Cys Asp Phe Thr Asp Lys Leu
   915 920 925
   Asp Lys Thr Val Pro Ser Thr Ala Asn Gly Lys Lys Leu Phe Ile Cys
   930 935 940
   Arg Lys His Leu Lys Glu Phe Asp Thr Phe Thr Leu Lys Cys Asn Val
   945 950 955 960
   Asn Lys Thr Gln Tyr Pro Asn Ile Glu Ile Phe Pro Lys Thr Leu Lys
   965 970 975
   Asp Lys Lys Glu Val Leu Lys Leu Asp Leu Asp Ile Gln Tyr Gln Met
   980 985 990
   Phe Ser Lys Phe Phe Lys Phe Asn Thr Gln Asn Ala Lys Tyr Leu Asn
   995 1000 1005
   Leu Tyr Pro Tyr Tyr Leu Ile Phe Pro Phe Asn His Ile Gly Lys
   1010 1015 1020
   Lys Glu Leu Lys Asn Asn Pro Thr Tyr Lys Asn His Lys Asp Val
   1025 1030 1035
   Lys Tyr Phe Glu Gln Ser Ser Val Leu Ser Pro Leu Ser Ser Ala
   1040 1045 1050
   Asp Ser Leu Gly Lys Leu Leu Asn Phe Leu Asp Thr Gln Glu Thr
   1055 1060 1065
   Val Cys Leu Thr Glu Lys Ile Arg Tyr Leu Asn Leu Ser Ile Asn
   1070 1075 1080
   Glu Leu Gly Ser Asp Asn Asn Thr Phe Ser Val Thr Phe Gln Val
   1085 1090 1095
   Pro Pro Tyr Ile Asp Ile Lys Glu Pro Phe Tyr Phe Met Phe Gly
   1100 1105 1110
   Cys Asn Asn Asn Lys Gly Glu Gly Asn Ile Gly Ile Val Glu Leu
   1115 1120 1125
   Leu Ile Ser Lys Gln Glu Glu Lys Ile Lys Gly Cys Asn Phe His
   1130 1135 1140
   Glu Ser Lys Leu Asp Tyr Phe Asn Glu Asn Ile Ser Ser Asp Thr
   1145 1150 1155
   His Glu Cys Thr Leu His Ala Tyr Glu Asn Asp Ile Ile Gly Phe
   1160 1165 1170
   Asn Cys Leu Glu Thr Thr His Pro Asn Glu Val Glu Val Glu Val
   1175 1180 1185
   Glu Asp Ala Glu Ile Tyr Leu Gln Pro Glu Asn Cys Phe Asn Asn
   1190 1195 1200
   Val Tyr Lys Gly Leu Asn Ser Val Asp Ile Thr Thr Ile Leu Lys
   1205 1210 1215
   Asn Ala Gln Thr Tyr Asn Ile Asn Asn Lys Lys Thr Pro Thr Phe
   1220 1225 1230
   Leu Lys Ile Pro Pro Tyr Asn Leu Leu Glu Asp Val Glu Ile Ser
   1235 1240 1245
   Cys Gln Cys Thr Ile Lys Gln Val Val Lys Lys Ile Lys Val Ile
   1250 1255 1260
   Ile Thr Lys Asn Asp Thr Val Leu Leu Lys Arg Glu Val Gln Ser
   1265 1270 1275
   Glu Ser Thr Leu Asp Asp Lys Ile Tyr Lys Cys Glu His Glu Asn
   1280 1285 1290
   Phe Ile Asn Pro Arg Val Asn Lys Thr Phe Asp Glu Asn Val Glu
   1295 1300 1305
   Tyr Thr Cys Asn Ile Lys Ile Glu Asn Phe Phe Asn Tyr Ile Gln
   1310 1315 1320
   Ile Phe Cys Pro Ala Lys Asp Leu Gly Ile Tyr Lys Asn Ile Gln
   1325 1330 1335
   Met Tyr Tyr Asp Ile Val Lys Pro Thr Arg Val Pro Gln Phe Lys
   1340 1345 1350
   Lys Phe Asn Asn Glu Glu Leu His Lys Leu Ile Pro Asn Ser Glu
   1355 1360 1365
   Met Leu His Lys Thr Lys Glu Met Leu Ile Leu Tyr Asn Glu Glu
   1370 1375 1380
   Lys Val Asp Leu Leu His Phe Tyr Val Phe Leu Pro Ile Tyr Ile
   1385 1390 1395
   Lys Asp Ile Tyr Glu Phe Asn Ile Val Cys Asp Asn Ser Lys Thr
   1400 1405 1410
   Met Trp Lys Asn Gln Leu Gly Gly Lys Val Ile Tyr His Ile Thr
   1415 1420 1425
   Val Ser Lys Arg Glu Gln Lys Val Lys Gly Cys Ser Phe Asp Asn
   1430 1435 1440
   Glu His Ala His Met Phe Ser Tyr Asn Lys Thr Asn Val Lys Asn
   1445 1450 1455
   Cys Ile Ile Asp Ala Lys Pro Lys Asp Leu Ile Gly Phe Val Cys
   1460 1465 1470
   Pro Ser Gly Thr Leu Lys Leu Thr Asn Cys Phe Lys Asp Ala Ile
   1475 1480 1485
   Val His Thr Asn Leu Thr Asn Ile Asn Gly Ile Leu Tyr Leu Lys
   1490 1495 1500
   Asn Asn Leu Ala Asn Phe Thr Tyr Lys His Gln Phe Asn Tyr Met
   1505 1510 1515
   Glu Ile Pro Ala Leu Met Asp Asn Asp Ile Ser Phe Lys Cys Ile
   1520 1525 1530
   Cys Val Asp Leu Lys Lys Lys Lys Tyr Asn Val Lys Ser Pro Leu
   1535 1540 1545
   Gly Pro Lys Val Leu Arg Ala Leu Tyr Lys Lys Leu Asn Ile Lys
   1550 1555 1560
   Phe Asp Asn Tyr Val Thr Gly Thr Asp Gln Asn Lys Tyr Leu Met
   1565 1570 1575
   Thr Tyr Met Asp Leu His Leu Ser His Lys Arg Asn Tyr Leu Lys
   1580 1585 1590
   Glu Leu Phe His Asp Leu Gly Lys Lys Lys Pro Ala Asp Thr Asp
   1595 1600 1605
   Ala Asn Pro Glu Ser Ile Ile Glu Ser Leu Ser Ile Asn Glu Ser
   1610 1615 1620
   Asn Glu Ser Gly Pro Phe Pro Thr Gly Asp Val Asp Ala Glu His
   1625 1630 1635
   Leu Ile Leu Glu Gly Tyr Asp Thr Trp Glu Ser Leu Tyr Asp Glu
   1640 1645 1650
   Gln Leu Glu Glu Val Ile Tyr Asn Asp Ile Glu Ser Leu Glu Leu
   1655 1660 1665
   Lys Asp Ile Glu Gln Tyr Val Leu Gln Val Asn Leu Lys Ala Pro
   1670 1675 1680
   Lys Leu Met Met Ser Ala Gln Ile His Asn Asn Arg His Val Cys
   1685 1690 1695
   Asp Phe Ser Lys Asn Asn Leu Ile Val Pro Glu Ser Leu Lys Lys
   1700 1705 1710
   Lys Glu Glu Leu Gly Gly Asn Pro Val Asn Ile His Cys Tyr Ala
   1715 1720 1725
   Leu Leu Lys Pro Leu Asp Thr Leu Tyr Val Lys Cys Pro Thr Ser
   1730 1735 1740
   Lys Asp Asn Tyr Glu Ala Ala Lys Val Asn Ile Ser Glu Asn Asp
   1745 1750 1755
   Asn Glu Tyr Glu Leu Gln Val Ile Ser Leu Ile Glu Lys Arg Phe
   1760 1765 1770
   His Asn Phe Glu Thr Leu Glu Ser Lys Lys Pro Gly Asn Gly Asp
   1775 1780 1785
   Val Val Val His Asn Gly Val Val Asp Thr Gly Pro Val Leu Asp
   1790 1795 1800
   Asn Ser Thr Phe Glu Lys Tyr Phe Lys Asn Ile Lys Ile Lys Pro
   1805 1810 1815
   Asp Lys Phe Phe Glu Lys Val Ile Asn Glu Tyr Asp Asp Thr Glu
   1820 1825 1830
   Glu Glu Lys Asp Leu Glu Ser Ile Leu Pro Gly Ala Ile Val Ser
   1835 1840 1845
   Pro Met Lys Val Leu Lys Lys Lys Asp Pro Phe Thr Ser Tyr Ala
   1850 1855 1860
   Ala Phe Val Val Pro Pro Ile Val Pro Lys Asp Leu His Phe Lys
   1865 1870 1875
   Val Glu Cys Asn Asn Thr Glu Tyr Lys Asp Glu Asn Gln Tyr Ile
   1880 1885 1890
   Ser Gly Tyr Asn Gly Ile Ile His Ile Asp Ile Ser Asn Ser Asn
   1895 1900 1905
   Arg Lys Ile Asn Gly Cys Asp Phe Ser Thr Asn Asn Ser Ser Ile
   1910 1915 1920
   Leu Thr Ser Ser Val Lys Leu Val Asn Gly Glu Thr Lys Asn Cys
   1925 1930 1935
   Glu Ile Asn Ile Asn Asn Asn Glu Val Phe Gly Ile Ile Cys Asp
   1940 1945 1950
   Asn Glu  Thr Asn Leu Asp Pro  Glu Lys Cys Phe His  Glu Ile Tyr
       1955                 1960                 1965
   Ser Lys  Asp Asn Lys Thr Val  Lys Lys Phe Arg Glu  Val Ile Pro
       1970                 1975                 1980
   Asn Ile  Asp Ile Phe Ser Leu  His Asn Ser Asn Lys  Lys Lys Val
       1985                 1990                 1995
   Ala Tyr  Ala Lys Val Pro Leu  Asp Tyr Ile Asn Lys  Leu Leu Phe
       2000                 2005                 2010
   Ser Cys  Ser Cys Lys Thr Ser  His Thr Asn Thr Ile  Gly Thr Met
       2015                 2020                 2025
   Lys Val  Thr Leu Asn Lys Asp  Glu Lys Glu Glu Glu  Asp Phe Lys
       2030                 2035                 2040
   Thr Ala  Gln Gly Ile Lys His  Asn Asn Val His Leu  Cys Asn Phe
       2045                 2050                 2055
   Phe Asp  Asn Pro Glu Leu Thr  Phe Asp Asn Asn Lys  Ile Val Leu
       2060                 2065                 2070
   Cys Lys  Ile Asp Ala Glu Leu  Phe Ser Glu Val Ile  Ile Gln Leu
       2075                 2080                 2085
   Pro Ile  Phe Gly Thr Lys Asn  Val Glu Glu Gly Val  Gln Asn Glu
       2090                 2095                 2100
   Glu Tyr  Lys Lys Phe Ser Leu  Lys Pro Ser Leu Val  Phe Asp Asp
       2105                 2110                 2115
   Asn Asn  Asn Asp Ile Lys Val  Ile Gly Lys Glu Lys  Asn Glu Val
       2120                 2125                 2130
   Ser Ile  Ser Leu Ala Leu Lys  Gly Val Tyr Gly Asn  Arg Ile Phe
       2135                 2140                 2145
   Thr Phe  Asp Lys Asn Gly Lys  Lys Gly Glu Gly Ile  Ser Phe Phe
       2150                 2155                 2160
   Ile Pro  Pro Ile Lys Gln Asp  Thr Asp Leu Lys Phe  Ile Ile Asn
       2165                 2170                 2175
   Glu Thr  Ile Asp Asn Ser Asn  Ile Lys Gln Arg Gly  Leu Ile Tyr
       2180                 2185                 2190
   Ile Phe Val Arg Lys Asn Val Ser Glu Asn Ser Phe Lys Leu Cys
   2195 2200 2205
   Asp Phe Thr Thr Gly Ser Thr Ser Leu Met Glu Leu Asn Ser Gln
   2210 2215 2220
   Val Lys Glu Lys Lys Cys Thr Val Lys Ile Lys Lys Gly Asp Ile
   2225 2230 2235
   Phe Gly Leu Lys Cys Pro Lys Gly Phe Ala Ile Phe Pro Gln Ala
   2240 2245 2250
   Cys Phe Ser Asn Val Leu Leu Glu Tyr Tyr Lys Ser Asp Tyr Glu
   2255 2260 2265
   Asp Ser Glu His Ile Asn Tyr Tyr Ile His Lys Asp Lys Lys Tyr
   2270 2275 2280
   Asn Leu Lys Pro Lys Asp Val Ile Glu Leu Met Asp Glu Asn Phe
   2285 2290 2295
   Arg Glu Leu Gln Asn Ile Gln Gln Tyr Thr Gly Ile Ser Asn Ile
   2300 2305 2310
   Thr Asp Val Leu His Phe Lys Asn Phe Asn Leu Gly Asn Leu Pro
   2315 2320 2325
   Leu Asn Phe Lys Asn His Tyr Ser Thr Ala Tyr Ala Lys Val Pro
   2330 2335 2340
   Asp Thr Phe Asn Ser Ile Ile Asn Phe Ser Cys Asn Cys Tyr Asn
   2345 2350 2355
   Pro Glu Lys His Val Tyr Gly Thr Met Gln Val Glu Ser Asp Asn
   2360 2365 2370
   Arg Asn Phe Asp Asn Ile Lys Lys Asn Glu Asn Val Ile Lys Asn
   2375 2380 2385
   Phe Leu Leu Pro Asn Ile Glu Lys Tyr Ala Leu Leu Leu Asp Asp
   2390 2395 2400
   Glu Glu Arg Gln Lys Lys Ile Lys Gln Gln Gln Glu Glu Glu Gln
   2405 2410 2415
   Gln Glu Gln Ile Leu Lys Asp Gln Asp Asp Arg Leu Ser Arg His
   2420 2425 2430
   Asp Asp Tyr Asn Lys Asn His Thr Tyr Ile Leu Tyr Asp Ser Asn
   2435 2440 2445
   Glu His Ile Cys Asp Tyr Glu Lys Asn Glu Ser Leu Ile Ser Thr
   2450 2455 2460
   Leu Pro Asn Asp Thr Lys Lys Ile Gln Lys Ser Ile Cys Lys Ile
   2465 2470 2475
   Asn Ala Lys Ala Leu Asp Val Val Thr Ile Lys Cys Pro His Thr
   2480 2485 2490
   Lys Asn Phe Thr Pro Lys Asp Tyr Phe Pro Asn Ser Ser Leu Ile
   2495 2500 2505
   Thr Asn Asp Lys Lys Ile Val Ile Thr Phe Asp Lys Lys Asn Phe
   2510 2515 2520
   Val Thr Tyr Ile Asp Pro Thr Lys Lys Thr Phe Ser Leu Lys Asp
   2525 2530 2535
   Ile Tyr Ile Gln Ser Phe Tyr Gly Val Ser Leu Asp His Leu Asn
   2540 2545 2550
   Gln Ile Lys Lys Ile His Glu Glu Trp Asp Asp Val His Leu Phe
   2555 2560 2565
   Tyr Pro Pro His Asn Val Leu His Asn Val Val Leu Asn Asn His
   2570 2575 2580
   Ile Val Asn Leu Ser Ser Ala Leu Glu Gly Val Leu Phe Met Lys
   2585 2590 2595
   Ser Lys Val Thr Gly Asp Glu Thr Ala Thr Lys Lys Asn Thr Thr
   2600 2605 2610
   Leu Pro Thr Asp Gly Val Ser Ser Ile Leu Ile Pro Pro Tyr Val
   2615 2620 2625
   Lys Glu Asp Ile Thr Phe His Leu Phe Cys Gly Lys Ser Thr Thr
   2630 2635 2640
   Lys Lys Pro Asn Lys Lys Asn Thr Ser Leu Ala Leu Ile His Ile
   2645 2650 2655
   His Ile Ser Ser Asn Arg Asn Ile Ile His Gly Cys Asp Phe Leu
   2660 2665 2670
   Tyr Leu Glu Asn Gln Thr Asn Asp Ala Ile Ser Asn Asn Asn Asn
   2675 2680 2685
   Asn Ser Tyr Ser Ile Phe Thr His Asn Lys Asn Thr Glu Asn Asn
   2690 2695 2700
   Leu Ile Cys Asp Ile Ser Leu Ile Pro Lys Thr Val Ile Gly Ile
   2705 2710 2715
   Lys Cys Pro Asn Lys Lys Leu Asn Pro Gln Thr Cys Phe Asp Glu
   2720 2725 2730
   Val Tyr Tyr Val Lys Gln Glu Asp Val Pro Ser Lys Thr Ile Thr
   2735 2740 2745
   Ala Asp Lys Tyr Asn Thr Phe Ser Lys Asp Lys Ile Gly Asn Ile
   2750 2755 2760
   Leu Lys Asn Ala Ile Ser Ile Asn Asn Pro Asp Glu Lys Asp Asn
   2765 2770 2775
   Thr Tyr Thr Tyr Leu Ile Leu Pro Glu Lys Phe Glu Glu Glu Leu
   2780 2785 2790
   Ile Asp Thr Lys Lys Val Leu Ala Cys Thr Cys Asp Asn Lys Tyr
   2795 2800 2805
   Ile Ile His Met Lys Ile Glu Lys Ser Thr Met Asp Lys Ile Lys
   2810 2815 2820
   Ile Asp Glu Lys Lys Thr Ile Gly Lys Asp Ile Cys Lys Tyr Asp
   2825 2830 2835
   Val Thr Thr Lys Val Ala Thr Cys Glu Ile Ile Asp Thr Ile Asp
   2840 2845 2850
   Ser Ser Val Leu Lys Glu His His Thr Val His Tyr Ser Ile Thr
   2855 2860 2865
   Leu Ser Arg Trp Asp Lys Leu Ile Ile Lys Tyr Pro Thr Asn Glu
   2870 2875 2880
   Lys Thr His Phe Glu Asn Phe Phe Val Asn Pro Phe Asn Leu Lys
   2885 2890 2895
   Asp Lys Val Leu Tyr Asn Tyr Asn Lys Pro Ile Asn Ile Glu His
   2900 2905 2910
   Ile Leu Pro Gly Ala Ile Thr Thr Asp Ile Tyr Asp Thr Arg Thr
   2915 2920 2925
   Lys Ile Lys Gln Tyr Ile Leu Arg Ile Pro Pro Tyr Val His Lys
       2930                2935                2940
   Asp Ile His Phe Ser Leu Glu Phe Asn Asn Ser Leu Ser Leu Thr
       2945                2950                2955
   Lys Gln Asn Gln Asn Ile Ile Tyr Gly Asn Val Ala Lys Ile Phe
       2960                2965                2970
   Ile His Ile Asn Gln Gly Tyr Lys Glu Ile His Gly Cys Asp Phe
       2975                2980                2985
   Thr Gly Lys Tyr Ser His Leu Phe Thr Tyr Ser Lys Lys Pro Leu
       2990                2995                3000
   Pro Asn Asp Asp Asp Ile Cys Asn Val Thr Ile Gly Asn Asn Thr
       3005                3010                3015
   Phe Ser Gly Phe Ala Cys Leu Ser His Phe Glu Leu Lys Pro Asn
       3020                3025                3030
   Asn Cys Phe Ser Ser Val Tyr Asp Tyr Asn Glu Ala Asn Lys Val
       3035                3040                3045
   Lys Lys Leu Phe Asp Leu Ser Thr Lys Val Glu Leu Asp His Ile
       3050                3055                3060
   Lys Gln Asn Thr Ser Gly Tyr Thr Leu Ser Tyr Ile Ile Phe Asn
       3065                3070                3075
   Lys Glu Ser Thr Lys Leu Lys Phe Ser Cys Thr Cys Ser Ser Asn
       3080                3085                3090
   Tyr Ser Asn Tyr Thr Ile Arg Ile Thr Phe Asp Pro Asn Tyr Ile
       3095                3100                3105
   Ile Pro Glu Pro Gln Ser Arg Ala Ile Ile Lys Tyr Val Asp Leu
       3110                3115                3120
   Gln Asp Lys Asn Phe Ala Lys Tyr Leu Arg Lys Leu
       3125                3130                3135
<210> 20
   <211> 76
   <212> DNA
   <213> Influenza A
<220>
   <223> Forward primer, Anhui/Indonesia, Vietnam
<400> 20
   caccatggga gtgaaagttc tttttgccct tatttgtatt gctgtggccg aggcctgcga 60
   tcttgatggt gttaag 76
<210> 21
   <211> 75
   <212> DNA
   <213> Influenza A
<220>
   <223> Forward primer, globular domain, California
<400> 21
   caccatggga gtgaaagttc tttttgccct tatttgtatt gctgtggccg aggcctgtaa 60
   gttgaggggt gttgc 75
<210> 22
   <211> 20
   <212> DNA
   <213> Alfalfa mosaic virus
<220>
   <223> Reverse primer
<400> 22
   tcaatgacga tcaagatcgt 20
<210> 23
   <211> 22
   <212> PRT
   <213> Influenza A
<220>
   <223> Matrix protein 2, California/04/2009/H1N1, M2 peptide
<300>
   <308> ACP44153
<400> 23
   Leu Thr Glu Val Glu Thr Pro Thr Arg Ser Glu Trp Glu Cys Arg Cys
   1 5 10 15
   Ser Asp Ser Ser Asp Pro
   20
<210> 24
   <211> 498
   <212> PRT
   <213> Influenza A
<220>
   <223> Nucleocapsid protein, California/04/2009/H1N1, NP
<300>
   <308> ACP41106
<400> 24
   Met Ala Ser Gln Gly Thr Lys Arg Ser Tyr Glu Gln Met Glu Thr Gly
   1               5                   10                  15
   Gly Glu Arg Gln Asp Ala Thr Glu Ile Arg Ala Ser Val Gly Arg Met
               20                  25                  30
   Ile Gly Gly Ile Gly Arg Phe Tyr Ile Gln Met Cys Thr Glu Leu Lys
           35                  40                  45
   Leu Ser Asp Tyr Asp Gly Arg Leu Ile Gln Asn Ser Ile Thr Ile Glu
       50                  55                  60
   Arg Met Val Leu Ser Ala Phe Asp Glu Arg Arg Asn Lys Tyr Leu Glu
   65                  70                  75                  80
   Glu His Pro Ser Ala Gly Lys Asp Pro Lys Lys Thr Gly Gly Pro Ile
                   85                  90                  95
   Tyr Arg Arg Val Asp Gly Lys Trp Met Arg Glu Leu Ile Leu Tyr Asp
               100                 105                 110
   Lys Glu Glu Ile Arg Arg Val Trp Arg Gln Ala Asn Asn Gly Glu Asp
           115                 120                 125
   Ala Thr Ala Gly Leu Thr His Ile Met Ile Trp His Ser Asn Leu Asn
       130                 135                 140
   Asp Ala Thr Tyr Gln Arg Thr Arg Ala Leu Val Arg Thr Gly Met Asp
   145                 150                 155                 160
   Pro Arg Met Cys Ser Leu Met Gln Gly Ser Thr Leu Pro Arg Arg Ser
                   165                 170                 175
   Gly Ala Ala Gly Ala Ala Val Lys Gly Val Gly Thr Ile Ala Met Glu
               180                 185                 190
   Leu Ile Arg Met Ile Lys Arg Gly Ile Asn Asp Arg Asn Phe Trp Arg
           195                 200                 205
   Gly Glu Asn Gly Arg Arg Thr Arg Val Ala Tyr Glu Arg Met Cys Asn
       210                 215                 220
   Ile Leu Lys Gly Lys Phe Gln Thr Ala Ala Gln Arg Ala Met Met Asp
   225                 230                 235                 240
   Gln Val Arg Glu Ser Arg Asn Pro Gly Asn Ala Glu Ile Glu Asp Leu
                   245                 250                 255
   Ile Phe Leu Ala Arg Ser Ala Leu Ile Leu Arg Gly Ser Val Ala His
               260                 265                 270
   Lys Ser Cys Leu Pro Ala Cys Val Tyr Gly Leu Ala Val Ala Ser Gly
           275                 280                 285
   His Asp Phe Glu Arg Glu Gly Tyr Ser Leu Val Gly Ile Asp Pro Phe
       290                 295                 300
   Lys Leu Leu Gln Asn Ser Gln Val Val Ser Leu Met Arg Pro Asn Glu
   305                 310                 315                 320
   Asn Pro Ala His Lys Ser Gln Leu Val Trp Met Ala Cys His Ser Ala
                   325                 330                 335
   Ala Phe Glu Asp Leu Arg Val Ser Ser Phe Ile Arg Gly Lys Lys Val
               340                 345                 350
   Ile Pro Arg Gly Lys Leu Ser Thr Arg Gly Val Gln Ile Ala Ser Asn
           355                 360                 365
   Glu Asn Val Glu Thr Met Asp Ser Asn Thr Leu Glu Leu Arg Ser Arg
       370                 375                 380
   Tyr Trp Ala Ile Arg Thr Arg Ser Gly Gly Asn Thr Asn Gln Gln Lys
   385                 390                 395                 400
   Ala Ser Ala Gly Gln Ile Ser Val Gln Pro Thr Phe Ser Val Gln Arg
                   405                 410                 415
   Asn Leu Pro Phe Glu Arg Ala Thr Val Met Ala Ala Phe Ser Gly Asn
               420                 425                 430
   Asn Glu Gly Arg Thr Ser Asp Met Arg Thr Glu Val Ile Arg Met Met
           435                 440                 445
   Glu Ser Ala Lys Pro Glu Asp Leu Ser Phe Gln Gly Arg Gly Val Phe
       450                 455                 460
   Glu Leu Ser Asp Glu Lys Ala Thr Asn Pro Ile Val Pro Ser Phe Asp
   465                 470                 475                 480
   Met Ser Asn Glu Gly Ser Tyr Phe Phe Gly Asp Asn Ala Glu Glu Tyr
                   485                 490                 495
   Asp Ser
<210> 25
   <211> 568
   <212> PRT
   <213> Influenza A
<220>
   <223> Hemagglutinin, bar-headed goose/Qinghai/05/2005/H5N1, HAQ
<300>
   <308> ABA29447
<400> 25
   Met Glu Arg Ile Val Leu Leu Leu Ala Ile Val Ser Leu Val Lys Gln
   1               5                   10                  15
   Ile Cys Ile Gly Tyr His Ala Asn Asn Ser Thr Glu Gln Val Asp Thr
               20                  25                  30
   Ile Met Glu Lys Asn Val Thr Val Thr His Ala Gln Asp Ile Leu Glu
           35                  40                  45
   Lys Thr His Asn Gly Lys Leu Cys Asp Leu Asp Gly Val Lys Pro Leu
       50                  55                  60
   Ile Leu Arg Asp Cys Ser Val Ala Gly Trp Leu Leu Gly Asn Pro Met
   65                  70                  75                  80
   Cys Asp Glu Phe Leu Asn Val Pro Glu Trp Ser Tyr Ile Val Glu Lys
                   85                  90                  95
   Ile Asn Pro Ala Asn Asp Leu Cys Tyr Pro Gly Asn Phe Asn Asp Tyr
               100                 105                 110
   Glu Glu Leu Lys His Leu Leu Ser Arg Ile Asn His Phe Glu Lys Ile
           115                 120                 125
   Gln Ile Ile Pro Lys Ser Ser Trp Ser Asp His Glu Ala Ser Ser Gly
       130                 135                 140
   Val Ser Ser Ala Cys Pro Tyr Gln Gly Arg Ser Ser Phe Phe Arg Asn
   145                 150                 155                 160
   Val Val Trp Leu Ile Lys Lys Asn Asn Ala Tyr Pro Thr Ile Lys Arg
                   165                 170                 175
   Ser Tyr Asn Asn Thr Asn Gln Glu Asp Leu Leu Val Leu Trp Gly Ile
               180                 185                 190
   His His Pro Asn Asp Ala Ala Glu Gln Thr Arg Leu Tyr Gln Asn Pro
           195                 200                 205
   Thr Thr Tyr Ile Ser Val Gly Thr Ser Thr Leu Asn Gln Arg Leu Val
       210                 215                 220
   Pro Lys Ile Ala Thr Arg Ser Lys Val Asn Gly Gln Ser Gly Arg Met
   225                 230                 235                 240
   Glu Phe Phe Trp Thr Ile Leu Lys Pro Asn Asp Ala Ile Asn Phe Glu
                   245                 250                 255
   Ser Asn Gly Asn Phe Ile Ala Pro Glu Asn Ala Tyr Lys Ile Val Lys
               260                 265                 270
   Lys Gly Asp Ser Thr Ile Met Lys Ser Glu Leu Glu Tyr Gly Asn Cys
           275                 280                 285
   Asn Thr Lys Cys Gln Thr Pro Ile Gly Ala Ile Asn Ser Ser Met Pro
       290                 295                 300
   Phe His Asn Ile His Pro Leu Thr Ile Gly Glu Cys Pro Lys Tyr Val
   305                 310                 315                 320
   Lys Ser Asn Arg Leu Val Leu Ala Thr Gly Leu Arg Asn Ser Pro Gln
                   325                 330                 335
   Gly Glu Arg Arg Arg Lys Lys Arg Gly Leu Phe Gly Ala Ile Ala Gly
               340                 345                 350
   Phe Ile Glu Gly Gly Trp Gln Gly Met Val Asp Gly Trp Tyr Gly Tyr
           355                 360                 365
   His His Ser Asn Glu Gln Gly Ser Gly Tyr Ala Ala Asp Lys Glu Ser
       370                 375                 380
   Thr Gln Lys Ala Ile Asp Gly Val Thr Asn Lys Val Asn Ser Ile Ile
   385                 390                 395                 400
   Asp Lys Met Asn Thr Gln Phe Glu Ala Val Gly Arg Glu Phe Asn Asn
                   405                 410                 415
   Leu Glu Arg Arg Ile Glu Asn Leu Asn Lys Lys Met Glu Asp Gly Phe
               420                 425                 430
   Leu Asp Val Trp Thr Tyr Asn Ala Glu Leu Leu Val Leu Met Glu Asn
           435                 440                 445
   Glu Arg Thr Leu Asp Phe His Asp Ser Asn Val Lys Asn Leu Tyr Asp
       450                 455                 460
   Lys Val Arg Leu Gln Leu Arg Asp Asn Ala Lys Glu Leu Gly Asn Gly
   465                 470                 475                 480
   Cys Phe Glu Phe Tyr His Arg Cys Asp Asn Glu Cys Met Glu Ser Val
                   485                 490                 495
   Arg Asn Gly Thr Tyr Asp Tyr Pro Gln Tyr Ser Glu Glu Ala Arg Leu
               500                 505                 510
   Lys Arg Glu Glu Ile Ser Gly Val Lys Leu Glu Ser Ile Gly Thr Tyr
           515                 520                 525
   Gln Ile Leu Ser Ile Tyr Ser Thr Val Ala Ser Ser Leu Ala Leu Ala
       530                 535                 540
   Ile Met Val Ala Gly Leu Ser Leu Trp Met Cys Ser Asn Gly Ser Leu
   545                 550                 555                 560
   Gln Cys Arg Ile Ala Phe Lys Val
                   565
<210> 26
   <211> 449
   <212> PRT
   <213> Influenza A
<220>
   <223> Neuraminidase, Anhui/1/2005/H5N1, NAA
<300>
   <308> ABU94738
<400> 26
   Met Asn Pro Asn Gln Lys Ile Ile Thr Ile Gly Ser Ile Cys Met Val
   1               5                   10                  15
   Ile Gly Ile Val Ser Leu Met Leu Gln Ile Gly Asn Met Ile Ser Ile
               20                  25                  30
   Trp Val Ser His Ser Ile Gln Thr Gly Asn Gln His Gln Ala Glu Pro
           35                  40                  45
   Ile Arg Asn Ala Asn Phe Leu Thr Glu Asn Ala Val Ala Ser Val Thr
       50                  55                  60
   Leu Ala Gly Asn Ser Ser Leu Cys Pro Val Arg Gly Trp Ala Val His
   65                  70                  75                  80
   Ser Lys Asp Asn Ser Ile Arg Ile Gly Ser Lys Gly Asp Val Phe Val
                   85                  90                  95
   Ile Arg Glu Pro Phe Ile Ser Cys Ser His Leu Glu Cys Arg Thr Phe
                   100                 105                 110
   Phe Leu Thr Gln Gly Ala Leu Leu Asn Asp Lys His Ser Asn Gly Thr
           115                 120                 125
   Val Lys Asp Arg Ser Pro His Arg Thr Leu Met Ser Cys Pro Val Gly
       130                 135                 140
   Glu Ala Pro Ser Pro Tyr Asn Ser Arg Phe Glu Ser Val Ala Trp Ser
   145                 150                 155                 160
   Ala Ser Ala Cys His Asp Gly Thr Ser Trp Leu Thr Ile Gly Ile Ser
                   165                 170                 175
   Gly Pro Asp Asn Gly Ala Val Ala Val Leu Lys Tyr Asn Gly Ile Ile
               180                 185                 190
   Thr Asp Thr Ile Lys Ser Trp Arg Asn Asn Ile Leu Arg Thr Gln Glu
           195                 200                 205
   Ser Glu Cys Ala Cys Val Asn Gly Ser Cys Phe Thr Val Met Thr Asp
       210                 215                 220
   Gly Pro Ser Asn Gly Gln Ala Ser Tyr Lys Ile Phe Lys Met Glu Lys
   225                 230                 235                 240
   Gly Lys Val Val Lys Ser Val Glu Leu Asn Ala Pro Asn Tyr His Tyr
                   245                 250                 255
   Glu Glu Cys Ser Cys Tyr Pro Gly Ala Gly Glu Ile Thr Cys Val Cys
               260                 265                 270
   Arg Asp Asn Trp His Gly Ser Asn Arg Pro Trp Val Ser Phe Asn Gln
           275                 280                 285
   Asn Leu Glu Tyr Gln Ile Gly Tyr Ile Cys Ser Gly Val Phe Gly Asp
       290                 295                 300
   Asn Pro Arg Pro Asn Asp Gly Thr Gly Ser Cys Gly Pro Val Ser Pro
   305                 310                 315                 320
   Asn Gly Ala Tyr Gly Ile Lys Gly Phe Ser Phe Lys Tyr Gly Asn Gly
                   325                 330                 335
   Val Trp Ile Gly Arg Thr Lys Ser Thr Asn Ser Arg Ser Gly Phe Glu
               340                 345                 350
   Met Ile Trp Asp Pro Asn Gly Trp Thr Glu Thr Asp Ser Asn Phe Ser
           355                 360                 365
   Val Lys Gln Asp Ile Val Ala Ile Thr Asp Trp Ser Gly Tyr Ser Gly
       370                 375                 380
   Ser Phe Val Gln His Pro Glu Leu Thr Gly Leu Asp Cys Ile Arg Pro
   385                 390                 395                 400
   Cys Phe Trp Val Glu Leu Ile Arg Gly Arg Pro Lys Glu Ser Thr Ile
                   405                 410                 415
   Trp Thr Ser Gly Ser Ser Ile Ser Phe Cys Gly Val Asn Ser Asp Thr
               420                 425                 430
   Val Gly Trp Ser Trp Pro Asp Gly Ala Glu Leu Pro Phe Thr Ile Asp
           435                 440                 445
   Lys
<210> 27
   <211> 436
   <212> PRT
   <213> Influenza A
<220>
   <223> Neuraminidase, Indonesia/5/2005/H5N1, NAI
<300>
   <308> ABP51965
<400> 27
   Met Asn Pro Asn Gln Lys Ile Ile Thr Ile Gly Ser Ile Cys Met Val
   1               5                   10                  15
   Ile Gly Ile Val Ser Leu Met Leu Gln Ile Gly Asn Met Ile Ser Ile
               20                  25                  30
   Trp Val Ile His Ser Ile Gln Thr Gly Asn Gln His Gln Ala Glu Ser
           35                  40                  45
   Ile Ser Asn Thr Asn Pro Leu Thr Glu Lys Ala Val Ala Ser Val Thr
       50                  55                  60
   Leu Ala Gly Asn Ser Ser Leu Cys Pro Ile Arg Gly Trp Ala Val His
   65                  70                  75                  80
   Ser Lys Asp Asn Asn Ile Arg Ile Gly Ser Lys Gly Asp Val Phe Val
                   85                  90                  95
   Ile Arg Glu Pro Phe Ile Ser Cys Ser His Leu Glu Cys Arg Thr Phe
               100                 105                 110
   Phe Leu Thr Gln Gly Ala Leu Leu Asn Asp Lys His Ser Asn Gly Thr
           115                 120                 125
   Val Lys Asp Arg Ser Pro His Arg Thr Leu Met Ser Cys Pro Val Gly
       130                 135                 140
   Glu Ala Pro Ser Pro Tyr Asn Ser Arg Phe Glu Ser Val Ala Trp Ser
   145                 150                 155                 160
   Ala Ser Ala Cys His Asp Gly Thr Ser Trp Leu Thr Ile Gly Ile Ser
                   165                 170                 175
   Gly Pro Asp Asn Glu Ala Val Ala Val Leu Lys Tyr Asn Gly Ile Ile
               180                 185                 190
   Thr Asp Thr Ile Lys Ser Trp Arg Asn Asp Ile Leu Arg Thr Gln Glu
           195                 200                 205
   Ser Glu Cys Ala Cys Val Asn Gly Ser Cys Phe Thr Val Met Thr Asp
       210                 215                 220
   Gly Pro Ser Asn Gly Gln Ala Ser Tyr Lys Ile Phe Lys Met Glu Lys
   225                 230                 235                 240
   Gly Lys Val Val Lys Ser Val Glu Leu Asp Ala Pro Asn Tyr His Tyr
                   245                 250                 255
   Glu Glu Cys Ser Cys Tyr Pro Asp Ala Gly Glu Ile Thr Cys Val Cys
               260                 265                 270
   Arg Asp Asn Trp His Gly Ser Asn Arg Pro Trp Val Ser Phe Asn Gln
           275                 280                 285
   Asn Leu Glu Tyr Gln Ile Gly Tyr Ile Cys Ser Gly Val Phe Gly Asp
       290                 295                 300
   Asn Pro Arg Pro Asn Asp Gly Thr Gly Ser Cys Gly Pro Met Ser Pro
   305                 310                 315                 320
   Asn Gly Ala Tyr Gly Val Lys Gly Phe Ser Phe Lys Tyr Gly Asn Gly
                   325                 330                 335
   Val Trp Ile Gly Arg Thr Lys Ser Thr Asn Ser Arg Ser Gly Phe Glu
               340                 345                 350
   Met Ile Trp Asp Pro Asn Gly Trp Thr Gly Thr Asp Ser Ser Phe Ser
           355                 360                 365
   Val Lys Gln Asp Ile Val Ala Ile Thr Asp Trp Ser Gly Tyr Ser Gly
   370 375 380
   Ser Phe Val Gln His Pro Glu Leu Thr Gly Leu Asp Cys Ile Arg Pro
   385 390 395 400
   Cys Phe Trp Val Glu Leu Ile Arg Gly Arg Pro Lys Glu Ser Thr Ile
   405 410 415
   Trp Thr Ser Gly Ser Ser Ile Ser Phe Cys Gly Val Asn Ser Asp Thr
   420 425 430
   Val Ser Trp Ser
   435
<210> 28
   <211> 471
   <212> PRT
   <213> Influenza A
<220>
   <223> Neuraminidase, Netherlands/219/2003/H7N7, NANL
<300>
   <308> AAR11367
<400> 28
   Met Asn Pro Asn Gln Lys Leu Phe Ala Leu Ser Gly Val Ala Ile Ala
   1               5                   10                  15
   Leu Ser Val Leu Asn Leu Leu Ile Gly Ile Ser Asn Val Gly Leu Asn
               20                  25                  30
   Val Ser Leu His Leu Lys Glu Lys Gly Pro Lys Gln Glu Glu Asn Leu
           35                  40                  45
   Thr Cys Thr Thr Ile Asn Gln Asn Asn Thr Thr Val Val Glu Asn Thr
       50                  55                  60
   Tyr Val Asn Asn Thr Thr Ile Ile Thr Lys Gly Thr Asp Leu Lys Thr
   65                  70                  75                  80
   Pro Ser Tyr Leu Leu Leu Asn Lys Ser Leu Cys Asn Val Glu Gly Trp
                   85                  90                  95
   Val Val Ile Ala Lys Asp Asn Ala Val Arg Phe Gly Glu Ser Glu Gln
               100                 105                 110
   Ile Ile Val Thr Arg Glu Pro Tyr Val Ser Cys Asp Pro Thr Gly Cys
           115                 120                 125
   Lys Met Tyr Ala Leu His Gln Gly Thr Thr Ile Arg Asn Lys His Ser
       130                 135                 140
   Asn Gly Thr Ile His Asp Arg Thr Ala Phe Arg Gly Leu Ile Ser Thr
   145                 150                 155                 160
   Pro Leu Gly Thr Pro Pro Thr Val Ser Asn Ser Asp Phe Met Cys Val
                   165                 170                 175
   Gly Trp Ser Ser Thr Thr Cys His Asp Gly Ile Ala Arg Met Thr Ile
               180                 185                 190
   Cys Ile Gln Gly Asn Asn Asp Asn Ala Thr Ala Thr Val Tyr Tyr Asn
           195                 200                 205
   Arg Arg Leu Thr Thr Thr Ile Lys Thr Trp Ala Arg Asn Ile Leu Arg
       210                 215                 220
   Thr Gln Glu Ser Glu Cys Val Cys His Asn Gly Thr Cys Ala Val Val
   225                 230                 235                 240
   Met Thr Asp Gly Ser Ala Ser Ser Gln Ala Tyr Thr Lys Val Met Tyr
                   245                 250                 255
   Phe His Lys Gly Leu Val Val Lys Glu Glu Glu Leu Arg Gly Ser Ala
               260                 265                 270
   Arg His Ile Glu Glu Cys Ser Cys Tyr Gly His Asn Gln Lys Val Thr
           275                 280                 285
   Cys Val Cys Arg Asp Asn Trp Gln Gly Ala Asn Arg Pro Ile Ile Glu
       290                 295                 300
   Ile Asp Met Ser Thr Leu Glu His Thr Ser Arg Tyr Val Cys Thr Gly
   305                 310                 315                 320
   Ile Leu Thr Asp Thr Ser Arg Pro Gly Asp Lys Ser Ser Gly Asp Cys
                   325                 330                 335
   Ser Asn Pro Ile Thr Gly Ser Pro Gly Val Pro Gly Val Lys Gly Phe
               340                 345                 350
   Gly Phe Leu Asn Gly Asp Asn Thr Trp Leu Gly Arg Thr Ile Ser Pro
           355                 360                 365
   Arg Ser Arg Ser Gly Phe Glu Met Leu Lys Ile Pro Asn Ala Gly Thr
       370                 375                 380
   Asp Pro Asn Ser Arg Ile Ala Glu Arg Gln Glu Ile Val Asp Asn Asn
   385 390 395 400
   Asn Trp Ser Gly Tyr Ser Gly Ser Phe Ile Asp Tyr Trp Asn Asp Asn
   405 410 415
   Ser Glu Cys Tyr Asn Pro Cys Phe Tyr Val Glu Leu Ile Arg Gly Arg
   420 425 430
   Pro Glu Glu Ala Lys Tyr Val Trp Trp Ala Ser Asn Ser Leu Ile Ala
   435 440 445
   Leu Cys Gly Ser Pro Phe Pro Val Gly Ser Gly Ser Phe Pro Asp Gly
   450 455 460
   Ala Gln Ile Gln Tyr Phe Ser
   465 470
<210> 29
   <211> 449
   <212> PRT
   <213> Influenza A
<220>
   <223> Neuraminidase, bar-headed goose/Qinghai/05/2005/H5N1, NAQ
<300>
   <308> ABA29448
<400> 29
   Met Asn Pro Asn Gln Lys Ile Ile Thr Ile Gly Ser Ile Cys Met Val
   1               5                   10                  15
   Ile Gly Ile Val Ser Leu Met Leu Gln Ile Gly Asn Met Ile Ser Ile
               20                  25                  30
   Trp Val Ser His Ser Ile Gln Thr Gly Asn Gln Arg Gln Ala Glu Pro
           35                  40                  45
   Ile Ser Asn Thr Lys Phe Leu Thr Glu Lys Ala Val Ala Ser Val Thr
       50                  55                  60
   Leu Ala Gly Asn Ser Ser Leu Cys Pro Ile Ser Gly Trp Ala Val Tyr
   65                  70                  75                  80
   Ser Lys Asp Asn Ser Ile Arg Ile Gly Ser Arg Gly Asp Val Phe Val
                   85                  90                  95
   Ile Arg Glu Pro Phe Ile Ser Cys Ser His Leu Glu Cys Arg Thr Phe
               100                 105                 110
   Phe Leu Thr Gln Gly Ala Leu Leu Asn Asp Lys His Ser Asn Gly Thr
             115                 120                 125
   Val Lys Asp Arg Ser Pro His Arg Thr Leu Met Ser Cys Pro Val Gly
       130                 135                 140
   Glu Ala Pro Ser Pro Tyr Asn Ser Arg Phe Glu Ser Val Ala Trp Ser
   145                 150                 155                 160
   Ala Ser Ala Cys His Asp Gly Thr Ser Trp Leu Thr Ile Gly Ile Ser
                   165                 170                 175
   Gly Pro Asp Asn Gly Ala Val Ala Val Leu Lys Tyr Asn Gly Ile Ile
               180                 185                 190
   Thr Asp Thr Ile Lys Ser Trp Arg Asn Asn Ile Leu Arg Thr Gln Glu
           195                 200                 205
   Ser Glu Cys Ala Cys Val Asn Gly Ser Cys Phe Thr Val Met Thr Asp
       210                 215                 220
   Gly Pro Ser Asn Gly Gln Ala Ser Tyr Lys Ile Phe Lys Met Glu Lys
   225                 230                 235                 240
   Gly Lys Val Val Lys Ser Val Glu Leu Asp Ala Pro Asn Tyr His Tyr
                   245                 250                 255
   Glu Glu Cys Ser Cys Tyr Pro Asp Ala Gly Glu Ile Thr Cys Val Cys
               260                 265                 270
   Arg Asp Asn Trp His Gly Ser Asn Arg Pro Trp Val Ser Phe Asn Gln
           275                 280                 285
   Asn Leu Glu Tyr Gln Ile Gly Tyr Ile Cys Ser Gly Val Phe Gly Asp
       290                 295                 300
   Asn Pro Arg Pro Asn Asp Gly Thr Gly Ser Cys Gly Pro Val Ser Pro
   305                 310                 315                 320
   Asn Gly Ala Tyr Gly Val Lys Gly Phe Ser Phe Lys Tyr Gly Asn Gly
                   325                 330                 335
   Val Trp Ile Gly Arg Thr Lys Ser Thr Asn Ser Arg Ser Gly Phe Glu
               340                 345                 350
   Met Ile Trp Asp Pro Asn Gly Trp Thr Gly Thr Asp Ser Ser Phe Ser
           355                 360                 365
   Val Lys Gln Asp Ile Val Ala Ile Thr Asp Trp Ser Gly Tyr Ser Gly
   370 375 380
   Ser Phe Val Gln His Pro Glu Leu Thr Gly Leu Asp Cys Ile Arg Pro
   385 390 395 400
   Cys Phe Trp Val Glu Leu Ile Arg Gly Arg Pro Lys Glu Ser Thr Ile
   405 410 415
   Trp Thr Ser Gly Ser Ser Ile Ser Phe Cys Gly Val Asn Ser Asp Thr
   420 425 430
   Val Ser Trp Ser Trp Pro Asp Gly Ala Glu Leu Pro Phe Thr Ile Asp
   435 440 445
   Lys
<210> 30
   <211> 449
   <212> PRT
   <213> Influenza A
<220>
   <223> Neuraminidase, Vietnam/1194/2004/H5N1, NAV
<300>
   <308> ABP52007
<400> 30
   Met Asn Pro Asn Gln Lys Ile Ile Thr Ile Gly Ser Ile Cys Met Val
   1               5                   10                  15
   Thr Gly Ile Val Ser Leu Met Leu Gln Ile Gly Asn Met Ile Ser Ile
               20                  25                  30
   Trp Val Ser His Ser Ile His Thr Gly Asn Gln His Gln Ser Glu Pro
           35                  40                  45
   Ile Ser Asn Thr Asn Leu Leu Thr Glu Lys Ala Val Ala Ser Val Lys
       50                  55                  60
   Leu Ala Gly Asn Ser Ser Leu Cys Pro Ile Asn Gly Trp Ala Val Tyr
   65                  70                  75                  80
   Ser Lys Asp Asn Ser Ile Arg Ile Gly Ser Lys Gly Asp Val Phe Val
                   85                  90                  95
   Ile Arg Glu Pro Phe Ile Ser Cys Ser His Leu Glu Cys Arg Thr Phe
               100                 105                 110
   Phe Leu Thr Gln Gly Ala Leu Leu Asn Asp Lys His Ser Asn Gly Thr
           115                 120                 125
   Val Lys Asp Arg Ser Pro His Arg Thr Leu Met Ser Cys Pro Val Gly
       130                 135                 140
   Glu Ala Pro Ser Pro Tyr Asn Ser Arg Phe Glu Ser Val Ala Trp Ser
   145                 150                 155                 160
   Ala Ser Ala Cys His Asp Gly Thr Ser Trp Leu Thr Ile Gly Ile Ser
                   165                 170                 175
   Gly Pro Asp Asn Gly Ala Val Ala Val Leu Lys Tyr Asn Gly Ile Ile
               180                 185                 190
   Thr Asp Thr Ile Lys Ser Trp Arg Asn Asn Ile Leu Arg Thr Gln Glu
           195                 200                 205
   Ser Glu Cys Ala Cys Val Asn Gly Ser Cys Phe Thr Val Met Thr Asp
       210                 215                 220
   Gly Pro Ser Asn Gly Gln Ala Ser His Lys Ile Phe Lys Met Glu Lys
   225                 230                 235                 240
   Gly Lys Val Val Lys Ser Val Glu Leu Asp Ala Pro Asn Tyr His Tyr
                   245                 250                 255
   Glu Glu Cys Ser Cys Tyr Pro Asp Ala Gly Glu Ile Thr Cys Val Cys
               260                 265                 270
   Arg Asp Asn Trp His Gly Ser Asn Arg Pro Trp Val Ser Phe Asn Gln
           275                 280                 285
   Asn Leu Glu Tyr Gln Ile Gly Tyr Ile Cys Ser Gly Val Phe Gly Asp
       290                 295                 300
   Asn Pro Arg Pro Asn Asp Gly Thr Gly Ser Cys Gly Pro Val Ser Ser
   305                 310                 315                 320
   Asn Gly Ala Gly Gly Val Lys Gly Phe Ser Phe Lys Tyr Gly Asn Gly
                   325                 330                 335
   Val Trp Ile Gly Arg Thr Lys Ser Thr Asn Ser Arg Ser Gly Phe Glu
               340                 345                 350
   Met Ile Trp Asp Pro Asn Gly Trp Thr Glu Thr Asp Ser Ser Phe Ser
           355                 360                 365
   Val Lys Gln Asp Ile Val Ala Ile Thr Asp Trp Ser Gly Tyr Ser Gly
       370                 375                 380
   Ser Phe Val Gln His Pro Glu Leu Thr Gly Leu Asp Cys Ile Arg Pro
   385 390 395 400
   Cys Phe Trp Val Glu Leu Ile Arg Gly Arg Pro Lys Glu Ser Thr Ile
   405 410 415
   Trp Thr Ser Gly Ser Ser Ile Ser Phe Cys Gly Val Asn Ser Asp Thr
   420 425 430
   Val Gly Trp Ser Trp Pro Asp Gly Ala Glu Leu Pro Phe Thr Ile Asp
   435 440 445
   Lys
<210> 31
   <211> 469
   <212> PRT
   <213> Influenza A
<220>
   <223> Neuraminidase, California/04/2009/H1N1, NAC04
<300>
   <308> ACP41107
<400> 31
   Met Asn Pro Asn Gln Lys Ile Ile Thr Ile Gly Ser Val Cys Met Thr
   1               5                   10                  15
   Ile Gly Met Ala Asn Leu Ile Leu Gln Ile Gly Asn Ile Ile Ser Ile
               20                  25                  30
   Trp Ile Ser His Ser Ile Gln Leu Gly Asn Gln Asn Gln Ile Glu Thr
           35                  40                  45
   Cys Asn Gln Ser Val Ile Thr Tyr Glu Asn Asn Thr Trp Val Asn Gln
       50                  55                  60
   Thr Tyr Val Asn Ile Ser Asn Thr Asn Phe Ala Ala Gly Gln Ser Val
   65                  70                  75                  80
   Val Ser Val Lys Leu Ala Gly Asn Ser Ser Leu Cys Pro Val Ser Gly
                   85                  90                  95
   Trp Ala Ile Tyr Ser Lys Asp Asn Ser Val Arg Ile Gly Ser Lys Gly
               100                 105                 110
   Asp Val Phe Val Ile Arg Glu Pro Phe Ile Ser Cys Ser Pro Leu Glu
           115                 120                 125
   Cys Arg Thr Phe Phe Leu Thr Gln Gly Ala Leu Leu Asn Asp Lys His
       130                 135                 140
   Ser Asn Gly Thr Ile Lys Asp Arg Ser Pro Tyr Arg Thr Leu Met Ser
   145                 150                 155                 160
   Cys Pro Ile Gly Glu Val Pro Ser Pro Tyr Asn Ser Arg Phe Glu Ser
                   165                 170                 175
   Val Ala Trp Ser Ala Ser Ala Cys His Asp Gly Ile Asn Trp Leu Thr
               180                 185                 190
   Ile Gly Ile Ser Gly Pro Asp Asn Gly Ala Val Ala Val Leu Lys Tyr
           195                 200                 205
   Asn Gly Ile Ile Thr Asp Thr Ile Lys Ser Trp Arg Asn Asn Ile Leu
       210                 215                 220
   Arg Thr Gln Glu Ser Glu Cys Ala Cys Val Asn Gly Ser Cys Phe Thr
   225                 230                 235                 240
   Val Met Thr Asp Gly Pro Ser Asn Gly Gln Ala Ser Tyr Lys Ile Phe
                   245                 250                 255
   Arg Ile Glu Lys Gly Lys Ile Val Lys Ser Val Glu Met Asn Ala Pro
               260                 265                 270
   Asn Tyr His Tyr Glu Glu Cys Ser Cys Tyr Pro Asp Ser Ser Glu Ile
           275                 280                 285
   Thr Cys Val Cys Arg Asp Asn Trp His Gly Ser Asn Arg Pro Trp Val
       290                 295                 300
   Ser Phe Asn Gln Asn Leu Glu Tyr Gln Ile Gly Tyr Ile Cys Ser Gly
   305                 310                 315                 320
   Ile Phe Gly Asp Asn Pro Arg Pro Asn Asp Lys Thr Gly Ser Cys Gly
                   325                 330                 335
   Pro Val Ser Ser Asn Gly Ala Asn Gly Val Lys Gly Phe Ser Phe Lys
               340                 345                 350
   Tyr Gly Asn Gly Val Trp Ile Gly Arg Thr Lys Ser Ile Ser Ser Arg
           355                 360                 365
   Asn Gly Phe Glu Met Ile Trp Asp Pro Asn Gly Trp Thr Gly Thr Asp
       370                 375                 380
   Asn Asn Phe Ser Ile Lys Gln Asp Ile Val Gly Ile Asn Glu Trp Ser
   385 390 395 400
   Gly Tyr Ser Gly Ser Phe Val Gln His Pro Glu Leu Thr Gly Leu Asp
   405 410 415
   Cys Ile Arg Pro Cys Phe Trp Val Glu Leu Ile Arg Gly Arg Pro Lys
   420 425 430
   Glu Asn Thr Ile Trp Thr Ser Gly Ser Ser Ile Ser Phe Cys Gly Val
   435 440 445
   Asn Ser Asp Thr Val Gly Trp Ser Trp Pro Asp Gly Ala Glu Leu Pro
   450 455 460
   Phe Thr Ile Asp Lys
   465
<210> 32
   <211> 469
   <212> PRT
   <213> Influenza A
<220>
   <223> Neuraminidase, California/06/2009/H1N1, NAC06
<300>
   <308> ACP52564
<400> 32
   Met Asn Pro Asn Gln Lys Ile Ile Thr Ile Gly Ser Val Cys Met Thr
   1               5                   10                  15
   Ile Gly Met Ala Asn Leu Ile Leu Gln Ile Gly Asn Ile Ile Ser Ile
               20                  25                  30
   Trp Ile Ser His Ser Ile Gln Leu Gly Asn Gln Asn Gln Ile Glu Thr
           35                  40                  45
   Cys Asn Gln Ser Val Ile Thr Tyr Glu Asn Asn Thr Trp Val Asn Gln
       50                  55                  60
   Thr Tyr Val Asn Ile Ser Asn Thr Asn Phe Ala Ala Gly Gln Ser Val
   65                  70                  75                  80
   Val Ser Val Lys Leu Ala Gly Asn Ser Ser Leu Cys Pro Val Gly Gly
                   85                  90                  95
   Trp Ala Ile Tyr Ser Lys Asp Asn Ser Val Arg Ile Gly Ser Lys Gly
               100                 105                 110
   Asp Val Phe Val Ile Arg Glu Pro Phe Ile Ser Cys Ser Pro Leu Glu
           115                 120                 125
   Cys Arg Thr Phe Phe Leu Thr Gln Gly Ala Leu Leu Asn Asp Lys His
       130                 135                 140
   Ser Asn Gly Thr Ile Lys Asp Arg Ser Pro Tyr Arg Thr Leu Met Ser
   145                 150                 155                 160
   Cys Pro Ile Gly Glu Val Pro Ser Pro Tyr Asn Ser Arg Phe Glu Ser
                   165                 170                 175
   Val Ala Trp Ser Ala Ser Ala Cys His Asp Gly Ile Asn Trp Leu Thr
               180                 185                 190
   Ile Gly Ile Ser Gly Pro Asp Asn Gly Ala Val Ala Val Leu Lys Tyr
           195                 200                 205
   Asn Gly Ile Ile Thr Asp Thr Ile Lys Ser Trp Arg Asn Asn Ile Leu
       210                 215                 220
   Arg Thr Gln Glu Ser Glu Cys Ala Cys Val Asn Gly Ser Cys Phe Thr
   225                 230                 235                 240
   Val Met Thr Asp Gly Pro Ser Asn Gly Gln Ala Ser Tyr Lys Ile Phe
                   245                 250                 255
   Arg Ile Glu Lys Gly Lys Ile Val Lys Ser Val Glu Met Asn Ala Pro
               260                 265                 270
   Asn Tyr His Tyr Glu Glu Cys Ser Cys Tyr Pro Asp Ser Ser Glu Ile
           275                 280                 285
   Thr Cys Val Cys Arg Asp Asn Trp His Gly Ser Asn Arg Pro Trp Val
       290                 295                 300
   Ser Phe Asn Gln Asn Leu Glu Tyr Gln Ile Gly Tyr Ile Cys Ser Gly
   305                 310                 315                 320
   Ile Phe Gly Asp Asn Pro Arg Pro Asn Asp Lys Thr Gly Ser Cys Gly
                   325                 330                 335
   Pro Val Ser Ser Asn Gly Ala Asn Gly Val Lys Gly Phe Ser Phe Lys
               340                 345                 350
   Tyr Gly Asn Gly Val Trp Ile Gly Arg Thr Lys Ser Ile Ser Ser Arg
           355                 360                 365
   Asn Gly Phe Glu Met Ile Trp Asp Pro Asn Gly Trp Thr Gly Thr Asp
   370 375 380
   Asn Asn Phe Ser Ile Lys Gln Asp Ile Val Gly Ile Asn Glu Trp Ser
   385 390 395 400
   Gly Tyr Ser Gly Ser Phe Val Gln His Pro Glu Leu Thr Gly Leu Asp
   405 410 415
   Cys Ile Arg Pro Cys Phe Trp Val Glu Leu Ile Arg Gly Arg Pro Lys
   420 425 430
   Glu Asn Thr Ile Trp Thr Ser Gly Ser Ser Ile Ser Phe Cys Gly Val
   435 440 445
   Asn Ser Asp Thr Val Gly Trp Ser Trp Pro Asp Gly Ala Glu Leu Pro
   450 455 460
   Phe Thr Ile Asp Lys
   465
<210> 33
   <211> 469
   <212> PRT
   <213> Influenza A
<220>
   <223> Neuraminidase, Brisbane/10/2007/H3N2, NAB(N2)
<300>
   <308> ABW23407
<400> 33
   Met Asn Pro Asn Gln Lys Ile Ile Thr Ile Gly Ser Val Ser Leu Thr
   1               5                   10                  15
   Ile Ser Thr Ile Cys Phe Phe Met Gln Ile Ala Ile Leu Ile Thr Thr
               20                  25                  30
   Val Thr Leu His Phe Lys Gln Tyr Glu Phe Asn Ser Pro Pro Asn Asn
           35                  40                  45
   Gln Val Met Leu Cys Glu Pro Thr Ile Ile Glu Arg Asn Ile Thr Glu
       50                  55                  60
   Ile Val Tyr Leu Thr Asn Thr Thr Ile Glu Lys Glu Ile Cys Pro Lys
   65                  70                  75                  80
   Leu Ala Glu Tyr Arg Asn Trp Ser Lys Pro Gln Cys Asp Ile Thr Gly
                   85                  90                  95
   Phe Ala Pro Phe Ser Lys Asp Asn Ser Ile Arg Leu Ser Ala Gly Gly
               100                 105                 110
   Asp Ile Trp Val Thr Arg Glu Pro Tyr Val Ser Cys Asp Pro Asp Lys
           115                 120                 125
   Cys Tyr Gln Phe Ala Leu Gly Gln Gly Thr Thr Leu Asn Asn Val His
       130                 135                 140
   Ser Asn Asp Thr Val Arg Asp Arg Thr Pro Tyr Arg Thr Leu Leu Met
   145                 150                 155                 160
   Asn Glu Leu Gly Val Pro Phe His Leu Gly Thr Lys Gln Val Cys Ile
                   165                 170                 175
   Ala Trp Ser Ser Ser Ser Cys His Asp Gly Lys Ala Trp Leu His Val
               180                 185                 190
   Cys Ile Thr Gly Asp Asp Lys Asn Ala Thr Ala Ser Phe Ile Tyr Asn
           195                 200                 205
   Gly Arg Leu Val Asp Ser Ile Val Ser Trp Ser Lys Glu Ile Leu Arg
       210                 215                 220
   Thr Gln Glu Ser Glu Cys Val Cys Ile Asn Gly Thr Cys Thr Val Val
   225                 230                 235                 240
   Met Thr Asp Gly Ser Ala Ser Gly Lys Ala Asp Thr Lys Ile Leu Phe
                   245                 250                 255
   Ile Glu Glu Gly Lys Ile Val His Thr Ser Thr Leu Ser Gly Ser Ala
               260                 265                 270
   Gln His Val Glu Glu Cys Ser Cys Tyr Pro Arg Tyr Pro Gly Val Arg
           275                 280                 285
   Cys Val Cys Arg Asp Asn Trp Lys Gly Ser Asn Arg Pro Ile Val Asp
       290                 295                 300
   Ile Asn Ile Lys Asp His Ser Thr Val Ser Ser Tyr Val Cys Ser Gly
   305                 310                 315                 320
   Leu Val Gly Asp Thr Pro Arg Lys Asn Asp Ser Ser Ser Ser Ser His
                   325                 330                 335
   Cys Leu Asp Pro Asn Asn Glu Glu Gly Gly His Gly Val Lys Gly Trp
               340                 345                 350
   Ala Phe Asp Asp Gly Asn Asp Val Trp Met Gly Arg Thr Ile Ser Glu
   355 360 365
   Lys Ser Arg Leu Gly Tyr Glu Thr Phe Lys Val Ile Glu Gly Trp Ser
   370 375 380
   Asn Pro Lys Ser Lys Leu Gln Ile Asn Arg Gln Val Ile Val Asp Arg
   385 390 395 400
   Gly Asn Arg Ser Gly Tyr Ser Gly Ile Phe Ser Val Glu Gly Lys Ser
   405 410 415
   Cys Ile Asn Arg Cys Phe Tyr Val Glu Leu Ile Arg Gly Arg Lys Glu
   420 425 430
   Glu Thr Glu Val Leu Trp Thr Ser Asn Ser Ile Val Val Phe Cys Gly
   435 440 445
   Thr Ser Gly Thr Tyr Gly Thr Gly Ser Trp Pro Asp Gly Ala Asp Ile
   450 455 460
   Asn Leu Met Pro Ile
   465
<210> 34
   <211> 466
   <212> PRT
   <213> Influenza B
<220>
   <223> Neuraminidase, Brisbane like sequence, NAB(B)
<300>
   <308> ACN29381
<400> 34
   Met Leu Pro Ser Thr Ile Gln Thr Leu Thr Leu Phe Leu Thr Ser Gly
   1               5                   10                  15
   Gly Val Leu Leu Ser Leu Tyr Val Ser Ala Ser Leu Ser Tyr Leu Leu
               20                  25                  30
   Tyr Ser Asp Ile Leu Leu Lys Phe Ser Pro Thr Glu Ile Thr Ala Pro
           35                  40                  45
   Thr Met Pro Leu Asp Cys Ala Asn Ala Ser Asn Val Gln Ala Val Asn
       50                  55                  60
   Arg Ser Ala Thr Lys Gly Val Thr Leu Leu Leu Pro Glu Pro Glu Trp
   65                  70                  75                  80
   Thr Tyr Pro Arg Leu Ser Cys Pro Gly Ser Thr Phe Gln Lys Ala Leu
   85 90 95
   Leu Ile Ser Pro His Arg Phe Gly Glu Thr Lys Gly Asn Ser Ala Pro
   100 105 110
   Leu Ile Ile Arg Glu Pro Phe Ile Ala Cys Gly Pro Asn Glu Cys Lys
   115 120 125
   His Phe Ala Leu Thr His Tyr Ala Ala Gln Pro Gly Gly Tyr Tyr Asn
   130 135 140
   Gly Thr Arg Gly Asp Arg Asn Lys Leu Arg His Leu Ile Ser Val Lys
   145 150 155 160
   Leu Gly Lys Ile Pro Thr Val Glu Asn Ser Ile Phe His Met Ala Ala
   165 170 175
   Trp Ser Gly Ser Ala Cys His Asp Gly Lys Glu Trp Thr Tyr Ile Gly
   180 185 190
   Val Asp Gly Pro Asp Asn Asn Ala Leu Leu Lys Val Lys Tyr Gly Glu
   195 200 205
   Ala Tyr Thr Asp Thr Tyr His Ser Tyr Ala Asn Lys Ile Leu Arg Thr
   210 215 220
   Gln Glu Ser Ala Cys Asn Cys Ile Gly Gly Asn Cys Tyr Leu Met Ile
   225 230 235 240
   Thr Asp Gly Ser Ala Ser Gly Val Ser Glu Cys Arg Phe Leu Lys Ile
   245 250 255
   Arg Glu Gly Arg Ile Ile Lys Glu Ile Phe Pro Thr Gly Arg Val Lys
   260 265 270
   His Thr Glu Glu Cys Thr Cys Gly Phe Ala Ser Asn Lys Thr Ile Glu
   275 280 285
   Cys Ala Cys Arg Asp Asn Ser Tyr Thr Ala Lys Arg Pro Phe Val Lys
   290 295 300
   Leu Asn Val Glu Thr Asp Thr Ala Glu Ile Arg Leu Met Cys Thr Asp
   305 310 315 320
   Thr Tyr Leu Asp Thr Pro Arg Pro Asn Asp Gly Ser Ile Thr Gly Pro
   325 330 335
   Cys Glu Ser Asn Gly Asp Lys Gly Ser Gly Gly Ile Lys Gly Gly Phe
   340 345 350
   Val His Gln Arg Met Glu Ser Lys Ile Gly Arg Trp Tyr Ser Arg Thr
           355                 360                 365
   Met Ser Lys Thr Glu Arg Met Gly Met Gly Leu Tyr Val Lys Tyr Asp
       370                 375                 380
   Gly Asp Pro Trp Ala Asp Ser Asp Ala Leu Ala Phe Ser Gly Val Met
   385                 390                 395                 400
   Val Ser Met Lys Glu Pro Gly Trp Tyr Ser Phe Gly Phe Glu Ile Lys
                   405                 410                 415
   Asp Lys Lys Cys Asp Val Pro Cys Ile Gly Ile Glu Met Val His Asp
               420                 425                 430
   Gly Gly Lys Glu Thr Trp His Ser Ala Ala Thr Ala Ile Tyr Cys Leu
           435                 440                 445
   Met Gly Ser Gly Gln Leu Leu Trp Asp Thr Val Thr Gly Val Asp Met
       450                 455                 460
   Ala Leu
   465
<210> 35
   <211> 467
   <212> PRT
   <213> Influenza A
<220>
   <223> Neuraminidase, Brisbane/59/2007/H1N1, NAB(N1)
<300>
   <308> ACA28847
<400> 35
   Met Asn Pro Asn Gln Lys Ile Ile Thr Ile Gly Ser Ile Ser Ile Ala
   1               5                   10                  15
   Ile Gly Ile Ile Ser Leu Met Leu Gln Ile Gly Asn Ile Ile Ser Ile
               20                  25                  30
   Trp Ala Ser His Ser Ile Gln Thr Gly Ser Gln Asn Asn Thr Gly Ile
           35                  40                  45
   Cys Asn Gln Arg Ile Ile Thr Tyr Glu Asn Ser Thr Trp Val Asn His
       50                  55                  60
   Thr Tyr Val Asn Ile Asn Asn Thr Asn Val Val Ala Gly Glu Asp Lys
   65                  70                  75                  80
   Thr Ser Val Thr Leu Ala Gly Asn Ser Ser Leu Cys Ser Ile Ser Gly
                   85                  90                  95
   Trp Ala Ile Tyr Thr Lys Asp Asn Ser Ile Arg Ile Gly Ser Lys Gly
               100                 105                 110
   Asp Val Phe Val Ile Arg Glu Pro Phe Ile Ser Cys Ser His Leu Glu
           115                 120                 125
   Cys Arg Thr Phe Phe Leu Thr Gln Gly Ala Leu Leu Asn Asp Lys His
       130                 135                 140
   Ser Asn Gly Thr Val Lys Asp Arg Ser Pro Tyr Arg Ala Leu Met Ser
   145                 150                 155                 160
   Cys Pro Leu Gly Glu Ala Pro Ser Pro Tyr Asn Ser Lys Phe Glu Ser
                   165                 170                 175
   Val Ala Trp Ser Ala Ser Ala Cys His Asp Gly Met Gly Trp Leu Thr
               180                 185                 190
   Ile Gly Ile Ser Gly Pro Asp Asn Gly Ala Val Ala Val Leu Lys Tyr
           195                 200                 205
   Asn Gly Ile Ile Thr Gly Thr Ile Lys Ser Trp Lys Lys Gln Ile Leu
       210                 215                 220
   Arg Thr Gln Glu Ser Glu Cys Val Cys Met Asn Gly Ser Cys Phe Thr
   225                 230                 235                 240
   Ile Met Thr Asp Gly Pro Ser Asn Lys Ala Ala Ser Tyr Lys Ile Phe
                   245                 250                 255
   Lys Ile Glu Lys Gly Lys Val Thr Lys Ser Ile Glu Leu Asn Ala Pro
               260                 265                 270
   Asn Phe His Tyr Glu Glu Cys Ser Cys Tyr Pro Asp Thr Gly Ile Val
           275                 280                 285
   Met Cys Val Cys Arg Asp Asn Trp His Gly Ser Asn Arg Pro Trp Val
       290                 295                 300
   Ser Phe Asn Gln Asn Leu Asp Tyr Gln Ile Gly Tyr Ile Cys Ser Gly
   305                 310                 315                 320
   Val Phe Gly Asp Asn Pro Arg Pro Glu Asp Gly Glu Gly Ser Cys Asn
                   325                 330                 335
   Pro Val Thr Val Asp Gly Ala Asn Gly Val Lys Gly Phe Ser Tyr Lys
               340                 345                 350
   Tyr Asp Asn Gly Val Trp Ile Gly Arg Thr Lys Ser Asn Arg Leu Arg
           355                 360                 365
   Lys Gly Phe Glu Met Ile Trp Asp Pro Asn Gly Trp Thr Asn Thr Asp
       370                 375                 380
   Ser Asp Phe Ser Val Lys Gln Asp Val Val Ala Ile Thr Asp Trp Ser
   385                 390                 395                 400
   Gly Tyr Ser Gly Ser Phe Val Gln His Pro Glu Leu Thr Gly Leu Asp
                   405                 410                 415
   Cys Ile Arg Pro Cys Phe Trp Val Glu Leu Val Arg Gly Leu Pro Arg
               420                 425                 430
   Glu Asn Thr Thr Ile Trp Thr Ser Gly Ser Ser Ile Ser Phe Cys Gly
           435                 440                 445
   Val Asn Ser Asp Thr Ala Asn Trp Ser Trp Pro Asp Gly Ala Glu Leu
       450                 455                 460
   Pro Phe Thr
   465
<210> 36
   <211> 466
   <212> PRT
   <213> Influenza B
<220>
   <223> Neuraminidase, Florida/4/2006, NAF
<300>
   <308> ACA33351
<400> 36
   Met Leu Pro Ser Thr Ile Gln Thr Leu Thr Leu Phe Leu Thr Ser Gly
   1               5                   10                  15
   Gly Val Leu Leu Ser Leu Tyr Val Ser Ala Ser Leu Ser Tyr Leu Leu
               20                  25                  30
   Tyr Ser Asp Ile Leu Leu Lys Phe Ser Gln Thr Glu Ile Thr Ala Pro
           35                  40                  45
   Ile Met Pro Leu Asp Cys Ala Asn Ala Ser Asn Val Gln Ala Val Asn
       50                  55                  60
   Arg Ser Ala Ala Lys Gly Val Thr Leu Leu Leu Pro Glu Pro Glu Trp
   65                  70                  75                  80
   Thr Tyr Pro Arg Leu Ser Cys Pro Gly Ser Thr Phe Gln Lys Ala Leu
                   85                  90                  95
   Leu Ile Ser Pro His Arg Phe Gly Glu Thr Lys Gly Asn Ser Ala Pro
               100                 105                 110
   Leu Ile Ile Arg Glu Pro Phe Ile Ala Cys Gly Pro Thr Glu Cys Lys
           115                 120                 125
   His Phe Ala Leu Thr His Tyr Ala Ala Gln Pro Gly Gly Tyr Tyr Asn
       130                 135                 140
   Gly Thr Arg Glu Asp Arg Asn Lys Leu Arg His Leu Ile Ser Val Lys
   145                 150                 155                 160
   Leu Gly Lys Ile Pro Thr Val Glu Asn Ser Ile Phe His Met Ala Ala
                   165                 170                 175
   Trp Ser Gly Ser Ala Cys His Asp Gly Lys Glu Trp Thr Tyr Ile Gly
               180                 185                 190
   Val Asp Gly Pro Asp Ser Asn Ala Leu Leu Lys Ile Lys Tyr Gly Glu
           195                 200                 205
   Ala Tyr Thr Asp Thr Tyr His Ser Tyr Ala Lys Asn Ile Leu Arg Thr
       210                 215                 220
   Gln Glu Ser Ala Cys Asn Cys Ile Gly Gly Asp Cys Tyr Leu Met Ile
   225                 230                 235                 240
   Thr Asp Gly Pro Ala Ser Gly Ile Ser Glu Cys Arg Phe Leu Lys Ile
                   245                 250                 255
   Arg Glu Gly Arg Ile Ile Lys Glu Ile Phe Pro Thr Gly Arg Val Lys
               260                 265                 270
   His Thr Glu Glu Cys Thr Cys Gly Phe Ala Ser Asn Lys Thr Ile Glu
           275                 280                 285
   Cys Ala Cys Arg Asp Asn Ser Tyr Thr Ala Lys Arg Pro Phe Val Lys
       290                 295                 300
   Leu Asn Val Glu Thr Asp Thr Ala Glu Ile Arg Leu Met Cys Thr Glu
   305                 310                 315                 320
   Thr Tyr Leu Asp Thr Pro Arg Pro Asn Asp Gly Ser Ile Thr Gly Pro
                   325                 330                 335
   Cys Glu Ser Asp Gly Asp Lys Gly Ser Gly Gly Ile Lys Gly Gly Phe
               340                 345                 350
   Val His Gln Arg Met Ala Ser Lys Ile Gly Arg Trp Tyr Ser Arg Thr
           355                 360                 365
   Met Ser Lys Thr Lys Arg Met Gly Met Gly Leu Tyr Val Lys Tyr Asp
       370                 375                 380
   Gly Asp Pro Trp Thr Asp Ser Glu Ala Leu Ala Leu Ser Gly Val Met
   385                 390                 395                 400
   Val Ser Met Glu Glu Pro Gly Trp Tyr Ser Phe Gly Phe Glu Ile Lys
                   405                 410                 415
   Asp Lys Lys Cys Asp Val Pro Cys Ile Gly Ile Glu Met Val His Asp
               420                 425                 430
   Gly Gly Lys Thr Thr Trp His Ser Ala Ala Thr Ala Ile Tyr Cys Leu
           435                 440                 445
   Met Gly Ser Gly Gln Leu Leu Trp Asp Thr Val Thr Gly Val Asp Met
       450                 455                 460
   Ala Leu
   465
<210> 37
   <211> 466
   <212> PRT
   <213> Influenza B
<220>
   <223> Neuraminidase, Malaysia/2506/2004, NAM
<400> 37
   Met Leu Pro Ser Thr Ile Gln Thr Leu Thr Leu Phe Leu Thr Ser Gly
   1               5                   10                  15
   Gly Val Leu Leu Ser Leu Tyr Val Ser Ala Ser Leu Ser Tyr Leu Leu
               20                  25                  30
   Tyr Ser Asp Ile Leu Leu Lys Phe Pro Ser Thr Glu Ile Thr Ala Pro
           35                  40                  45
   Thr Met Pro Leu Asp Cys Ala Asn Ala Ser Asn Val Gln Ala Val Asn
       50                  55                  60
   Arg Ser Ala Thr Lys Gly Val Thr Leu Leu Leu Pro Glu Pro Glu Trp
   65                  70                  75                  80
   Thr Tyr Pro Arg Leu Ser Cys Pro Gly Ser Thr Phe Gln Lys Ala Leu
                   85                  90                  95
   Leu Ile Ser Pro His Arg Phe Gly Glu Thr Lys Gly Asn Ser Ala Pro
               100                 105                 110
   Leu Ile Ile Arg Glu Pro Phe Ile Ala Cys Gly Pro Lys Glu Cys Lys
           115                 120                 125
   His Phe Ala Leu Thr His Tyr Ala Ala Gln Pro Gly Gly Tyr Tyr Asn
       130                 135                 140
   Gly Thr Arg Gly Asp Arg Asn Lys Leu Arg His Leu Ile Ser Val Lys
   145                 150                 155                 160
   Leu Gly Lys Ile Pro Thr Val Glu Asn Ser Ile Phe His Met Ala Ala
                   165                 170                 175
   Trp Ser Gly Ser Ala Cys His Asp Gly Lys Glu Trp Thr Tyr Ile Gly
               180                 185                 190
   Val Asp Gly Pro Asp Asn Asn Ala Leu Leu Lys Ile Lys Tyr Gly Glu
           195                 200                 205
   Ala Tyr Thr Asp Thr Tyr His Ser Tyr Ala Asn Asn Ile Leu Arg Thr
       210                 215                 220
   Gln Glu Ser Ala Cys Asn Cys Ile Gly Gly Asn Cys Tyr Leu Met Ile
   225                 230                 235                 240
   Thr Asp Gly Ser Ala Ser Gly Val Ser Glu Cys Arg Phe Leu Lys Ile
                   245                 250                 255
   Arg Glu Gly Arg Ile Ile Lys Glu Ile Phe Pro Thr Gly Arg Ile Lys
               260                 265                 270
   His Thr Glu Glu Cys Thr Cys Gly Phe Ala Ser Asn Lys Thr Ile Glu
           275                 280                 285
   Cys Ala Cys Arg Asp Asn Ser Tyr Thr Ala Lys Arg Pro Phe Val Lys
       290                 295                 300
   Leu Asn Val Glu Thr Asp Thr Ala Glu Ile Arg Leu Met Cys Thr Glu
   305                 310                 315                 320
   Thr Tyr Leu Asp Thr Pro Arg Pro Asp Asp Gly Ser Ile Thr Gly Pro
   325 330 335
   Cys Glu Ser Asn Gly Asp Lys Gly Ser Gly Gly Ile Lys Gly Gly Phe
   340 345 350
   Val His Gln Arg Met Ala Ser Lys Ile Gly Arg Trp Tyr Ser Arg Thr
   355 360 365
   Met Ser Lys Thr Lys Arg Met Gly Met Gly Leu Tyr Val Lys Tyr Asp
   370 375 380
   Gly Asp Pro Trp Ala Asp Ser Asp Ala Leu Ala Phe Ser Gly Val Met
   385 390 395 400
   Val Ser Met Glu Glu Pro Gly Trp Tyr Ser Phe Gly Phe Glu Ile Lys
   405 410 415
   Asp Lys Lys Cys Asp Val Pro Cys Ile Gly Ile Glu Met Val His Asp
   420 425 430
   Gly Gly Lys Glu Thr Trp His Ser Ala Ala Thr Ala Ile Tyr Cys Leu
   435 440 445
   Met Gly Ser Gly Gln Leu Leu Trp Asp Thr Val Thr Gly Val Asn Met
   450 455 460
   Ala Leu
   465
<210> 38
   <211> 470
   <212> PRT
   <213> Influenza A
<220>
   <223> Neuraminidase, New Caledonia/20/99/H1N1, NANC
<300>
   <308> ABQ10080
<400> 38
   Met Asn Pro Asn Gln Lys Ile Ile Thr Ile Gly Ser Ile Ser Ile Ala
   1               5                   10                  15
   Ile Gly Ile Ile Ser Leu Met Leu Gln Ile Gly Asn Ile Ile Ser Ile
               20                  25                  30
   Trp Ala Ser His Ser Ile Gln Thr Gly Ser Gln Asn His Thr Gly Val
           35                  40                  45
   Cys Asn Gln Arg Ile Ile Thr Tyr Glu Asn Ser Thr Trp Val Asn His
       50                  55                  60
   Thr Tyr Val Asn Ile Asn Asn Thr Asn Val Val Ala Gly Lys Asp Lys
   65                  70                  75                  80
   Thr Ser Val Thr Leu Ala Gly Asn Ser Ser Leu Cys Ser Ile Ser Gly
                   85                  90                  95
   Trp Ala Ile Tyr Thr Lys Asp Asn Ser Ile Arg Ile Gly Ser Lys Gly
               100                 105                 110
   Asp Val Phe Val Ile Arg Glu Pro Phe Ile Ser Cys Ser His Leu Glu
           115                 120                 125
   Cys Arg Thr Phe Phe Leu Thr Gln Gly Ala Leu Leu Asn Asp Lys His
       130                 135                 140
   Ser Asn Gly Thr Val Lys Asp Arg Ser Pro Tyr Arg Ala Leu Met Ser
   145                 150                 155                 160
   Cys Pro Leu Gly Glu Ala Pro Ser Pro Tyr Asn Ser Lys Phe Glu Ser
                   165                 170                 175
   Val Ala Trp Ser Ala Ser Ala Cys His Asp Gly Met Gly Trp Leu Thr
               180                 185                 190
   Ile Gly Ile Ser Gly Pro Asp Asn Gly Ala Val Ala Val Leu Lys Tyr
           195                 200                 205
   Asn Gly Ile Ile Thr Glu Thr Ile Lys Ser Trp Lys Lys Arg Ile Leu
       210                 215                 220
   Arg Thr Gln Glu Ser Glu Cys Val Cys Val Asn Gly Ser Cys Phe Thr
   225                 230                 235                 240
   Ile Met Thr Asp Gly Pro Ser Asn Gly Ala Ala Ser Tyr Lys Ile Phe
                   245                 250                 255
   Lys Ile Glu Lys Gly Lys Val Thr Lys Ser Ile Glu Leu Asn Ala Pro
               260                 265                 270
   Asn Phe His Tyr Glu Glu Cys Ser Cys Tyr Pro Asp Thr Gly Thr Val
           275                 280                 285
   Met Cys Val Cys Arg Asp Asn Trp His Gly Ser Asn Arg Pro Trp Val
       290                 295                 300
   Ser Phe Asn Gln Asn Leu Asp Tyr Gln Ile Gly Tyr Ile Cys Ser Gly
   305 310 315 320
   Val Phe Gly Asp Asn Pro Arg Pro Lys Asp Gly Glu Gly Ser Cys Asn
   325 330 335
   Pro Val Thr Val Asp Gly Ala Asp Gly Val Lys Gly Phe Ser Tyr Lys
   340 345 350
   Tyr Gly Asn Gly Val Trp Ile Gly Arg Thr Lys Ser Asn Arg Leu Arg
   355 360 365
   Lys Gly Phe Glu Met Ile Trp Asp Pro Asn Gly Trp Thr Asp Thr Asp
   370 375 380
   Ser Asp Phe Ser Val Lys Gln Asp Val Val Ala Ile Thr Asp Trp Ser
   385 390 395 400
   Gly Tyr Ser Gly Ser Phe Val Gln His Pro Glu Leu Thr Gly Leu Asp
   405 410 415
   Cys Ile Arg Pro Cys Phe Trp Val Glu Leu Val Arg Gly Leu Pro Arg
   420 425 430
   Glu Asn Thr Thr Ile Trp Thr Ser Gly Ser Ser Ile Ser Phe Cys Gly
   435 440 445
   Val Asn Ser Asp Thr Ala Asn Trp Ser Trp Pro Asp Gly Ala Glu Leu
   450 455 460
   Pro Phe Thr Ile Asp Lys
   465 470
<210> 39
   <211> 457
   <212> PRT
   <213> Influenza A
<220>
   <223> Neuraminidase, Solomon Islands/3/2006/H1N1, NASI
<300>
   <308> ABU99068
<400> 39
   Met Asn Pro Asn Gln Lys Ile Ile Thr Ile Gly Ser Ile Ser Ile Ala
   1               5                   10                  15
   Ile Gly Ile Ile Ser Leu Ile Leu Gln Ile Gly Asn Ile Ile Ser Ile
               20                  25                  30
   Trp Ala Ser His Ser Ile Gln Thr Gly Ser Gln Asn His Thr Gly Ile
           35                  40                  45
   Cys Asn Gln Arg Ile Ile Thr Tyr Glu Asn Ser Thr Trp Val Asn Asn
       50                  55                  60
   Thr Tyr Val Asn Ile Asn Asn Thr Asn Val Val Ala Glu Lys Asp Lys
   65                  70                  75                  80
   Thr Ser Val Thr Leu Ala Gly Asn Ser Ser Leu Cys Ser Ile Ser Gly
                   85                  90                  95
   Trp Ala Ile Tyr Thr Lys Asp Asn Ser Ile Arg Ile Gly Ser Lys Gly
               100                 105                 110
   Asp Val Phe Val Ile Arg Glu Pro Phe Ile Ser Cys Ser His Leu Glu
           115                 120                 125
   Cys Arg Thr Phe Phe Leu Thr Gln Gly Ala Leu Leu Asn Asp Lys His
       130                 135                 140
   Ser Asn Gly Thr Val Lys Asp Arg Ser Pro Tyr Arg Thr Leu Met Ser
   145                 150                 155                 160
   Cys Pro Leu Gly Glu Ala Pro Ser Pro Tyr Asn Ser Arg Phe Glu Ser
                   165                 170                 175
   Val Ala Trp Ser Ala Ser Ala Cys His Asp Gly Met Gly Trp Leu Thr
               180                 185                 190
   Ile Gly Ile Ser Gly Pro Asp Asn Gly Ala Val Ala Val Leu Lys Tyr
           195                 200                 205
   Asn Gly Ile Ile Thr Glu Thr Ile Lys Ser Trp Lys Lys Arg Ile Leu
       210                 215                 220
   Arg Thr Gln Glu Ser Glu Cys Val Cys Met Asn Gly Ser Cys Phe Thr
   225                 230                 235                 240
   Ile Met Thr Asp Gly Pro Ser Asn Gly Ala Ala Ser Tyr Lys Ile Phe
                   245                 250                 255
   Lys Ile Glu Lys Gly Lys Val Thr Lys Thr Ile Glu Leu Asn Ala Pro
               260                 265                 270
   Asn Phe His Tyr Glu Glu Cys Ser Cys Tyr Pro Asp Thr Gly Thr Val
           275                 280                 285
   Met Cys Val Cys Arg Asp Asn Trp His Gly Ser Asn Arg Pro Trp Val
   290 295 300
   Ser Phe Asn Gln Asn Leu Asp Tyr Gln Ile Gly Tyr Ile Cys Ser Gly
   305 310 315 320
   Val Phe Gly Asp Asn Pro Arg Pro Lys Asp Gly Glu Gly Ser Cys Asn
   325 330 335
   Pro Val Thr Val Asp Gly Ala Asp Gly Val Lys Gly Phe Ser Tyr Lys
   340 345 350
   Tyr Gly Asn Gly Val Trp Ile Gly Arg Thr Lys Ser Asn Arg Leu Arg
   355 360 365
   Lys Gly Phe Glu Met Ile Trp Asp Pro Asn Gly Trp Thr Asn Thr Asp
   370 375 380
   Ser Asp Phe Ser Val Lys Gln Asp Val Val Ala Ile Thr Asp Trp Ser
   385 390 395 400
   Gly Tyr Ser Gly Ser Phe Val Gln His Pro Glu Leu Thr Gly Leu Asp
   405 410 415
   Cys Ile Arg Pro Cys Phe Trp Val Glu Leu Val Arg Gly Leu Pro Arg
   420 425 430
   Glu Asn Thr Thr Ile Trp Thr Ser Gly Ser Ser Ile Ser Phe Cys Gly
   435 440 445
   Val Asn Ser Gly Thr Ala Asn Trp Ser
   450 455
<210> 40
   <211> 469
   <212> PRT
   <213> Influenza A
<220>
   <223> Neuraminidase, Wisconsin/67/2005/H3N2, NAWI
<400> 40
   Met Asn Pro Asn Gln Lys Ile Ile Thr Ile Gly Ser Val Ser Leu Thr
   1               5                   10                  15
   Ile Ser Thr Ile Cys Phe Phe Met Gln Ile Ala Ile Leu Ile Thr Thr
               20                  25                  30
   Val Thr Leu His Phe Lys Gln Tyr Glu Phe Asn Ser Pro Pro Asn Asn
           35                  40                  45
   Gln Val Met Leu Cys Glu Pro Thr Ile Ile Glu Arg Asn Ile Thr Glu
       50                  55                  60
   Ile Val Tyr Leu Thr Asn Thr Thr Ile Glu Lys Glu Ile Cys Pro Lys
   65                  70                  75                  80
   Leu Ala Glu Tyr Arg Asn Trp Ser Lys Pro Gln Cys Asn Ile Thr Gly
                   85                  90                  95
   Phe Ala Pro Phe Ser Lys Asp Asn Ser Ile Arg Leu Ser Ala Gly Gly
               100                 105                 110
   Asp Ile Trp Val Thr Arg Glu Pro Tyr Val Ser Cys Asp Pro Asp Lys
           115                 120                 125
   Cys Tyr Gln Phe Ala Leu Gly Gln Gly Thr Thr Leu Asn Asn Val His
       130                 135                 140
   Ser Asn Asp Thr Val His Asp Arg Thr Pro Tyr Arg Thr Leu Leu Met
   145                 150                 155                 160
   Asn Glu Leu Gly Val Pro Phe His Leu Gly Thr Lys Gln Val Cys Ile
                   165                 170                 175
   Ala Trp Ser Ser Ser Ser Cys His Asp Gly Lys Ala Trp Leu His Val
               180                 185                 190
   Cys Val Thr Gly Asp Asp Lys Asn Ala Thr Ala Ser Phe Ile Tyr Asn
           195                 200                 205
   Gly Arg Leu Val Asp Ser Ile Val Ser Trp Ser Lys Glu Ile Leu Arg
       210                 215                 220
   Thr Gln Glu Ser Glu Cys Val Cys Ile Asn Gly Thr Cys Thr Val Val
   225                 230                 235                 240
   Met Thr Asp Gly Ser Ala Ser Gly Lys Ala Asp Thr Lys Ile Leu Phe
                   245                 250                 255
   Ile Glu Glu Gly Lys Ile Val His Thr Ser Thr Leu Ser Gly Ser Ala
               260                 265                 270
   Gln His Val Glu Glu Cys Ser Cys Tyr Pro Arg Tyr Leu Gly Val Arg
           275                 280                 285
   Cys Val Cys Arg Asp Asn Trp Lys Gly Ser Asn Arg Pro Ile Val Asp
       290                 295                 300
   Ile Asn Ile Lys Asp Tyr Ser Ile Val Ser Ser Tyr Val Cys Ser Gly
   305                 310                 315                 320
   Leu Val Gly Asp Thr Pro Arg Lys Asn Asp Ser Ser Ser Ser Ser His
                   325                 330                 335
   Cys Leu Asp Pro Asn Asn Glu Glu Gly Gly His Gly Val Lys Gly Trp
               340                 345                 350
   Ala Phe Asp Asp Gly Asn Asp Val Trp Met Gly Arg Thr Ile Ser Glu
           355                 360                 365
   Lys Leu Arg Ser Gly Tyr Glu Thr Phe Lys Val Ile Glu Gly Trp Ser
       370                 375                 380
   Asn Pro Asn Ser Lys Leu Gln Ile Asn Arg Gln Val Ile Val Asp Arg
   385                 390                 395                 400
   Gly Asn Arg Ser Gly Tyr Ser Gly Ile Phe Ser Val Glu Gly Lys Ser
                   405                 410                 415
   Cys Ile Asn Arg Cys Phe Tyr Val Glu Leu Ile Arg Gly Arg Lys Glu
               420                 425                 430
   Glu Thr Glu Val Leu Trp Thr Ser Asn Ser Ile Val Val Phe Cys Gly
           435                 440                 445
   Thr Ser Gly Thr Tyr Gly Thr Gly Ser Trp Pro Asp Gly Ala Asp Ile
       450                 455                 460
   Asn Leu Met Pro Ile
   465
<210> 41
   <211> 469
   <212> PRT
   <213> Influenza A
<220>
   <223> Neuraminidase, Wyoming/3/03/H3N2, NAWY
<300>
   <308> AAT08001
<400> 41
   Met Asn Pro Asn Gln Lys Ile Ile Thr Ile Gly Ser Val Ser Leu Thr
   1               5                   10                  15
   Ile Ser Thr Ile Cys Phe Phe Met Gln Ile Ala Ile Leu Ile Thr Thr
               20                  25                  30
   Val Thr Leu His Phe Lys Gln Tyr Glu Phe Asn Ser Pro Pro Asn Asn
           35                  40                  45
   Gln Val Met Leu Cys Glu Pro Thr Ile Ile Glu Arg Asn Ile Thr Glu
       50                  55                  60
   Ile Val Tyr Leu Thr Asn Thr Thr Ile Glu Lys Glu Ile Cys Pro Lys
   65                  70                  75                  80
   Leu Ala Glu Tyr Arg Asn Trp Ser Lys Pro Gln Cys Asn Ile Thr Gly
                   85                  90                  95
   Phe Ala Pro Phe Ser Lys Asp Asn Ser Ile Arg Leu Ser Ala Gly Gly
               100                 105                 110
   Asp Ile Trp Val Thr Arg Glu Pro Tyr Val Ser Cys Asp Pro Asp Lys
           115                 120                 125
   Cys Tyr Gln Phe Ala Leu Gly Gln Gly Thr Thr Leu Asn Asn Val His
       130                 135                 140
   Ser Asn Asp Thr Val His Asp Arg Thr Pro Tyr Arg Thr Leu Leu Met
   145                 150                 155                 160
   Asn Glu Leu Gly Val Pro Phe His Leu Gly Thr Lys Gln Val Cys Ile
                   165                 170                 175
   Ala Trp Ser Ser Ser Ser Cys His Asp Gly Lys Ala Trp Leu His Val
               180                 185                 190
   Cys Val Thr Gly Asp Asp Glu Asn Ala Thr Ala Ser Phe Ile Tyr Asn
           195                 200                 205
   Gly Arg Leu Val Asp Ser Ile Val Ser Trp Ser Lys Lys Ile Leu Arg
       210                 215                 220
   Thr Gln Glu Ser Glu Cys Val Cys Ile Asn Gly Thr Cys Thr Val Val
   225                 230                 235                 240
   Met Thr Asp Gly Ser Ala Ser Gly Lys Ala Asp Thr Lys Ile Leu Phe
                   245                 250                 255
   Ile Glu Glu Gly Lys Ile Val His Thr Ser Thr Leu Ser Gly Ser Ala
               260                 265                 270
   Gln His Val Glu Glu Cys Ser Cys Tyr Pro Arg Tyr Pro Gly Val Arg
           275                 280                 285
   Cys Val Cys Arg Asp Asn Trp Lys Gly Ser Asn Arg Pro Ile Val Asp
       290                 295                 300
   Ile Asn Ile Lys Asp Tyr Ser Ile Val Ser Ser Tyr Val Cys Ser Gly
   305 310 315 320
   Leu Val Gly Asp Thr Pro Arg Lys Asn Asp Ser Ser Ser Ser Ser His
   325 330 335
   Cys Leu Asp Pro Asn Asn Glu Glu Gly Gly His Gly Val Lys Gly Trp
   340 345 350
   Ala Phe Asp Asp Gly Asn Asp Val Trp Met Gly Arg Thr Ile Ser Glu
   355 360 365
   Lys Leu Arg Ser Gly Tyr Glu Thr Phe Lys Val Ile Glu Gly Trp Ser
   370 375 380
   Asn Pro Asn Ser Lys Leu Gln Ile Asn Arg Gln Val Ile Val Asp Arg
   385 390 395 400
   Gly Asn Arg Ser Gly Tyr Ser Gly Ile Phe Ser Val Glu Gly Lys Ser
   405 410 415
   Cys Ile Asn Arg Cys Phe Tyr Val Glu Leu Ile Arg Gly Arg Lys Gln
   420 425 430
   Glu Thr Glu Val Leu Trp Thr Ser Asn Ser Ile Val Val Phe Cys Gly
   435 440 445
   Thr Ser Gly Thr Tyr Gly Thr Gly Ser Trp Pro Asp Gly Ala Asp Ile
   450 455 460
   Asn Leu Met Pro Ile
   465
<210> 42
   <211> 565
   <212> PRT
   <213> Influenza A
<220>
   <223> Hemagglutinin, Brisbane/59/2007/H1N1, HAB(H1)
<300>
   <308> ACA28844
<400> 42
   Met Lys Val Lys Leu Leu Val Leu Leu Cys Thr Phe Thr Ala Thr Tyr
   1               5                   10                  15
   Ala Asp Thr Ile Cys Ile Gly Tyr His Ala Asn Asn Ser Thr Asp Thr
               20                  25                  30
   Val Asp Thr Val Leu Glu Lys Asn Val Thr Val Thr His Ser Val Asn
           35                  40                  45
   Leu Leu Glu Asn Ser His Asn Gly Lys Leu Cys Leu Leu Lys Gly Ile
       50                  55                  60
   Ala Pro Leu Gln Leu Gly Asn Cys Ser Val Ala Gly Trp Ile Leu Gly
   65                  70                  75                  80
   Asn Pro Glu Cys Glu Leu Leu Ile Ser Lys Glu Ser Trp Ser Tyr Ile
                   85                  90                  95
   Val Glu Lys Pro Asn Pro Glu Asn Gly Thr Cys Tyr Pro Gly His Phe
               100                 105                 110
   Ala Asp Tyr Glu Glu Leu Arg Glu Gln Leu Ser Ser Val Ser Ser Phe
           115                 120                 125
   Glu Arg Phe Glu Ile Phe Pro Lys Glu Ser Ser Trp Pro Asn His Thr
       130                 135                 140
   Val Thr Gly Val Ser Ala Ser Cys Ser His Asn Gly Glu Ser Ser Phe
   145                 150                 155                 160
   Tyr Arg Asn Leu Leu Trp Leu Thr Gly Lys Asn Gly Leu Tyr Pro Asn
                   165                 170                 175
   Leu Ser Lys Ser Tyr Ala Asn Asn Lys Glu Lys Glu Val Leu Val Leu
               180                 185                 190
   Trp Gly Val His His Pro Pro Asn Ile Gly Asp Gln Lys Ala Leu Tyr
           195                 200                 205
   His Thr Glu Asn Ala Tyr Val Ser Val Val Ser Ser His Tyr Ser Arg
       210                 215                 220
   Lys Phe Thr Pro Glu Ile Ala Lys Arg Pro Lys Val Arg Asp Gln Glu
   225                 230                 235                 240
   Gly Arg Ile Asn Tyr Tyr Trp Thr Leu Leu Glu Pro Gly Asp Thr Ile
                   245                 250                 255
   Ile Phe Glu Ala Asn Gly Asn Leu Ile Ala Pro Arg Tyr Ala Phe Ala
               260                 265                 270
   Leu Ser Arg Gly Phe Gly Ser Gly Ile Ile Asn Ser Asn Ala Pro Met
           275                 280                 285
   Asp Lys Cys Asp Ala Lys Cys Gln Thr Pro Gln Gly Ala Ile Asn Ser
       290                 295                 300
   Ser Leu Pro Phe Gln Asn Val His Pro Val Thr Ile Gly Glu Cys Pro
   305                 310                 315                 320
   Lys Tyr Val Arg Ser Ala Lys Leu Arg Met Val Thr Gly Leu Arg Asn
                   325                 330                 335
   Ile Pro Ser Ile Gln Ser Arg Gly Leu Phe Gly Ala Ile Ala Gly Phe
               340                 345                 350
   Ile Glu Gly Gly Trp Thr Gly Met Val Asp Gly Trp Tyr Gly Tyr His
           355                 360                 365
   His Gln Asn Glu Gln Gly Ser Gly Tyr Ala Ala Asp Gln Lys Ser Thr
       370                 375                 380
   Gln Asn Ala Ile Asn Gly Ile Thr Asn Lys Val Asn Ser Val Ile Glu
   385                 390                 395                 400
   Lys Met Asn Thr Gln Phe Thr Ala Val Gly Lys Glu Phe Asn Lys Leu
                   405                 410                 415
   Glu Arg Arg Met Glu Asn Leu Asn Lys Lys Val Asp Asp Gly Phe Ile
               420                 425                 430
   Asp Ile Trp Thr Tyr Asn Ala Glu Leu Leu Val Leu Leu Glu Asn Glu
           435                 440                 445
   Arg Thr Leu Asp Phe His Asp Ser Asn Val Lys Asn Leu Tyr Glu Lys
       450                 455                 460
   Val Lys Ser Gln Leu Lys Asn Asn Ala Lys Glu Ile Gly Asn Gly Cys
   465                 470                 475                 480
   Phe Glu Phe Tyr His Lys Cys Asn Asp Glu Cys Met Glu Ser Val Lys
                   485                 490                 495
   Asn Gly Thr Tyr Asp Tyr Pro Lys Tyr Ser Glu Glu Ser Lys Leu Asn
               500                 505                 510
   Arg Glu Lys Ile Asp Gly Val Lys Leu Glu Ser Met Gly Val Tyr Gln
           515                 520                 525
   Ile Leu Ala Ile Tyr Ser Thr Val Ala Ser Ser Leu Val Leu Leu Val
       530                 535                 540
   Ser Leu Gly Ala Ile Ser Phe Trp Met Cys Ser Asn Gly Ser Leu Gln
   545 550 555 560
   Cys Arg Ile Cys Ile
   565
<210> 43
   <211> 584
   <212> PRT
   <213> Influenza B
<220>
   <223> Hemagglutinin, Florida/4/2006, HAF
<300>
   <308> aca33493
<400> 43
   Met Lys Ala Ile Ile Val Leu Leu Met Val Val Thr Ser Asn Ala Asp
   1               5                   10                  15
   Arg Ile Cys Thr Gly Ile Thr Ser Ser Asn Ser Pro His Val Val Lys
               20                  25                  30
   Thr Ala Thr Gln Gly Glu Val Asn Val Thr Gly Val Ile Pro Leu Thr
           35                  40                  45
   Thr Thr Pro Thr Lys Ser Tyr Phe Ala Asn Leu Lys Gly Thr Arg Thr
       50                  55                  60
   Arg Gly Lys Leu Cys Pro Asp Cys Leu Asn Cys Thr Asp Leu Asp Val
   65                  70                  75                  80
   Ala Leu Gly Arg Pro Met Cys Val Gly Thr Thr Pro Ser Ala Lys Ala
                   85                  90                  95
   Ser Ile Leu His Glu Val Lys Pro Val Thr Ser Gly Cys Phe Pro Ile
               100                 105                 110
   Met His Asp Arg Thr Lys Ile Arg Gln Leu Pro Asn Leu Leu Arg Gly
           115                 120                 125
   Tyr Glu Asn Ile Arg Leu Ser Thr Gln Asn Val Ile Asp Ala Glu Lys
       130                 135                 140
   Ala Pro Gly Gly Pro Tyr Arg Leu Gly Thr Ser Gly Ser Cys Pro Asn
   145                 150                 155                 160
   Ala Thr Ser Lys Ser Gly Phe Phe Ala Thr Met Ala Trp Ala Val Pro
                   165                 170                 175
   Lys Asp Asn Asn Lys Asn Ala Thr Asn Pro Leu Thr Val Glu Val Pro
   180 185 190
   Tyr Ile Cys Thr Glu Gly Glu Asp Gln Ile Thr Val Trp Gly Phe His
   195 200 205
   Ser Asp Asp Lys Thr Gln Met Lys Asn Leu Tyr Gly Asp Ser Asn Pro
   210 215 220
   Gln Lys Phe Thr Ser Ser Ala Asn Gly Val Thr Thr His Tyr Val Ser
   225 230 235 240
   Gln Ile Gly Ser Phe Pro Asp Gln Thr Glu Asp Gly Gly Leu Pro Gln
   245 250 255
   Ser Gly Arg Ile Val Val Asp Tyr Met Met Gln Lys Pro Gly Lys Thr
   260 265 270
   Gly Thr Ile Val Tyr Gln Arg Gly Val Leu Leu Pro Gln Lys Val Trp
   275 280 285
   Cys Ala Ser Gly Arg Ser Lys Val Ile Lys Gly Ser Leu Pro Leu Ile
   290 295 300
   Gly Glu Ala Asp Cys Leu His Glu Lys Tyr Gly Gly Leu Asn Lys Ser
   305 310 315 320
   Lys Pro Tyr Tyr Thr Gly Glu His Ala Lys Ala Ile Gly Asn Cys Pro
   325 330 335
   Ile Trp Val Lys Thr Pro Leu Lys Leu Ala Asn Gly Thr Lys Tyr Arg
   340 345 350
   Pro Pro Ala Lys Leu Leu Lys Glu Arg Gly Phe Phe Gly Ala Ile Ala
   355 360 365
   Gly Phe Leu Glu Gly Gly Trp Glu Gly Met Ile Ala Gly Trp His Gly
   370 375 380
   Tyr Thr Ser His Gly Ala His Gly Val Ala Val Ala Ala Asp Leu Lys
   385 390 395 400
   Ser Thr Gln Glu Ala Ile Asn Lys Ile Thr Lys Asn Leu Asn Ser Leu
   405 410 415
   Ser Glu Leu Glu Val Lys Asn Leu Gln Arg Leu Ser Gly Ala Met Asp
   420 425 430
   Glu Leu His Asn Glu Ile Leu Glu Leu Asp Glu Lys Val Asp Asp Leu
   435 440 445
   Arg Ala Asp Thr Ile Ser Ser Gln Ile Glu Leu Ala Val Leu Leu Ser
   450 455 460
   Asn Glu Gly Ile Ile Asn Ser Glu Asp Glu His Leu Leu Ala Leu Glu
   465 470 475 480
   Arg Lys Leu Lys Lys Met Leu Gly Pro Ser Ala Val Glu Ile Gly Asn
   485 490 495
   Gly Cys Phe Glu Thr Lys His Lys Cys Asn Gln Thr Cys Leu Asp Arg
   500 505 510
   Ile Ala Ala Gly Thr Phe Asn Ala Gly Glu Phe Ser Leu Pro Thr Phe
   515 520 525
   Asp Ser Leu Asn Ile Thr Ala Ala Ser Leu Asn Asp Asp Gly Leu Asp
   530 535 540
   Asn His Thr Ile Leu Leu Tyr Tyr Ser Thr Ala Ala Ser Ser Leu Ala
   545 550 555 560
   Val Thr Leu Met Leu Ala Ile Phe Ile Val Tyr Met Val Ser Arg Asp
   565 570 575
   Asn Val Ser Cys Ser Ile Cys Leu
   580
<210> 44
   <211> 586
   <212> PRT
   <213> Influenza B
<220>
   <223> Hemagglutinin, Malaysia/2506/2004, HAM, 2004-like sequence
<400> 44
   Met Lys Ala Ile Ile Val Leu Leu Met Val Val Thr Ser Asn Ala Asp
   1               5                   10                  15
   Arg Ile Ile Cys Thr Gly Ile Thr Ser Ser Asn Ser Pro His Val Val
               20                  25                  30
   Lys Thr Ala Thr Gln Gly Glu Val Asn Val Thr Gly Val Ile Pro Leu
           35                  40                  45
   Thr Thr Thr Pro Thr Lys Ser His Phe Ala Asn Leu Lys Gly Thr Glu
       50                  55                  60
   Thr Arg Gly Lys Leu Cys Pro Lys Cys Leu Asn Cys Thr Asp Leu Asp
   65                  70                  75                  80
   Val Ala Leu Gly Arg Pro Lys Cys Thr Gly Asn Ile Pro Ser Ala Arg
                   85                  90                  95
   Val Ser Ile Leu His Glu Val Arg Pro Val Thr Ser Gly Cys Phe Pro
               100                 105                 110
   Ile Met His Asp Arg Thr Lys Ile Arg Gln Leu Pro Asn Leu Leu Arg
           115                 120                 125
   Gly Tyr Glu His Ile Arg Leu Ser Thr His Asn Val Ile Asn Ala Glu
       130                 135                 140
   Asn Ala Pro Gly Gly Pro Tyr Lys Ile Gly Thr Ser Gly Ser Cys Pro
   145                 150                 155                 160
   Asn Val Thr Asn Gly Asn Gly Phe Phe Ala Thr Met Ala Trp Ala Val
                   165                 170                 175
   Pro Lys Asn Asp Asn Asn Lys Thr Ala Thr Asn Ser Leu Thr Ile Glu
               180                 185                 190
   Val Pro Tyr Ile Cys Thr Glu Gly Glu Asp Gln Ile Thr Val Trp Gly
           195                 200                 205
   Phe His Ser Asp Asn Glu Thr Gln Met Ala Lys Leu Tyr Gly Asp Ser
       210                 215                 220
   Lys Pro Gln Lys Phe Thr Ser Ser Ala Asn Gly Val Thr Thr His Tyr
   225                 230                 235                 240
   Val Ser Gln Ile Gly Gly Phe Pro Asn Gln Thr Glu Asp Gly Gly Leu
                   245                 250                 255
   Pro Gln Ser Gly Arg Ile Val Val Asp Tyr Met Val Gln Lys Ser Gly
               260                 265                 270
   Lys Thr Gly Thr Ile Thr Tyr Gln Arg Gly Ile Leu Leu Pro Gln Lys
           275                 280                 285
   Val Trp Cys Ala Ser Gly Arg Ser Lys Val Ile Lys Gly Ser Leu Pro
       290                 295                 300
   Leu Ile Gly Glu Ala Asp Cys Leu His Glu Lys Tyr Gly Gly Leu Asn
   305                 310                 315                 320
   Lys Ser Lys Pro Tyr Tyr Thr Gly Glu His Ala Lys Ala Ile Gly Asn
                   325                 330                 335
   Cys Pro Ile Trp Val Lys Thr Pro Leu Lys Leu Ala Asn Gly Thr Lys
               340                 345                 350
   Tyr Arg Pro Pro Ala Lys Leu Leu Lys Glu Arg Gly Phe Phe Gly Ala
           355                 360                 365
   Ile Ala Gly Phe Leu Glu Gly Gly Trp Glu Gly Met Ile Ala Gly Trp
       370                 375                 380
   His Gly Tyr Thr Ser His Gly Ala His Gly Val Ala Val Ala Ala Asp
   385                 390                 395                 400
   Leu Lys Ser Thr Gln Glu Ala Ile Asn Lys Ile Thr Lys Asn Leu Asn
                   405                 410                 415
   Ser Leu Ser Glu Leu Glu Val Lys Asn Leu Gln Arg Leu Ser Gly Ala
               420                 425                 430
   Met Asp Glu Leu His Asn Glu Ile Leu Glu Leu Asp Glu Lys Val Asp
           435                 440                 445
   Asp Leu Arg Ala Asp Thr Ile Ser Ser Gln Ile Glu Leu Ala Val Leu
       450                 455                 460
   Leu Ser Asn Glu Gly Ile Ile Asn Ser Glu Asp Glu His Leu Leu Ala
   465                 470                 475                 480
   Leu Glu Arg Lys Leu Lys Lys Met Leu Gly Pro Ser Ala Val Glu Ile
                   485                 490                 495
   Gly Asn Gly Cys Phe Glu Thr Lys His Lys Cys Asn Gln Thr Cys Leu
               500                 505                 510
   Asp Arg Ile Ala Ala Gly Thr Phe Asp Ala Gly Glu Phe Ser Leu Pro
           515                 520                 525
   Thr Phe Asp Ser Leu Asn Ile Thr Ala Ala Ser Leu Asn Asp Asp Gly
       530                 535                 540
   Leu Asp Asn His Thr Ile Leu Leu Tyr Tyr Ser Thr Ala Ala Ser Ser
   545                 550                 555                 560
   Leu Ala Val Thr Leu Met Ile Ala Ile Phe Val Val Tyr Met Val Ser
                   565                 570                 575
   Arg Asp Asn Val Ser Cys Ser Ile Cys Leu
   580 585
<210> 45
   <211> 565
   <212> PRT
   <213> Influenza A
<220>
   <223> Hemagglutinin, New Caledonia/20/99/H1N1, HANC
<300>
   <308> AAP34324
<400> 45
   Met Lys Ala Lys Leu Leu Val Leu Leu Cys Thr Phe Thr Ala Thr Tyr
   1               5                   10                  15
   Ala Asp Thr Ile Cys Ile Gly Tyr His Ala Asn Asn Ser Thr Asp Thr
               20                  25                  30
   Val Asp Thr Val Leu Glu Lys Asn Val Thr Val Thr His Ser Val Asn
           35                  40                  45
   Leu Leu Glu Asp Ser His Asn Gly Lys Leu Cys Leu Leu Lys Gly Ile
       50                  55                  60
   Ala Pro Leu Gln Leu Gly Asn Cys Ser Val Ala Gly Trp Ile Leu Gly
   65                  70                  75                  80
   Asn Pro Glu Cys Glu Leu Leu Ile Ser Lys Glu Ser Trp Ser Tyr Ile
                   85                  90                  95
   Val Glu Thr Pro Asn Pro Glu Asn Gly Thr Cys Tyr Pro Gly Tyr Phe
               100                 105                 110
   Ala Asp Tyr Glu Glu Leu Arg Glu Gln Leu Ser Ser Val Ser Ser Phe
           115                 120                 125
   Glu Arg Phe Glu Ile Phe Pro Lys Glu Ser Ser Trp Pro Asn His Thr
       130                 135                 140
   Val Thr Gly Val Ser Ala Ser Cys Ser His Asn Gly Lys Ser Ser Phe
   145                 150                 155                 160
   Tyr Arg Asn Leu Leu Trp Leu Thr Gly Lys Asn Gly Leu Tyr Pro Asn
                   165                 170                 175
   Leu Ser Lys Ser Tyr Val Asn Asn Lys Glu Lys Glu Val Leu Val Leu
               180                 185                 190
   Trp Gly Val His His Pro Pro Asn Ile Gly Asn Gln Arg Ala Leu Tyr
           195                 200                 205
   His Thr Glu Asn Ala Tyr Val Ser Val Val Ser Ser His Tyr Ser Arg
       210                 215                 220
   Arg Phe Thr Pro Glu Ile Ala Lys Arg Pro Lys Val Arg Asp Gln Glu
   225                 230                 235                 240
   Gly Arg Ile Asn Tyr Tyr Trp Thr Leu Leu Glu Pro Gly Asp Thr Ile
                   245                 250                 255
   Ile Phe Glu Ala Asn Gly Asn Leu Ile Ala Pro Trp Tyr Ala Phe Ala
               260                 265                 270
   Leu Ser Arg Gly Phe Gly Ser Gly Ile Ile Thr Ser Asn Ala Pro Met
           275                 280                 285
   Asp Glu Cys Asp Ala Lys Cys Gln Thr Pro Gln Gly Ala Ile Asn Ser
       290                 295                 300
   Ser Leu Pro Phe Gln Asn Val His Pro Val Thr Ile Gly Glu Cys Pro
   305                 310                 315                 320
   Lys Tyr Val Arg Ser Ala Lys Leu Arg Met Val Thr Gly Leu Arg Asn
                   325                 330                 335
   Ile Pro Ser Ile Gln Ser Arg Gly Leu Phe Gly Ala Ile Ala Gly Phe
               340                 345                 350
   Ile Glu Gly Gly Trp Thr Gly Met Val Asp Gly Trp Tyr Gly Tyr His
           355                 360                 365
   His Gln Asn Glu Gln Gly Ser Gly Tyr Ala Ala Asp Gln Lys Ser Thr
       370                 375                 380
   Gln Asn Ala Ile Asn Gly Ile Thr Asn Lys Val Asn Ser Val Ile Glu
   385                 390                 395                 400
   Lys Met Asn Thr Gln Phe Thr Ala Val Gly Lys Glu Phe Asn Lys Leu
                   405                 410                 415
   Glu Arg Arg Met Glu Asn Leu Asn Lys Lys Val Asp Asp Gly Phe Leu
               420                 425                 430
   Asp Ile Trp Thr Tyr Asn Ala Glu Leu Leu Val Leu Leu Glu Asn Glu
           435                 440                 445
   Arg Thr Leu Asp Phe His Asp Ser Asn Val Lys Asn Leu Tyr Glu Lys
       450                 455                 460
   Val Lys Ser Gln Leu Lys Asn Asn Ala Lys Glu Ile Gly Asn Gly Cys
   465 470 475 480
   Phe Glu Phe Tyr His Lys Cys Asn Asn Glu Cys Met Glu Ser Val Lys
   485 490 495
   Asn Gly Thr Tyr Asp Tyr Pro Lys Tyr Ser Glu Glu Ser Lys Leu Asn
   500 505 510
   Arg Glu Lys Ile Asp Gly Val Lys Leu Glu Ser Met Gly Val Tyr Gln
   515 520 525
   Ile Leu Ala Ile Tyr Ser Thr Val Ala Ser Ser Leu Val Leu Leu Val
   530 535 540
   Ser Leu Gly Ala Ile Ser Phe Trp Met Cys Ser Asn Gly Ser Leu Gln
   545 550 555 560
   Cys Arg Ile Cys Ile
   565
<210> 46
   <211> 565
   <212> PRT
   <213> Influenza A
<220>
   <223> Hemagglutinin, Solomon Islands/3/2006/H1N1, HASI
<300>
   <308> ABU99069
<400> 46
   Met Lys Val Lys Leu Leu Val Leu Leu Cys Thr Phe Thr Ala Thr Tyr
   1               5                   10                  15
   Ala Asp Thr Ile Cys Ile Gly Tyr His Ala Asn Asn Ser Thr Asp Thr
               20                  25                  30
   Val Asp Thr Val Leu Glu Lys Asn Val Thr Val Thr His Ser Val Asn
           35                  40                  45
   Leu Leu Glu Asp Ser His Asn Gly Lys Leu Cys Leu Leu Lys Gly Ile
       50                  55                  60
   Ala Pro Leu Gln Leu Gly Asn Cys Ser Val Ala Gly Trp Ile Leu Gly
   65                  70                  75                  80
   Asn Pro Glu Cys Glu Leu Leu Ile Ser Arg Glu Ser Trp Ser Tyr Ile
                   85                  90                  95
   Val Glu Lys Pro Asn Pro Glu Asn Gly Thr Cys Tyr Pro Gly His Phe
               100                 105                 110
   Ala Asp Tyr Glu Glu Leu Arg Glu Gln Leu Ser Ser Val Ser Ser Phe
           115                 120                 125
   Glu Arg Phe Glu Ile Phe Pro Lys Glu Ser Ser Trp Pro Asn His Thr
       130                 135                 140
   Thr Thr Gly Val Ser Ala Ser Cys Ser His Asn Gly Glu Ser Ser Phe
   145                 150                 155                 160
   Tyr Lys Asn Leu Leu Trp Leu Thr Gly Lys Asn Gly Leu Tyr Pro Asn
                   165                 170                 175
   Leu Ser Lys Ser Tyr Ala Asn Asn Lys Glu Lys Glu Val Leu Val Leu
               180                 185                 190
   Trp Gly Val His His Pro Pro Asn Ile Gly Asp Gln Arg Ala Leu Tyr
           195                 200                 205
   His Lys Glu Asn Ala Tyr Val Ser Val Val Ser Ser His Tyr Ser Arg
       210                 215                 220
   Lys Phe Thr Pro Glu Ile Ala Lys Arg Pro Lys Val Arg Asp Gln Glu
   225                 230                 235                 240
   Gly Arg Ile Asn Tyr Tyr Trp Thr Leu Leu Glu Pro Gly Asp Thr Ile
                   245                 250                 255
   Ile Phe Glu Ala Asn Gly Asn Leu Ile Ala Pro Arg Tyr Ala Phe Ala
               260                 265                 270
   Leu Ser Arg Gly Phe Gly Ser Gly Ile Ile Asn Ser Asn Ala Pro Met
           275                 280                 285
   Asp Glu Cys Asp Ala Lys Cys Gln Thr Pro Gln Gly Ala Ile Asn Ser
       290                 295                 300
   Ser Leu Pro Phe Gln Asn Val His Pro Val Thr Ile Gly Glu Cys Pro
   305                 310                 315                 320
   Lys Tyr Val Arg Ser Ala Lys Leu Arg Met Val Thr Gly Leu Arg Asn
                   325                 330                 335
   Ile Pro Ser Ile Gln Ser Arg Gly Leu Phe Gly Ala Ile Ala Gly Phe
               340                 345                 350
   Ile Glu Gly Gly Trp Thr Gly Met Val Asp Gly Trp Tyr Gly Tyr His
           355                 360                 365
   His Gln Asn Glu Gln Gly Ser Gly Tyr Ala Ala Asp Gln Lys Ser Thr
       370                 375                 380
   Gln Asn Ala Ile Asn Gly Ile Thr Asn Lys Val Asn Ser Val Ile Glu
   385                 390                 395                 400
   Lys Met Asn Thr Gln Phe Thr Ala Val Gly Lys Glu Phe Asn Lys Leu
                   405                 410                 415
   Glu Arg Arg Met Glu Asn Leu Asn Lys Lys Val Asp Asp Gly Phe Ile
               420                 425                 430
   Asp Ile Trp Thr Tyr Asn Ala Glu Leu Leu Val Leu Leu Glu Asn Glu
           435                 440                 445
   Arg Thr Leu Asp Phe His Asp Ser Asn Val Lys Asn Leu Tyr Glu Lys
       450                 455                 460
   Val Lys Ser Gln Leu Lys Asn Asn Ala Lys Glu Ile Gly Asn Gly Cys
   465                 470                 475                 480
   Phe Glu Phe Tyr His Lys Cys Asn Asp Glu Cys Met Glu Ser Val Lys
                   485                 490                 495
   Asn Gly Thr Tyr Asp Tyr Pro Lys Tyr Ser Glu Glu Ser Lys Leu Asn
               500                 505                 510
   Arg Glu Lys Ile Asp Gly Val Lys Leu Glu Ser Met Gly Val Tyr Gln
           515                 520                 525
   Ile Leu Ala Ile Tyr Ser Thr Val Ala Ser Ser Leu Val Leu Leu Val
       530                 535                 540
   Ser Leu Gly Ala Ile Ser Phe Trp Met Cys Ser Asn Gly Ser Leu Gln
   545                 550                 555                 560
   Cys Arg Ile Cys Ile
                   565
<210> 47
   <211> 566
   <212> PRT
   <213> Influenza A
<220>
   <223> Hemagglutinin, Wisconsin/67/2005/H3N2, HAWI
<400> 47
   Met Lys Thr Ile Ile Ala Leu Ser Tyr Ile Leu Cys Leu Val Phe Ala
   1               5                   10                  15
   Gln Lys Leu Pro Gly Asn Asp Asn Ser Thr Ala Thr Leu Cys Leu Gly
               20                  25                  30
   His His Ala Val Pro Asn Gly Thr Ile Val Lys Thr Ile Thr Asn Asp
           35                  40                  45
   Gln Ile Glu Val Thr Asn Ala Thr Glu Leu Val Gln Ser Ser Ser Thr
       50                  55                  60
   Gly Gly Ile Cys Asp Ser Pro His Gln Ile Leu Asp Gly Glu Asn Cys
   65                  70                  75                  80
   Thr Leu Ile Asp Ala Leu Leu Gly Asp Pro Gln Cys Asp Gly Phe Gln
                   85                  90                  95
   Asn Lys Lys Trp Asp Leu Phe Val Glu Arg Ser Lys Ala Tyr Ser Asn
               100                 105                 110
   Cys Tyr Pro Tyr Asp Val Pro Asp Tyr Ala Ser Leu Arg Ser Leu Val
           115                 120                 125
   Ala Ser Ser Gly Thr Leu Glu Phe Asn Asp Glu Ser Phe Asn Trp Thr
       130                 135                 140
   Gly Val Thr Gln Asn Gly Thr Ser Ser Ser Cys Lys Arg Arg Ser Asn
   145                 150                 155                 160
   Asn Ser Phe Phe Ser Arg Leu Asn Trp Leu Thr His Leu Lys Phe Lys
                   165                 170                 175
   Tyr Pro Ala Leu Asn Val Thr Met Pro Asn Asn Glu Lys Phe Asp Lys
               180                 185                 190
   Leu Tyr Ile Trp Gly Val His His Pro Val Thr Asp Asn Asp Gln Ile
           195                 200                 205
   Phe Leu Tyr Ala Gln Ala Ser Gly Arg Ile Thr Val Ser Thr Lys Arg
       210                 215                 220
   Ser Gln Gln Thr Val Ile Pro Asn Ile Gly Ser Arg Pro Arg Ile Arg
   225                 230                 235                 240
   Asn Ile Pro Ser Arg Ile Ser Ile Tyr Trp Thr Ile Val Lys Pro Gly
                   245                 250                 255
   Asp Ile Leu Leu Ile Asn Ser Thr Gly Asn Leu Ile Ala Pro Arg Gly
   260 265 270
   Tyr Phe Lys Ile Arg Ser Gly Lys Ser Ser Ile Met Arg Ser Asp Ala
   275 280 285
   Pro Ile Gly Lys Cys Asn Ser Glu Cys Ile Thr Pro Asn Gly Ser Ile
   290 295 300
   Pro Asn Asp Lys Pro Phe Gln Asn Val Asn Arg Ile Thr Tyr Gly Ala
   305 310 315 320
   Cys Pro Arg Tyr Val Lys Gln Asn Thr Leu Lys Leu Ala Thr Gly Met
   325 330 335
   Arg Asn Val Pro Glu Lys Gln Thr Arg Gly Ile Phe Gly Ala Ile Ala
   340 345 350
   Gly Phe Ile Glu Asn Gly Trp Glu Gly Met Val Asp Gly Trp Tyr Gly
   355 360 365
   Phe Arg His Gln Asn Ser Glu Gly Ile Gly Gln Ala Ala Asp Leu Lys
   370 375 380
   Ser Thr Gln Ala Ala Ile Asn Gln Ile Asn Gly Lys Leu Asn Arg Leu
   385 390 395 400
   Ile Gly Lys Thr Asn Glu Lys Phe His Gln Ile Glu Lys Glu Phe Ser
   405 410 415
   Glu Val Glu Gly Arg Ile Gln Asp Leu Glu Lys Tyr Val Glu Asp Thr
   420 425 430
   Lys Ile Asp Leu Trp Ser Tyr Asn Ala Glu Leu Leu Val Ala Leu Glu
   435 440 445
   Asn Gln His Thr Ile Asp Leu Thr Asp Ser Glu Met Asn Lys Leu Phe
   450 455 460
   Glu Arg Thr Lys Lys Gln Leu Arg Glu Asn Ala Glu Asp Met Gly Asn
   465 470 475 480
   Gly Cys Phe Lys Ile Tyr His Lys Cys Asp Asn Ala Cys Ile Gly Ser
   485 490 495
   Ile Arg Asn Gly Thr Tyr Asp His Asp Val Tyr Arg Asp Glu Ala Leu
   500 505 510
   Asn Asn Arg Phe Gln Ile Lys Gly Val Glu Leu Lys Ser Gly Tyr Lys
   515 520 525
   Asp Trp Ile Leu Trp Ile Ser Phe Ala Ile Ser Cys Phe Leu Leu Cys
   530 535 540
   Val Ala Leu Leu Gly Phe Ile Met Trp Ala Cys Gln Lys Gly Asn Ile
   545 550 555 560
   Arg Cys Asn Ile Cys Ile
   565
<210> 48
   <211> 566
   <212> PRT
   <213> Influenza A
<220>
   <223> Hemagglutinin, Wyoming/3/03/H3N2, HAWY
<300>
   <308> AAT08000
<400> 48
   Met Lys Thr Ile Ile Ala Leu Ser Tyr Ile Leu Cys Leu Val Phe Ser
   1               5                   10                  15
   Gln Lys Leu Pro Gly Asn Asp Asn Ser Thr Ala Thr Leu Cys Leu Gly
               20                  25                  30
   His His Ala Val Pro Asn Gly Thr Ile Val Lys Thr Ile Thr Asn Asp
           35                  40                  45
   Gln Ile Glu Val Thr Asn Ala Thr Glu Leu Val Gln Ser Ser Ser Thr
       50                  55                  60
   Gly Gly Ile Cys Asp Ser Pro His Gln Ile Leu Asp Gly Glu Asn Cys
   65                  70                  75                  80
   Thr Leu Ile Asp Ala Leu Leu Gly Asp Pro Gln Cys Asp Gly Phe Gln
                   85                  90                  95
   Asn Lys Lys Trp Asp Leu Phe Val Glu Arg Ser Lys Ala Tyr Ser Asn
               100                 105                 110
   Cys Tyr Pro Tyr Asp Val Pro Asp Tyr Ala Ser Leu Arg Ser Leu Val
           115                 120                 125
   Ala Ser Ser Gly Thr Leu Glu Phe Asn Asn Glu Ser Phe Asn Trp Ala
       130                 135                 140
   Gly Val Thr Gln Asn Gly Thr Ser Ser Ala Cys Lys Arg Arg Ser Asn
   145 150 155 160
   Lys Ser Phe Phe Ser Arg Leu Asn Trp Leu Thr His Leu Lys Tyr Lys
   165 170 175
   Tyr Pro Ala Leu Asn Val Thr Met Pro Asn Asn Glu Lys Phe Asp Lys
   180 185 190
   Leu Tyr Ile Trp Gly Val His His Pro Val Thr Asp Ser Asp Gln Ile
   195 200 205
   Ser Leu Tyr Ala Gln Ala Ser Gly Arg Ile Thr Val Ser Thr Lys Arg
   210 215 220
   Ser Gln Gln Thr Val Ile Pro Asn Ile Gly Tyr Arg Pro Arg Val Arg
   225 230 235 240
   Asp Ile Ser Ser Arg Ile Ser Ile Tyr Trp Thr Ile Val Lys Pro Gly
   245 250 255
   Asp Ile Leu Leu Ile Asn Ser Thr Gly Asn Leu Ile Ala Pro Arg Gly
   260 265 270
   Tyr Phe Lys Ile Arg Ser Gly Lys Ser Ser Ile Met Arg Ser Asp Ala
   275 280 285
   Pro Ile Gly Lys Cys Asn Ser Glu Cys Ile Thr Pro Asn Gly Ser Ile
   290 295 300
   Pro Asn Asp Lys Pro Phe Gln Asn Val Asn Arg Ile Thr Tyr Gly Ala
   305 310 315 320
   Cys Pro Arg Tyr Val Lys Gln Asn Thr Leu Lys Leu Ala Thr Gly Met
   325 330 335
   Arg Asn Val Pro Glu Lys Gln Thr Arg Gly Ile Phe Gly Ala Ile Ala
   340 345 350
   Gly Phe Ile Glu Asn Gly Trp Glu Gly Met Val Asp Gly Trp Tyr Gly
   355 360 365
   Phe Arg His Gln Asn Ser Glu Gly Thr Gly Gln Ala Ala Asp Leu Lys
   370 375 380
   Ser Thr Gln Ala Ala Ile Asn Gln Ile Asn Gly Lys Leu Asn Arg Leu
   385 390 395 400
   Ile Gly Lys Thr Asn Glu Lys Phe His Gln Ile Glu Lys Glu Phe Ser
                   405                 410                 415
   Glu Val Glu Gly Arg Ile Gln Asp Leu Glu Lys Tyr Val Glu Asp Thr
               420                 425                 430
   Lys Ile Asp Leu Trp Ser Tyr Asn Ala Glu Leu Leu Val Ala Leu Glu
           435                 440                 445
   Asn Gln His Thr Ile Asp Leu Thr Asp Ser Glu Met Asn Lys Leu Phe
       450                 455                 460
   Glu Arg Thr Lys Lys Gln Leu Arg Glu Asn Ala Glu Asp Met Gly Asn
   465                 470                 475                 480
   Gly Cys Phe Lys Ile Tyr His Lys Cys Asp Asn Ala Cys Ile Glu Ser
                   485                 490                 495
   Ile Arg Asn Gly Thr Tyr Asp His Asp Val Tyr Arg Asp Glu Ala Leu
               500                 505                 510
   Asn Asn Arg Phe Gln Ile Lys Gly Val Glu Leu Lys Ser Gly Tyr Lys
           515                 520                 525
   Asp Trp Ile Leu Trp Ile Ser Phe Ala Ile Ser Cys Phe Leu Leu Cys
       530                 535                 540
   Val Ala Leu Leu Gly Phe Ile Met Trp Ala Cys Gln Lys Gly Asn Ile
   545                 550                 555                 560
   Arg Cys Asn Ile Cys Ile
                   565
<210> 49
   <211> 287
   <212> PRT
   <213> Plasmodium falciparum
<400> 49
   Tyr Val Asp Glu Lys Glu Arg Gln Gly Glu Ile Tyr Pro Phe Gly Asp
   1               5                   10                  15
   Glu Glu Glu Lys Asp Glu Gly Gly Glu Ser Phe Thr Tyr Glu Lys Ser
               20                  25                  30
   Glu Val Asp Lys Thr Asp Leu Phe Lys Phe Ile Glu Gly Gly Glu Gly
           35                  40                  45
   Asp Asp Val Tyr Lys Val Asp Gly Ser Lys Val Leu Leu Asp Asp Asp
       50                  55                  60
   Thr Ile Ser Arg Val Ser Lys Lys His Thr Ala Arg Asp Gly Glu Tyr
   65                  70                  75                  80
   Gly Glu Tyr Gly Glu Ala Val Glu Asp Gly Glu Asn Val Ile Lys Ile
                   85                  90                  95
   Ile Arg Ser Val Leu Gln Ser Gly Ala Leu Pro Ser Val Gly Val Asp
               100                 105                 110
   Glu Leu Asp Lys Ile Asp Leu Ser Tyr Glu Thr Thr Glu Ser Gly Asp
           115                 120                 125
   Thr Ala Val Ser Glu Asp Ser Tyr Asp Lys Tyr Ala Ser Gln Asn Thr
       130                 135                 140
   Asn Lys Glu Tyr Val Cys Asp Phe Thr Asp Gln Leu Lys Pro Thr Glu
   145                 150                 155                 160
   Ser Gly Pro Lys Val Lys Lys Cys Glu Val Lys Val Asn Glu Pro Leu
                   165                 170                 175
   Ile Lys Val Lys Ile Ile Cys Pro Leu Lys Gly Ser Val Glu Lys Leu
               180                 185                 190
   Tyr Asp Asn Ile Glu Tyr Val Pro Lys Lys Ser Pro Tyr Val Val Leu
           195                 200                 205
   Thr Lys Glu Glu Thr Lys Leu Lys Glu Lys Leu Leu Ser Lys Leu Ile
       210                 215                 220
   Tyr Gly Leu Leu Ile Ser Pro Thr Val Asn Glu Lys Glu Asn Asn Phe
   225                 230                 235                 240
   Lys Glu Gly Val Ile Glu Phe Thr Leu Pro Pro Val Val His Lys Ala
                   245                 250                 255
   Thr Val Phe Tyr Phe Ile Cys Asp Asn Ser Lys Thr Glu Asp Asp Asn
               260                 265                 270
   Lys Lys Gly Asn Arg Gly Ile Val Glu Val Tyr Val Glu Pro Tyr
           275                 280                 285